# EUROPEAN PATENT SPECIFICATION

(11) **EP 3 775 254 B1**
(45) Date of publication and mention of the grant of the patent: **21.05.2025**
(21) Application number: 19717559.9
(22) Date of filing: 03.04.2019
(51) Int. Cl.: C12Q 1/04, C12Q 1/24, G01N 33/543, C12N 11/02, C12N 11/14

(54) **MICROORGANISM SEPARATION AND DETECTION**
TRENNUNG UND NACHWEIS VON MIKROORGANISMEN
SÉPARATION ET DÉTECTION DE MICROORGANISMES

(30) Priority: 03.04.2018 GB 201805479
(43) Date of publication of application: 17.02.2021
(73) Proprietor: Momentum Bioscience Limited, Abingdon, Oxfordshire OX14 4SA (GB)
(72) Inventor: LOCKHART, Daniel, Abingdon, Oxfordshire, OX14 4SA (GB); JAY, Paul, Abingdon, Oxfordshire, OX14 4SA (GB); TURNER, James, Abingdon, Oxfordshire, OX14 4SA (GB); ROGERS, Andrew, Abingdon, Oxfordshire, OX14 4SA (GB); CROW, Matthew, Abingdon, Oxfordshire, OX14 4SA (GB); MULLEN, William, Abingdon, Oxfordshire, OX14 4SA (GB)
(74) Representative: Boult Wade Tennant LLP
(86) International application number: PCT/GB2019/050959
(87) International publication number: WO 2019/193332

(56) References cited:
- EP-A1- 2 333 105
- EP-A1- 2 546 199
- EP-A1- 3 085 774
- WO-A1-2013/067399
- WO-A1-2016/024263
- WO-A1-2019/169340
- WO-A1-95/15397
- WO-A2-01/53525
- WO-A2-2014/151879
- KR-A- 20080 017 208
- US-A1- 2015 377 881
- SHEHADUL ISLAM MOHAMMED ET AL: "A Review on Macroscale and Microscale Cell Lysis Methods", MICROMACHINES, vol. 8, no. 3, 8 March 2017 (2017-03-08), Switzerland, pages 83, XP093221224, ISSN: 2072-666X, DOI: 10.3390/mi8030083
- LOONEN ANNE J. ET AL: "Comparison of Pathogen DNA Isolation Methods from Large Volumes of Whole Blood to Improve Molecular Diagnosis of Bloodstream Infections", PLOS ONE, vol. 8, no. 8, 15 August 2013 (2013-08-15), US, pages e72349, XP093221225, ISSN: 1932-6203, DOI: 10.1371/journal.pone.0072349
- JOHN F. REGAN ET AL: "A Sample Extraction Method for Faster, More Sensitive PCR-Based Detection of Pathogens in Blood Culture", THE JOURNAL OF MOLECULAR DIAGNOSTICS, vol. 14, no. 2, 1 March 2012 (2012-03-01), US, pages 120 - 129, XP055602079, ISSN: 1525-1578, DOI: 10.1016/j.jmoldx.2011.10.001

## Description

### FIELD OF THE INVENTION

The present invention relates generally to the field of separating microorganisms from non-microorganism cells in a sample and methods of detecting the absence or presence of microorganisms in a sample. The methods typically rely upon measuring microbial enzyme activity (if any) present in a sample where the sample also contains non-microorganism sources of enzyme activity. The invention relies upon effective isolation of the microorganism source of enzymatic activity. The methods of the invention therefore enable determination of the absence and presence of microbial pathogens in samples such as unpurified blood, blood culture and other body fluids. This invention also relates to kits comprising reagents useful for carrying out the methods.

### BACKGROUND TO THE INVENTION

Measuring the presence and levels of certain molecules which are associated with cell viability is important in a number of contexts. For example, measuring levels of ATP is useful in mammalian cells for growth analysis and toxicology purposes. Culture approaches can be used to detect small numbers of bacteria but such techniques require several days to complete, especially when attempting to detect small numbers of bacteria and also when detecting slower growing microorganisms.

Detection of adenylate kinase as an indicator of viability has also been proposed (Squirrell DJ, Murphy MJ, Leslie RL, Green JCD: A comparison of ATP and adenylate kinase as bacterial cell markers: correlation with agar plate counts). WO96/002665 describes a method for determining the presence and/or amount of microorganisms and/or their intracellular material present in a sample characterized in that the amount of adenylate kinase in the sample is estimated by mixing it with adenosine diphosphate (ADP), determining the amount of adenosine triphosphate (ATP) produced by the sample from this ADP, and relating the amount of ATP so produced to the presence/or amount of adenylate kinase and to microorganisms and/or their intracellular material, wherein the conversion of ADP to ATP is carried out in the presence of magnesium ions at a molar concentration sufficient to allow maximal conversion of ADP to ATP.

In WO2009/007719, NAD- dependent ligases are described as a useful indicator of the presence of a microorganism in a sample. Ligases are enzymes which catalyze ligation of nucleic acid molecules. The ligation reaction requires either ATP or NAD+ as co-factor depending upon the ligase concerned.

WO2011/130584 describes a method for detection of viable microorganisms based on detection of DNA or RNA polymerases in which a sample is contacted with a nucleic acid substrate that acts as a substrate for microbial polymerase, incubated under conditions suitable for polymerase activity from intact microorganisms and any resulting nucleic acid product is determined using a nucleic acid amplification technique such as quantitative polymerase chain reaction. Such assays have been termed "ETGA assays", where ETGA stands for Enzymatic Template Generation and Amplification. A problem with ETGA assays for viable microorganisms in crude samples is the presence of contaminating polymerase activity outside the microorganisms arising from host (e.g. human) cells and dead microorganisms. The ETGA assay is unable to distinguish microorganism polymerase activity from that of the host or from dead microorganisms.

WO2010/119270 describes a method for removing DNA ligase activity outside intact microorganisms.

WO2011/070507 describes the selective lysis of animal cells using a non-ionic detergent and a buffer.

WO/2017/182775 describes a method of detecting the absence or presence of a microorganism in a sample that may also contain non-microorganism cells comprising the selective lysis of non-microorganism cells, filtering the lysate and detecting the absence or presence of microorganisms retained within or upon the filter.

The use of magnetic beads coated with specific binding moieties such as antibodies is also known. The specificity of these products is defined by the specificity of the antibody or other binding ligand, which is generally chosen for a particular purpose to be highly specific to allow the isolation of a particular microorganism.

WO03/102184 describes methods, compositions and kits for concentrating or separating cells (e.g. bacteria) using flocculating agents, such as polyamines or cationic detergents, to form complexes with cells causing them to aggregate. The separation of the aggregated cells can be effected with a solid phase which is capable of binding the cells, such as magnetic beads.

WO01/53525 describes a method of isolating cells (e.g. microorganisms) from a sample which method comprises binding the cells to a solid support by means of a carbohydrate ligand immobilised on the solid support. A kit for performing such a method is sold by DiaSorin Molecular ("Bugs'n Beads^{™}" kit).

Other kits for isolating microorganisms include ApoH-Technologies Peps6 magnetic beads. The beads are coated with the synthetic molecule, Peps6, which is derived from the Apolipoprotein H protein (ApoH), also known as β-2 glycoprotein.

EP2546199 discloses a solid iron oxide support for isolating microorganisms. WO2013/067399 discloses materials and methods for immobilizing, isolating and concentrating cells using carboxylated surfaces. WO2014/151879 discloses using polylysine-coated particles to capture microorganisms. US2015/0377881 discloses methods and compositions for detecting and/or capturing a microbe in a test sample. KR2008/0017208 and EP1972688 disclose a method of amplifying nucleic acid from microorganisms using a nonplanar solid substrate. Regan et al. disclose a sample extraction method for PCR-based detection of pathogens in blood culture (J Mol Diagn. 2012 Mar-Apr;14(2):120-9. doi: 10.1016/j.jmoldx.2011.10.001). WO2016/024263 discloses methods for isolating microbial DNA from a blood sample. WO95/15397 discloses methods for recovering microorganisms from specimens containing blood components. EP2333105 discloses methods and devices for the selective lysis of cells in a sample comprising microorganisms. EP3085774 discloses methods for concentrating, purifying and/or isolating virus particles. WO2019/169340 which may only be relevant under Article 54(3) EPC discloses methods for preparing samples for antimicrobial susceptibility testing.

### DESCRIPTION OF THE INVENTION

The present inventors have recognised that in samples taken from subjects suspected of carrying a microbial infection there are much greater levels of nucleated blood cells (leukocytes) than previously imagined even though the majority of samples are not in fact from infected subjects. This has led to the requirement for improved methods of separating potential microbes from blood cells, in particular leukocytes, in blood samples taken from patients screened for infection. The invention relates to the separation of microorganisms from non-microorganism cells in a sample by selectively capturing microorganisms with particles (e.g. magnetic particles) forming particle-microorganism complexes and separating the particle-microorganism complexes from the non-microorganism cells (e.g. using a magnetic field). This provides a way to address the issue described above enabling the detection of the absence or presence of microorganisms in the sample. The inventors have surprisingly found that this separation can be achieved with particles (e.g. magnetic particles) that are not coated with ligands. The inventors have discovered that certain reagents are particularly useful in the methods to ensure good separation of microorganisms from non-microorganism cells, in particular in complex samples such as blood, milk and urine.

The invention is defined in the appended claims.

The invention provides a method of separating microorganisms from non-microorganism cells in a non-microorganism cell-containing sample, the method comprising: a) incubating the sample with particles to form particle-microorganism complexes, wherein the step of incubating is performed in the presence of sodium polyanethol sulfonate and a reagent that selectively lyses non-microorganism cells in the sample whilst retaining intact microorganisms present in the sample; and b) separating the particle-microorganism complexes from the non-microorganism cells, wherein the sample is a blood containing sample, wherein the particles have a diameter of between 0.1 and 3.0 µm, and wherein the outer surface of the particles comprises or is coated with any one or more of:
i) carboxylic acid groups;
ii) amino groups;
iii) hydrophobic groups; and
iv) streptavidin.

In the methods, the step of incubating is performed in the presence of sodium polyanethol sulfonate and a reagent that selectively lyses non-microorganism cells in the sample whilst retaining intact microorganisms present in the sample.

In the methods, the step of incubating may be performed in the presence of sodium polyanethol sulfonate and/or a detergent. A detergent is an example of a reagent that selectively lyses non-microorganism cells in the sample whilst retaining intact microorganisms present in the sample.

In the methods, the method may further comprise washing the separated particle-microorganism complexes to remove non-microorganism cells or lysate; optionally wherein the separated particle-microorganism complexes are washed with a solution comprising a detergent and/or sodium chloride.

The reagent that selectively lyses non-microorganism cells in the sample whilst retaining intact microorganisms present in the sample may comprise a combination of a detergent and one or more enzymes. The one or more enzymes may comprise a proteinase and/or a DNAse. Suitable detergents and enzymes are discussed herein.

In the methods, step b) may be performed by any suitable means of separation. For example, separation may be achieved using a magnetic field to attract the particle-microorganism complexes or centrifugation.

In the methods, step b) may further comprise removing the non-microorganism cells from the particle-microorganism complexes.

In the methods, selectively lysing non-microorganism cells in the sample whilst retaining intact any microorganisms present in the sample may comprise freezing and thawing the sample.

In the methods, selectively lysing non-microorganism cells in the sample whilst retaining intact any microorganisms present in the sample may comprise adding a detergent.

In the methods, step a) may be performed in the presence of a buffer. The buffer may have a pH between 7.4 and 8.5.

In the methods, step a) may be performed in the presence of sodium chloride. The sodium chloride may be present at a concentration of between 50 and 500 mM. Preferably, the sodium chloride may be present at a concentration around 150 mM.

In the methods, the reagent that selectively lyses non-microorganism cells in the sample whilst retaining intact microorganisms present in the sample may be a detergent. In the methods, the detergent may be non-ionic. In the methods, the detergent may not be conjugated to the particles capable of forming complexes with microorganisms. Thus, typically the detergent forms part of a solution to which the particles are added and does not form part of the particles themselves.

In the methods, the particles have a diameter of between 0.1 and 3 µm or between 0.1 and 2 µm. Preferably, the particles have a diameter of between 0.1 and 1.0 µm

In the methods, the particles may be (and typically are) magnetic. The particles may be superparamagnetic. The particles may comprise iron oxide. The iron oxide may comprise magnetite and/or maghemite. The iron oxide may not comprise a 1:1, 2:1, 3:1 or 4:1 ratio of Fe²⁺ and Fe³⁺.

The outer surface of the particles capable of forming complexes with microorganisms may comprise a polymer; optionally the polymer may be carbon-based. The polymer may not comprise an inorganic polymer. The polymer may comprise polystyrene and/or poly(styrene/divinyl benzene).

In the methods, the outer surface of the particles capable of forming complexes with microorganisms comprises or is coated with any one or more of: i) carboxylic acid groups; ii) amino groups; iii) hydrophobic groups; and iv) streptavidin; optionally the carboxylic acid groups; ii) amino groups; iii) hydrophobic groups may not be part of a polypeptide.

In the methods, the microorganism may be a pathogenic microorganism. For example, the pathogenic microorganism may be a pathogenic bacterium or fungus.

In the methods, the non-microorganism cells may comprise red blood cells and/or white blood cells.

In the methods, the sample may comprise non-microorganism cells at a concentration of between 20,000 and 5 million cells per millilitre. The sample may comprise non-microorganism cells at a concentration of at least around 100,000 cells per millilitre. Preferably, the sample may comprise non-microorganism cells at concentration of at least around 20,000 cells per millilitre.

The sample is one which contains, or is suspected to contain, microorganisms. The sample contains non-microorganism cells which can provide unwanted background when aiming to detect whether and potentially also identify and/or quantify microorganisms present in the sample. Thus, in some embodiments, the sample may comprise blood, urine, saliva or milk. The blood sample may be any sample containing blood cells. The blood sample may be whole blood or may comprise whole blood (e.g. blood broth).

In the methods, the particles (e.g. magnetic particles ) may have an outer polymeric surface that is not coated with any of (i) an antibody, (ii) a carbohydrate, (iii) a peptide derived from Apolipoprotein H protein, (iv) Mannose Binding Lectin, (v) a polyamine or (vi) a cationic detergent.

In the methods, the particles (e.g. magnetic particles) may have an outer surface (e.g. outer polymeric surface) that is not coated with any of (i) an antibody, (ii) a carbohydrate or (iii) an innate immune system protein.

In the methods, the particles (e.g. magnetic particles) may have an outer surface (e.g. outer polymeric surface) that is not coated with any of (i) an antibody, (ii) a carbohydrate, (iii) a peptide derived from Apolipoprotein H protein, (iv) Mannose Binding Lectin, or (v) a flocculating agent (e.g. a flocculating agent as defined in WO 03/102184).

In the methods, the particles (e.g. magnetic particles) may have an outer surface (e.g. outer polymeric surface) that is not coated with any of (i) an antibody, (ii) a carbohydrate, (iii) an innate immune system protein or (iv) a flocculating agent (e.g. a flocculating agent as defined in WO 03/102184).

In the methods, the particles (e.g. magnetic particles) may have an outer surface (e.g. outer polymeric surface) that is not coated with a ligand.

In the methods, the particles (e.g. magnetic particles) may have an outer surface (e.g. outer polymeric surface) that is coated with streptavidin and is not coated with a ligand.

In the methods, the particles (e.g. magnetic particles) may have an outer surface (e.g. an outer polymeric surface) that is coated only with streptavidin.

In the methods, the particles (e.g. magnetic particles) may have an outer surface (e.g. an outer polymeric) surface that is coated only with carboxyl groups.

In the methods, the particles (e.g. magnetic particles) may have an outer surface (e.g. outer polymeric surface) that is not coated with any molecule or moiety capable of binding to a microorganism.

In the methods, the particles (e.g. magnetic particles) may have an outer surface (e.g. an outer polymeric surface) that is not coated with any molecules or moieties.

By "coated" is meant attached to the outer surface (e.g. outer polymeric surface). The skilled person would be aware of means for the attachment of molecules and chemical groups to the outer surface (e.g. outer polymeric surface) of the particles (e.g. magnetic particles).

The separation methods of the invention are useful for enabling detection of whether or not a microorganism is found in a sample that also contains non-microorganism cells. Once the microorganisms have been separated from potential sources of background signal they can then be specifically and sensitively detected using a range of techniques. Accordingly, the invention also provides a method of detecting the absence or presence of a microorganism in a sample that may also contain non-microorganism cells comprising performing a method of the invention and detecting the absence or presence of microorganisms in the particle-microorganism complexes.

The invention provides a method of detecting the absence or presence of a microorganism in a sample that may also contain non-microorganism cells comprising performing a method of the invention and detecting the absence or presence of microorganisms in the particle-microorganism complexes.

The invention further provides a method of detecting the absence or presence of a microorganism in a sample that may also contain non-microorganism cells comprising performing a method of the invention and detecting the absence or presence of microorganisms in the particle-microorganism complexes; wherein the particles have an outer surface that is not coated with any of (i) an antibody, (ii) a carbohydrate, (iii) a peptide derived from Apolipoprotein H protein, (iv) a Mannose Binding Lectin protein.

In the methods, the step of incubating is performed in the presence of sodium polyanethol sulfonate and a reagent that selectively lyses non-microorganism cells in the sample whilst retaining intact microorganisms present in the sample.

In the methods, step c) may comprise (i) detecting an enzymatic activity of a nucleic acid molecule associated with the microorganism, (ii) detecting the microorganism directly by cytometry or microscopy, (iii) detecting the microorganism following cell culture, (iv) detecting the microorganism by PCR or (v) detecting the microorganism by nucleic acid sequencing.

In the methods, step c) may comprise the steps of: i) lysing the microorganisms in the particle-microorganism complexes; ii) incubating the lysate with a nucleic acid molecule which acts as a substrate for nucleic acid modifying activity of the microorganisms; and iii) specifically determining the absence or presence of a modified nucleic acid molecule resulting from the action of the nucleic acid modifying enzyme on the substrate nucleic acid molecule to indicate the absence or presence of the microorganism. In the methods, step (i) may comprise adding a lysis reagent containing the substrate nucleic acid molecule. In the methods, the nucleic acid modifying enzyme may comprise a DNA or RNA polymerase, optionally wherein the DNA polymerase is DNA polymerase I.

Since microorganisms are a common source of infection in a subject, the methods of the invention are useful for identifying infection caused by a microorganism. Accordingly, the invention also provides a method of detecting the absence or presence of a microorganism infection in a subject comprising performing any of the methods described herein (that detect microorganisms in a sample) on a sample from the subject.

The method may further comprise washing the separated particle-microorganism complexes to remove non-microorganism cells or lysate.

In the methods, step (b) may further comprise removing the non-microorganism cells from the particle-microorganism complexes.

In the methods, step b) may be performed by any suitable means of separation. For example, separation may be achieved using a magnetic field to attract the particle-microorganism complexes or centrifugation.

In the methods, step b) may further comprise removing the non-microorganism cells from the particle-microorganism complexes.

In the methods, selectively lysing non-microorganism cells in the sample whilst retaining intact any microorganisms present in the sample may comprise freezing and thawing the sample.

In the methods, selectively lysing non-microorganism cells in the sample whilst retaining intact any microorganisms present in the sample may comprise adding a detergent.

In the methods, step a) may be performed in the presence of a buffer. The buffer may have a pH between 7.4 and 8.5.

In the methods, step a) may be performed in the presence of sodium chloride. The sodium chloride may be present at a concentration of between 50 and 500 mM. Preferably, the sodium chloride may be present at a concentration around 150 mM.

In the methods, the reagent that selectively lyses non-microorganism cells in the sample whilst retaining intact microorganisms present in the sample may be a detergent. In the methods, the detergent may be non-ionic. In the methods, the detergent may not be conjugated to the particles capable of forming complexes with microorganisms. Thus, typically the detergent forms part of a solution to which the particles are added and does not form part of the particles themselves.

In the methods, the particles have a diameter of between 0.1 and 3 µm or between 0.1 and 2 µm. Preferably, the particles have a diameter of between 0.1 and 1.0 µm
In the methods, the particles may be (and typically are) magnetic. The particles may be superparamagnetic. The particles may comprise iron oxide. The iron oxide may comprise magnetite and/or maghemite. The iron oxide may not comprise a 1:1, 2:1, 3:1 or 4:1 ratio of Fe²⁺ and Fe³⁺.

The outer surface of the particles capable of forming complexes with microorganisms may comprise a polymer; optionally the polymer may be carbon-based. The polymer may not comprise an inorganic polymer. The polymer may comprise polystyrene and/or poly(styrene/divinyl benzene).

In the methods, the outer surface of the particles capable of forming complexes with microorganisms comprises or is coated with any one or more of: i) carboxylic acid groups; ii) amino groups; iii) hydrophobic groups; and iv) streptavidin; optionally the carboxylic acid groups; ii) amino groups; iii) hydrophobic groups may not be part of a polypeptide.

In the methods, the microorganism may be a pathogenic microorganism. For example, the pathogenic microorganism may be a pathogenic bacterium or fungus.

In the methods, the non-microorganism cells may comprise red blood cells and/or white blood cells.

In the methods, the sample may comprise non-microorganism cells at a concentration of between 20,000 and 5 million cells per millilitre. The sample may comprise non-microorganism cells at a concentration of at least around 100,000 cells per millilitre. Preferably, the sample may comprise non-microorganism cells at concentration of at least around 20,000 cells per millilitre.

The sample is one which contains, or is suspected to contain, microorganisms. The sample contains non-microorganism cells which can provide unwanted background when aiming to detect whether and potentially also identify and/or quantify microorganisms present in the sample. Thus, in some embodiments, the sample may comprise blood, urine, saliva or milk. The blood sample may be any sample containing blood cells. The blood sample may be whole blood or may comprise whole blood (e.g. blood broth).

In the methods, the particles (e.g. magnetic particles) may have an outer surface (e.g. outer polymeric surface) that is not coated with any of (i) an antibody, (ii) a carbohydrate, (iii) a peptide derived from Apolipoprotein H protein, (iv) a Mannose Binding Lectin protein, (v) a polyamine or (vi) a cationic detergent.

In the methods, the particles (e.g. magnetic particles) may have an outer polymeric surface that is not coated with any of (i) an antibody, (ii) a carbohydrate or (iii) an innate immune system protein.

In the methods, the particles (e.g. magnetic particles) may have an outer polymeric surface that is not coated with any of (i) an antibody, (ii) a carbohydrate, (iii) a peptide derived from Apolipoprotein H protein, (iv) Mannose Binding Lectin, or (v) a flocculating agent (e.g. a flocculating agent as defined in WO 03/102184).

In the methods, the particles (e.g. magnetic particles) may have an outer surface (e.g. outer polymeric surface) that is not coated with a ligand.

In the methods, the particles (e.g. magnetic particles) may have an outer surface (e.g. outer polymeric surface) that is not coated with any of (i) an antibody, (ii) a carbohydrate, (iii) an innate immune system protein or (iv) a flocculating agent (e.g. a flocculating agent as defined in WO 03/102184).

In the methods, the particles (e.g. magnetic particles) may have an outer surface (e.g. outer polymeric surface) that is coated with streptavidin and is not coated with a ligand.

In the methods, the particles (e.g. magnetic particles) may have an outer surface (e.g. outer polymeric surface) that is coated only with streptavidin.

In the methods, the particles (e.g. magnetic particles) may have an outer surface (e.g. outer polymeric surface) that is coated only with carboxyl groups.

In the methods, the particles (e.g. magnetic particles) may have an outer surface (e.g. outer polymeric surface) that is not coated with any molecule or moiety capable of binding to a microorganism.

In the methods, the particles (e.g. magnetic particles) may have an outer surface (e.g. outer polymeric surface) that is not coated with any molecules or moieties.

In the methods, step (c) may comprise (i) detecting an enzymatic activity of a nucleic acid molecule associated with the microorganism, (ii) detecting the microorganism directly by cytometry or microscopy, or (iii) detecting the microorganism following cell culture.

The detection of the absence or presence of microorganisms in the particle-microorganism complexes according to all relevant aspects of the invention can be performed according to any desired method. The method may involve detecting the simple absence or presence of the one or more microorganisms. It may involve quantification of the microorganisms, if present. It may also involve characterisation of the nature of the microorganism in some embodiments. Thus, detection of bacteria and/or fungi may be performed. Discrimination of gram positive versus gram negative bacteria may also be performed. Identification and antimicrobial susceptibility of the organisms may also be performed.

Detection may occur after the removal (or recovery) of the microorganisms from the particle-microorganism complexes. Recovered microorganisms may be lysed prior to detection. Recovery may be of the intact microorganisms or of a lysate following lysis of the microorganisms (as discussed in further detail herein).

Preferably, detection occurs without prior removal (or recovery) of the microorganisms from the particle-microorganism complexes. This embodiment is particularly useful in applying the invention to magnetic bead-processing instrumentation.

The detection of the absence or presence of microorganisms may comprise detecting an enzymatic activity or a nucleic acid molecule associated with the microorganism; detecting the microorganism directly by cytometry or microscopy; or detecting the microorganism following cell culture.

Detection of nucleic acid molecules associated with microorganisms is known in the art and may be performed at the DNA or RNA level. It can be performed by any suitable method, such as amplification (e.g. PCR) or sequencing (in particular next generation sequencing). Such methods may take advantage of sequence divergence between microorganisms and non-microorganisms, such as human, DNA and RNA. Such methods may involve lysing the microorganisms (e.g. present in the form of particle-microorganism complexes) in order to release the nucleic acid component.

Direct detection of microorganisms is also known. This may involve cytometric analysis, for example by flow cytometry. It may involve use of microscopy, for example to visualise the microorganisms recovered from particle-microorganism complexes or to visualise microorganisms in particle-microorganism complexes.

Microorganism detection may also be performed following cell culture, in order to expand the number of microorganisms. Thus, the microorganisms initially captured within particle-microorganism complexes can be cultured for a set period of time, prior to detection. Culture methods may permit direct detection of microorganisms in the original sample.

However, in preferred embodiments, the detection of the absence or presence of microorganisms may comprise detecting an enzymatic activity associated with the microorganism. Suitable enzymatic activities are typically nucleic acid modifying activities and are discussed in greater detail herein.

Accordingly, in the methods, step (c) may comprise the steps of: (i) lysing the microorganisms in the particle-microorganism complexes; (ii) incubating the lysate with a nucleic acid molecule which acts as a substrate for nucleic acid modifying activity of the microorganisms; and (iii) specifically determining the absence or presence of a modified nucleic acid molecule resulting from the action of the nucleic acid modifying enzyme on the substrate nucleic acid molecule to indicate the absence or presence of the microorganism.

In the methods, step (i) may comprise adding a lysis reagent containing the substrate nucleic acid molecule.

Incubating the sample refers to contacting the sample with the particles under conditions conducive to the formation of particle-microorganism complexes. In some embodiments, the step of incubating the sample with particles (e.g. magnetic particles) comprises contacting the particles (e.g. magnetic particles) with the sample for a fixed period of time (e.g. 30 minutes) at a specified temperature (e.g. 37 °C) The incubation may be performed with or without shaking (e.g. by a platform shaker, orbital shaker or shaking incubator set at 500-1000 rpm).

Lysis of microorganisms in the particle-microorganism complexes permits detection of nucleic acid molecules or enzymes within the microorganisms, such as nucleic acid modifying enzymes. Lysis may be achieved by addition of a lysis mixture. The lysis mixture is generally useful in the methods of the invention. The lysis mixture may include a specific mixture of components to ensure efficient lysis of microorganisms without adversely affecting nucleic acid molecules and/or enzyme activity, such as nucleic acid modifying activity, within the cells. The components may be selected from carrier/serum proteins such as BSA, surfactants/detergents, metal halide salts, buffers, chelators etc. In its basic form, the lysis mixture of the invention may include the following components:
1. A surfactant/detergent
2. Serum protein such as albumin (e.g. BSA)
3. Buffer
4. Nucleotides, such as dNTPs
5. Nucleic acid molecule (acting as a substrate in the assays of the invention).

A suitable lysis mixture is set forth below:
L1: 252 mL in 360 mL LM
   1.46% (w/v) BSA
   0.15% Triton X100
   0.15% Tween 20
L2: 36 mL in 360 mL LM
   100 mM Ammonium sulphate
   20 mM Magnesium sulphate heptahydrate
   100 mM Potassium chloride
   200 mM Tris-HCl [pH 8.0]
L3: 36 mL in 360 mL LM
   0.1 µM PTO-AS oligo
   0.1 µM PTO-S1 oligo
   20 mM Tris-HCl [pH 8.5]
   10 mM Potassium chloride
   10 µM EDTA
   10 mM dNTPs: 3.6 mL in 360 mL LM
PTO-IPC stock: ~180 µL in 360 mL LM
H2O: ~32.4 mL in 360 mL LM

By "PTO-AS oligo" is meant an antisense oligonucleotide comprising phosphorothioate nucleotides. By "PTO-S1 oligo" is meant a sense oligonucleotide comprising phosphorothioate nucleotides. The two oligonucleotides hybridise to one another to form the substrate nucleic acid molecule.

By "PTO-IPC" is meant an IPC molecule comprising phosphorothioate nucleotides.

Suitable substrate and IPC molecules are discussed in further detail herein.

Exemplary amounts and concentrations of each component are listed but may be modified as would be readily appreciated by one skilled in the art.

Lysis may also require disruption of the cells. For example, the cells may be disrupted using the lysis mixture in combination with physical and/or enzymatic means. Typically, however, the methods in which the cells are lysed avoid use of physical disruption. In some embodiments, physical disruption employs a disruptor. The disruptor may incorporate beads such as glass beads to lyse the cells. Suitable apparatus are commercially available and include the Disruptor Genie manufactured by Scientific Industries, Inc. Sonication may be utilised, for example applying an ultra sonic horn. Enzymatic disruption may require use of one or more agents selected from lysostaphin, lysozyme and/or lyticase in some embodiments.

Once the microorganisms, if present in the sample, are lysed, the released nucleic acid and/or enzymes may be detected to indicate whether microorganisms are present in the sample. In some embodiments, the lysate is incubated with a nucleic acid molecule which acts as a substrate for nucleic acid modifying activity (of the microorganisms). The absence or presence of a modified nucleic acid molecule resulting from the action of the nucleic acid modifying enzyme on the substrate nucleic acid molecule is then determined to indicate the absence or presence of the microorganism. The nucleic acid substrate molecule is designed according to the nucleic acid modifying activity that is to be detected. One skilled in the art is well able to design suitable substrate nucleic acid molecules. Although the initial sample contains non-microorganism sources of nucleic acid modifying activity, the methods of the invention prevent this contaminating activity acting on the substrate nucleic acid molecules.

The nucleic acid modifying enzyme may comprise a DNA or RNA polymerase, optionally wherein the DNA polymerase is DNA polymerase I.

The nucleic acid modifying enzyme may comprise a ligase, optionally wherein the nucleic acid modifying enzyme is an NAD-dependent ligase.

The invention further provides a method of detecting the absence or presence of a microorganism infection in a subject comprising performing the method of any of the methods described herein on a sample from the subject, optionally wherein the sample comprises blood from the subject.

The method may further comprise washing the separated particle-microorganism complexes to remove non-microorganism cells or lysate. The step of washing may remove inhibitors of the subsequent analysis e.g. PCR inhibitors. The step of washing may be performed under conditions that do not dissociate the particle-microorganism complexes.

According to the methods of the invention typical nucleic acid modifying activity that may be detected comprises polymerase and/or ligase activity. In certain embodiments, nucleic acid modifying enzyme comprises DNA or RNA polymerase. In some embodiments, the DNA polymerase comprises or is DNA polymerase I. In some embodiments, the nucleic acid modifying enzyme comprises a ligase. In certain embodiments, the nucleic acid modifying enzyme comprises or is an NAD-dependent or ATP-dependent ligase. NAD-dependent ligases are only found in (eu)bacteria and thus, detecting such activity may provide an additional level of specificity. This is discussed further in WO2009/007719 and WO2010/119270. Other nucleic acid modifying activities relevant to viability may alternatively be measured such as phosphatase, kinase and/or nuclease activity.

In some embodiments, the action of the nucleic acid modifying activity on the substrate nucleic acid molecule produces an extended nucleic acid molecule. This may be by strand extension (polymerase activity) and/or by ligation of two nucleic acid molecules (ligase activity). In some embodiments, a substrate that can be acted upon by either polymerase or ligase is utilised since either activity is indicative of the presence of a microorganism in the sample. In some embodiments, the relevant activity can be distinguished in terms of the novel nucleic acid molecule that is produced.

The substrate may be a template for the nucleic acid modifying activity of the microorganisms. For example, the substrate may be a template for a DNA or RNA polymerase, optionally wherein the DNA polymerase is DNA polymerase I.

Suitable nucleic acid molecules which acts as a substrate for nucleic acid modifying activity of the microorganisms are described in WO2011/130584, WO2010/119270 and WO2009/007719 . In the case of phosphatase activity, suitable nucleic acid molecules are disclosed in WO2006/123154, .

In specific embodiments, the (substrate) nucleic acid molecule used in the methods of the invention is at least partially double stranded and comprises uracil residues in the complementary strand and the step of specifically determining the absence or presence of the modified nucleic acid molecule comprises adding Uracil DNA Glycosylase (UDG) to the sample in order to degrade the uracil residues in the complementary strand.

In certain embodiments, the (substrate) nucleic acid molecule comprises DNA. In certain embodiments, the (substrate) nucleic acid molecule comprises DNA and is partially doublestranded.

In some embodiments, the (substrate) nucleic acid molecule comprises a nucleic acid consisting of a sense oligonucleotide (DNA) strand and an antisense oligonucleotide (DNA) strand, wherein the two strands overlap to form a double stranded region and a single stranded portion of the antisense oligonucleotide strand acts as a template with the sense oligonucleotide strand of the double stranded region acting as a primer to create an extension product in the presence of polymerase activity;

In certain embodiments, the first strand of the partially double stranded (substrate) nucleic acid molecule comprises (or consists of) synthetic nucleotides (e.g. phosphorothioate nucleotides) and the second (complementary) strand comprises (or consists of) uracil residues and, optionally, synthetic nucleotides (e.g. phosphorothioate nucleotides). Preferably, the double stranded region encompasses the 3' end regions of the first and second (complementary) strands. Preferably, the second (complementary) strand comprises a base (e.g. dideoxyCytidine) at its 3' end that blocks DNA polymerase-mediated extension of the second strand. Such partially double stranded (substrate) nucleic acid molecules are described, for example, in Zweitzig et al., 2012 (Characterization of a novel DNA polymerase activity assay enabling sensitive, quantitative and universal detection of viable microbes. Nucleic Acids Research 40, 14, e109, 1-12). Preferably, the double stranded region is at least 5, at least 10, at least 15, at least 20 or at least 25 nucleotides; optionally, the double stranded region is no more than 50 nucleotides. The first strand may be extended during an incubation step, as described herein, using unprotected (or standard) dNTPs by the polymerase activity of a microorganism in the sample to form an extended first strand that comprises unprotected (or standard) nucleotides. This step relies upon using the second strand as template (upstream of the region of complementarity between the first and second strands). Following the incubation step, the second (complementary) strand may be degraded by adding Uracil DNA Glycosylase (UDG) to the sample leaving the extended first strand as a single stranded molecule comprising synthetic nucleotides and unprotected nucleotides. Following degradation of the second strand, the extended first strand of the (substrate) nucleic acid molecule may be detected in an amplification step. The inventors have found that the use of a partially double stranded (substrate) nucleic acid molecule as described above improves the detection of a microorganism in the sample.

In some embodiments, the substrate nucleic acid molecule is pre-modified so as to protect it from nuclease activity i.e. the nucleic acid molecule is modified so as to protect it from nuclease activity before it is added to the assay. The inventors have determined that protection of the substrate nucleic acid molecule from nuclease activity is advantageous in the context of the assays of the invention. More specifically, incorporation of protected nucleic acid molecules into the methods of the invention improves sensitivity of detection. Any suitable means may be employed in order to protect the nucleic acid molecule from nuclease activity. Non-limiting examples include incorporation of methylation into the nucleic acid molecule, end modification such as protection of the 3' and/or 5' ends and incorporation of synthetic nucleotides. In specific embodiments, the synthetic nucleotides comprise phosphorothioate nucleotides and/or locked nucleic acid nucleotides. Preferably, the synthetic nucleotides are phosphorothioate nucleotides. In certain embodiments, the synthetic nucleotides replace at least one up to all of the nucleotides in the nucleic acid molecule.

The (substrate) nucleic acid molecules may include any natural nucleic acid and natural or synthetic analogues that are capable of being acted upon by nucleic acid modifying activity in order to generate a (novel detectable) nucleic acid molecule. The substrate may be extended and/or ligated in specific embodiments. Combinations of nucleic acid substrate molecules may be employed to permit detection of polymerase and ligase activity in some embodiments.

The nucleic acid substrate may be present in excess, and in particular in large molar excess, over the nucleic acid modifying activity (provided by the microorganisms) in the sample. Because a novel extended or ligated nucleic acid molecule is detected, only the presence of this molecule in the sample is essential for the detection methods to work effectively. Thus, it is not detrimental to the methods of the invention if other nucleic acid molecules are present in the sample such as from the microorganisms to be detected or from mammalian or other sources which may be found in the sample to be tested for example.

The inventors have previously investigated the use of an internal positive control (IPC) molecule in the context of their methods. Thus, according to all aspects, the invention may rely upon inclusion of an IPC molecule. In some embodiments, the IPC is included with the substrate nucleic acid molecule so that the IPC is exposed to identical conditions. In some embodiments, the IPC molecule is pre-modified so as to protect it from nuclease activity i.e. the nucleic acid molecule is modified so as to protect it from nuclease activity before it is added to the assay. The inventors have determined that protection of the IPC molecule from nuclease activity is advantageous in the context of the assays of the invention. Any suitable means may be employed in order to protect the nucleic acid molecule from nuclease activity. Non-limiting examples include incorporation of methylation into the nucleic acid molecule, end modification such as protection of the 3' and/or 5' ends and incorporation of synthetic nucleotides. In specific embodiments, the synthetic nucleotides comprise phosphorothioate nucleotides and/or locked nucleic acid nucleotides. Preferably, the synthetic nucleotides are phosphorothioate nucleotides. In certain embodiments, the synthetic nucleotides replace at least one up to all of the nucleotides in the IPC molecule. Preferably, the substrate and IPC molecules are modified in the same manner as it is advantageous for them to behave similarly in the assays of the invention.

In some embodiments, the internal positive control (IPC) nucleic acid molecule comprises identical primer binding sites to the substrate nucleic acid molecule such that there is competition for primer binding in a nucleic acid amplification reaction containing both the nucleic acid molecule and the IPC.

In all methods of the invention specifically determining the absence or presence of the modified nucleic acid molecule may comprise, consist essentially of or consist of a nucleic acid amplification step. This serves to make the methods of the invention maximally sensitive. Such amplification techniques are well known in the art, and include methods such as PCR, NASBA (Compton, 1991), 3SR (Fahy et al., 1991), Rolling circle replication, Transcription Mediated Amplification (TMA), strand displacement amplification (SDA) Clinical Chemistry 45: 777-784, 1999, the DNA oligomer self-assembly processes described in US6261846 , ligase chain reaction (LCR) (Barringer et al., 1990), selective amplification of target polynucleotide sequences (US 6410276), arbitrarily primed PCR (WO 90/06995), consensus sequence primed PCR (US 4437975), invader technology, strand displacement technology and nick displacement amplification (WO 2004/067726). The list above is not intended to be exhaustive. Any nucleic acid amplification technique may be used provided the appropriate nucleic acid product is specifically amplified.

Similarly, sequencing based methodologies may be employed in some embodiments to include any of the range of next generation sequencing platforms, such as sequencing by synthesis of clonally amplified sequences (Illumina), pyrosequencing, 454 sequencing (Roche), nanopore sequencing (e.g. Oxford Nanopore), ion torrent (ThermoFisher) and single molecule real-time (SMRT) sequencing (Pacific Biosystems). The fact that a novel nucleic acid molecule is generated means that a sequencing approach can confirm the presence or otherwise of the modified nucleic acid molecule and also provide quantification of that molecule.

Amplification is achieved with the use of amplification primers specific for the sequence of the modified nucleic acid molecule which is to be detected. In order to provide specificity for the nucleic acid molecules primer binding sites corresponding to a suitable region of the sequence may be selected. The skilled reader will appreciate that the nucleic acid molecules may also include sequences other than primer binding sites which are required for detection of the novel nucleic acid molecule produced by the modifying activity in the sample, for example RNA Polymerase binding sites or promoter sequences may be required for isothermal amplification technologies, such as NASBA, 3SR and TMA.

One or more primer binding sites may bridge the ligation/extension boundary of the substrate nucleic acid molecule such that an amplification product is only generated if ligation/extension has occurred, for example. Alternatively, primers may bind either side of the ligation/extension boundary and direct amplification across the boundary such that an amplification product is only generated (exponentially) if the ligated/extended nucleic acid molecule is formed. Primers and the substrate nucleic acid molecule(s) may be designed to avoid non-specific amplification (e.g. of genomic DNA in the sample).

Primers may incorporate synthetic nucleotide analogues as appropriate or may be RNA or PNA based for example, or mixtures thereof. The primers may be labelled, such as with fluorescent labels and/or FRET pairs, depending upon the mode of detection employed.

Probes may be utilised, again which may be labelled, as desired. The detection method may require use of nucleotide probes in addition to primers, or as an alternative to primers. For example, a branched DNA assay, which does not require use of primers, may be employed in some embodiments.

In certain aspects, the methods of the invention are carried out using nucleic acid amplification techniques in order to detect the modified nucleic acid molecule produced as a direct result of the action of nucleic acid-modifying activity on the substrate nucleic acid molecule which indicates the presence of a micro-organism in the sample. In certain embodiments the technique used is selected from PCR, NASBA, 3SR, TMA, SDA and DNA oligomer self-assembly.

Detection of the amplification products may be by routine methods, such as, for example, gel electrophoresis but in some embodiments is carried out using real-time or end-point detection methods.

A number of techniques for real-time or end-point detection of the products of an amplification reaction are known in the art. These include use of intercalating fluorescent dyes such as SYBR Green I (Sambrook and Russell, Molecular Cloning - A Laboratory Manual, Third edition), which allows the yield of amplified DNA to be estimated based upon the amount of fluorescence produced. Many of the real-time detection methods produce a fluorescent read-out that may be continuously monitored; specific examples including molecular beacons and fluorescent resonance energy transfer probes. Real-time and end-point techniques are advantageous because they keep the reaction in a "single tube". This means there is no need for downstream analysis in order to obtain results, leading to more rapidly obtained results. Furthermore keeping the reaction in a "single tube" environment reduces the risk of cross contamination and allows a quantitative output from the methods of the invention. This may be particularly important in the context of the present invention where health and safety concerns may be of paramount importance (such as in detecting potential microbial infection in a patient samples for example).

Real-time and end-point quantitation of PCR reactions may be accomplished using the TaqMan^{®} system (Applied Biosystems), see Holland et al; Detection of specific polymerase chain reaction product by utilising the 5'-3' exonuclease activity of Thermus aquaticus DNA polymerase; Proc. Natl. Acad. Sci. USA 88, 7276-7280 (1991), Gelmini et al. Quantitative polymerase chain reaction-based homogeneous assay with flurogenic probes to measure C-Erb-2 oncogene amplification. Clin. Chem. 43, 752-758 (1997) and Livak et al. Towards fully automated genome wide polymorphism screening. Nat. Genet. 9, 341-342 (19995) . This type of probe may be generically referred to as a hydrolytic probe. Suitable hydrolytic/Taqman probes for use in real time or end point detection are also provided. The probe may be suitably labelled, for example using the labels detailed below.

In the Molecular Beacon system, see Tyagi & Kramer. Molecular beacons - probes that fluoresce upon hybridization. Nat. Biotechnol. 14, 303-308 (1996) and Tyagi et al. Multicolor molecular beacons for allele discrimination. Nat. Biotechnol. 16, 49-53 (1998) , the beacons are hairpin-shaped probes with an internally quenched fluorophore whose fluorescence is restored when bound to its target. These probes may be referred to as hairpin probes.

A further real-time fluorescence based system which may be incorporated in the methods of the invention is the Scorpion system, see Detection of PCR products using self-probing amplicons and fluorescence by Whitcombe et al. Nature Biotechnology 17, 804 - 807 (01 Aug 1999). Additional real-time or end-point detection techniques which are well known to those skilled in the art and which are commercially available include Lightcycler^{®} technology, Amplifluour^{®} primer technology, DzyNA primers (Todd et al., Clinical Chemistry 46:5, 625-630 (2000)), or the Plexor^{™} qPCR and qRT-PCR Systems.

Thus, in further aspects of the invention the products of nucleic acid amplification are detected using real-time or end point techniques. In specific embodiments of the invention the real-time technique consists of using any one of hydrolytic probes (the Taqman^{®} system), FRET probes (Lightcycler^{®} system), hairpin primers (Amplifluour^{®} system), hairpin probes (the Molecular beacons system), hairpin probes incorporated into a primer (the Scorpion^{®} probe system), primers incorporating the complementary sequence of a DNAzyme and a cleavable fluorescent DNAzyme substrate (DzYNA), Plexor qPCR and oligonucleotide blocking systems.

Amplification products may be quantified to give an approximation of the microbial nucleic acid modifying activity in the sample and thus the level of microorganisms in the sample. Thus, "absence or presence" is intended to encompass quantification of the levels of microorganisms in the sample.

The inventors have further discovered that the optimal temperature for measuring nucleic acid modifying activity of the microorganisms may not be the same as the optimal temperature for lysis of microorganisms. Thus, in some embodiments, lysis of the microorganisms is performed at a lower temperature than the step of incubating the lysate with a nucleic acid molecule that acts as a substrate for nucleic acid modifying activity of the microorganisms. As already discussed, in some embodiments of the invention, the substrate nucleic acid molecule is included in the lysis reagent used to lyse the microorganisms. Such embodiments are consistent with the differing temperature preferences. Thus, even though the substrate nucleic acid molecule may be included in the lysis reagent, the initial lower temperature does not adversely affect the subsequent incubation at higher temperature, at which the substrate is modified by the nucleic acid modifying activity released from the microorganisms. Accordingly, in some embodiments the method involves a step of lysis of the microorganisms in which the lysis reagent contains a nucleic acid molecule which acts as a substrate for nucleic acid modifying activity of the microorganisms. This step is performed at a lower temperature than the subsequent step of incubating the lysate with the substrate nucleic acid molecule to enable the activity of the enzymes released from the microorganisms. Thus, the substrate is exposed to the initial lower temperature, followed by a higher temperature under which enzyme activity is enhanced.

In some embodiments, the step of incubating the lysate with a nucleic acid molecule that acts as a substrate for nucleic acid modifying activity of the microorganisms is performed at a temperature of at least around 30°C. The temperature may be optionally between around 30°C and 40°C or between around 32°C and 37°C, such as around 37°C.

In additional or alternative embodiments, the step of lysis of the microorganisms is performed at a temperature of no more than around 30°C, optionally between around 15°C and 30°C or between around 18°C and 25°C, such as around 18, 19, 20, 21, 22, 23, 24 or 25°C. In some embodiments, all steps prior to incubating the lysate with a nucleic acid molecule that acts as a substrate for nucleic acid modifying activity of the microorganisms are performed at a temperature of no more than around 30°C. The temperature may optionally be between around 15°C and 30°C or between around 18°C and 25°C, such as around 18, 19, 20, 21, 22, 23, 24 or 25°C.

Such a method may incorporate any one or more up to all of the embodiments described in relation to the various aspects of the invention.

In some embodiments, the method is further characterised in that the step of incubating the lysate with a substrate nucleic acid molecule is performed at a temperature of at least around 30°C, optionally between around 30°C and 40°C or between around 32°C and 37°C, such as around 37°C.

In additional or alternative embodiments, each of steps prior to incubating the lysate with a substrate nucleic acid molecule is performed at a temperature of no more than around 30°C, optionally between around 15°C and 30°C or between around 18°C and 25°C, such as around 18, 19, 20, 21, 22, 23, 24 or 25°C.

Prior to the step of incubating the sample with magnetic particles to form particle-microorganism complexes (i.e. prior to step (a)), the method may comprise selectively lysing non-microorganism cells in the sample whilst retaining intact microorganisms present in the sample.

The step of selectively lysing non-microorganism cells in the sample whilst retaining intact microorganisms present in the sample may comprise adding a combination of a detergent and one or more enzymes to the sample. The one or more enzymes may comprise a proteinase and/or a DNAse, optionally wherein the proteinase is proteinase K.

The step of selective lysis of non-microorganism cells in the sample whilst retaining intact any microorganisms present in the sample may prevent enzymatic activity from non-microorganism cells, such as leukocytes, falsely indicating the presence of microorganisms in the sample. Such selective lysis can be achieved by any suitable means as discussed further herein. Any suitable reagent that lyses non-microorganisms, in particular mammalian cells, present in the sample but does not lyse microorganisms in the sample may be utilised. The reagent may include a surfactant or detergent in some embodiments, such as a non-ionic detergent. Suitable examples include polyethylene glycol sorbitan monolaurate (Tween 20), for example at 5% w/v. The reagent may include a saponin, for example at 5% w/v. The reagent may include a metal halide salt, such as sodium chloride, for example at 8.5g/l. The reagent may include a mixture of all three components. The sample may be mixed with the reagent under suitable conditions to ensure lysis of non-microorganism cells, in particular mammalian cells, if present in the sample but no (or insignificant) lysis of microorganisms if present in the sample. The sample may be exposed to the reagent for a period of between around 5 and 30 minutes, such as 5, 10, 15, 20, 25 or 30 minutes. This step may be performed at any suitable temperature, for example between 15 and 30 degrees Celsius or at room temperature.

In some embodiments, according to all aspects of the invention, selective lysis of non-microorganism cells in the sample whilst retaining intact any microorganisms present in the sample comprises adding a combination of a detergent and one or more enzymes to the sample. Without wishing to be bound by any particular theory, the detergent selectively permeabilises non-microorganism cell membranes, whereas the microorganisms are protected by virtue of their cell wall. The enzymes are useful for breaking down released intracellular material and other cellular debris and may contribute to preventing carry over of released enzymatic activity. In some embodiments, the one or more enzymes comprise a proteinase and/or a nuclease. Suitable proteinases include proteinase K. Suitable nucleases include DNAses. In one embodiment, the reagent used to selectively lyse non-microorganism cells comprises a combination of triton X-100 and proteinase K. More specifically the lysis reagent may comprise 0.25% Triton X-100 and 4.8 µg/mL Proteinase K.

It is important to inactivate any relevant enzymatic activity released if the non-microorganism cells are lysed. The inventors have devised methods in which high pH conditions are utilised to ensure effective inactivation of the enzymatic activity. The microbial cells typically remain intact, at least during some of the treatment, and intracellular enzymatic activity is not significantly adversely affected by the high pH treatment. In addition, the inventors have previously shown that microbial enzymes are more resistant to the high pH treatment in any case.

Accordingly, after the step of selective lysis of non-microorganism cells in the sample whilst retaining intact any microorganisms present in the sample, the method may comprise exposing the lysate to high pH conditions. The duration of exposure to the high pH conditions is typically less than 20 minutes and may be not more than 10, 9, 8, 7, 6 or 5 minutes and may be around 5, 6, 7, 8, 9 or 10 minutes. In some embodiments the treatment is carried out for between around 2 and 15 minutes, such as around 5 minutes. By "around" is meant plus or minus 30 seconds.

Any suitable reagent may be in order to provide high pH conditions. In particular embodiments, the high pH conditions comprise contacting the sample with an alkali or a buffer. In particular embodiments, NaOH or Na2CO3 is used. In specific embodiments, the concentration of the NaOH or Na2CO3 is around 5mM or greater. The buffer may have a pKa value above 9. Examples of suitable buffers include borate, carbonate and pyrophosphate buffers.

The high pH conditions typically inhibit the activity of nucleic acid modifying enzymes including ATP-dependent ligase and polymerases from non-microorganism sources such as mammalian cells, but do not inhibit the activity of the microbial ligases or polymerases. This is primarily due to the differential lysis conditions employed in the methods to ensure that only the non-microorganism enzymes are exposed to the high pH conditions. However, it may also be due to the greater resistance of microbial enzymes to these conditions. "High pH" is generally a pH of at least around 10, such as around 10, 11, 12, 13 or 14. "Low pH" is generally a pH of less than or equal to around 4, such as around 4, 3, 2, or 1. By "around" is meant 0.5 of a pH unit either side of the stated value. Altering the pH of the sample may be achieved using any suitable means, as would be readily appreciated by one skilled in the art. Microbial enzymes such as polymerases and ligases may be resistant to extremes of pH, whereas corresponding mammalian enzymes may be inactivated under the same pH conditions. This assists with the selective detection of microbial enzymatic activity in a sample containing both mammalian cells and microbial cells. In specific embodiments, the conditions that inhibit the activity of non-microorganism nucleic acid modifying activity, such as ATP-dependent ligase, from mammalian cells but which do not inhibit the activity of the microorganism source of nucleic acid modifying activity, such as microbial ligases, comprise treating the sample with sodium hydroxide (NaOH) or sodium carbonate (Na2CO3). Such agents can readily be used, as shown herein, to increase the pH of the sample to high pH thus inactivating non-microorganism enzymatic activity whilst leaving the microbial (fungal and bacterial) enzymes active. Suitable concentrations and volumes of the appropriate agent can be applied by a skilled person. In certain embodiments, however, the NaOH is at least around 5mM NaOH. In some embodiments, the alkali concentration is no more than 10mM, such as 5, 6, 7, 8, 9 or 10mM.

In further embodiments, the pH is around 12 to inactivate mammalian nucleic acid modifying activity (such as polymerase and/or ATP-dependent ligase activity), but not microbial nucleic acid modifying activity (such as polymerase and/or ligase activity). In specific embodiments, pH conditions may be increased to at least around 11, or at least 11.2. This treatment may, after a certain period of time, result in lysis of microorganisms in the sample and thus lead to nucleic acid modifying activity (e.g. polymerase and/or ligase) release into the sample. Thus, in some embodiments, the lysis of microorganisms is achieved by high pH treatment. This permits detection of nucleic acid modifying activity (e.g. polymerases and/or ligases) in the sample, originating from the microorganism, without the need for a separate cell lysis step. Under these conditions, mammalian ligases (such as blood ATP-dependent ligases) are inactivated. However, typically the methods include a separate step for lysing microorganisms in the sample, as discussed in greater detail herein.

In some embodiments, the treatment under high pH conditions is stopped by adding a reagent to lower the pH. This is done before the microorganisms are lysed. Suitable reagents include a buffer and/or an acid. Thus, the pH may be reduced by adding a neutralisation buffer. In specific embodiments, the buffer comprises a Tris-HCl buffer (e.g. pH 7.2 or 8). Other suitable agents for lowering the pH include acids such as hydrochloric acid (HCl) and sulphuric acid (H2SO4). These (and other) acids may be incorporated into a buffer as would be readily appreciated by one skilled in the art. One specific reagent useful for treating the sample after the pH has been elevated comprises a combination of Ammonium sulphate, Magnesium sulphate heptahydrate, Potassium chloride and Tris-HCl. More specifically, the reagent may comprise 10 mM Ammonium sulphate, 2 mM Magnesium sulphate heptahydrate, 10 mM Potassium chloride and 20 mM Tris-HCl [pH 8.0].

Step (b) may further comprise removing the non-microorganism cells from the particle-microorganism complexes e.g. by aspiration.

A "sample" in the context of the present invention is one which contains non-microorganism cells and in which it is desirable to test for the presence of a microorganism, such as a fungus (e.g. a yeast) and/or a bacterium, expressing nucleic acid modifying activity. The sample is a blood containing sample. The sample may comprise, consist essentially of or consist of a clinical sample, such as a blood sample (to include whole blood, plasma, serum, such as a blood culture or blood broth). The methods of the invention are particularly applicable to the rapid determination of negative (and positive) blood cultures. Thus, the sample may comprise a blood culture sample (or blood broth sample) from a patient suspected of suffering from, or being screened for, a bloodstream infection. The sample may be any suitable volume such as 1 to 10ml, preferably a 1ml blood culture sample.

The sample being used will depend on various factors, such as availability, convenience and the condition that is being tested for. Typical samples which may be used, but which are not intended to limit the invention, include whole blood, serum, plasma, platelet, joint fluid and urine samples etc. taken from a patient, most preferably a human patient. The patient may be suspected of suffering from, or being screened for, a bloodstream infection. The patient may be a hospitalised patient. The sample may be taken from a subject comprising more than 5, 10 or 15 million white blood cells (WBC) per ml of blood. The methods of the invention represent *in vitro* tests. They are carried out on a sample removed from a subject. Described herein (not according to the invention) are methods that may additionally include the step of obtaining the sample from a subject. Methods of obtaining a suitable sample from a subject are well known in the art. Typically, however, the method may be carried out beginning with a sample that has already been isolated from the patient in a separate procedure. The methods will most preferably be carried out on a sample from a human, but the methods of the invention may have utility for many animals.

The methods of the invention may be used to complement any already available diagnostic techniques, potentially as a method of confirming an initial diagnosis. Alternatively, the methods may be used as a preliminary diagnosis method in their own right, since the methods provide a quick and convenient means of diagnosis. Furthermore, due to their inherent sensitivity, the methods of the invention require only a minimal sample, thus preventing unnecessary invasive surgery. Also, a large but non-concentrated sample may also be tested effectively according to the methods of the invention.

In specific embodiments according to all aspects of the invention, the microorganism that may be detected in the sample is a pathogenic microorganism, such as a pathogenic bacterium or fungus/yeast. The bacterium may be any bacterium which is capable of causing infection or disease in a subject, preferably a human subject. In one embodiment, the bacteria comprises or consists essentially of or consists of any one or more of Staphylococcus species, including *Staphylococcus epidermidis* and *Staphylococcus aureus* (and preferably methicillin resistant strains), Enterococcus species, Streptococcus species, Mycobacterium species, in particular *Mycobacterium tuberculosis*, Vibrio species, in particular *Vibrio cholerae*, Salmonella and/or *Escherichia coli* etc. The bacteria may comprise, consist essentially of or consist of Clostridium species and in particular *C*. *difficile* in certain embodiments. *C*. *difficile* is the major cause of antibiotic-associated diarrhoea and colitis, a healthcare associated intestinal infection that mostly affects elderly patients with other underlying diseases. Candida species such as *C*. *albicans, C. parapsilosis* and *C*. *glabrata* may be detected. Cryptococcus species such as *C*. *neoformans* may be detected. Fungaemia such as Candidaemia may be detected (presence or absence) using the invention. The microorganism is preferably (although this is not essential) indicated through its enzymatic activity. Thus, the methods provide an indication of viable, or recently so, microorganisms in the sample. After a period of time, if the microorganisms are not viable, the enzymatic activity would be lost from the sample. This represents an advantage of using enzymatic activity as an indicator of microorganisms in the sample over use of nucleic acid molecules, in particular DNA, which may persist for much longer.

The methods of the invention may involve identifying the nature of the infection, once the positive presence of a microorganism has been detected in the sample. Any suitable method may be employed for this further identification step.

The magnetic particles may be superparamagnetic particles.

The particles (e.g. magnetic particles) may have a greater affinity for the microorganisms than for the non-microorganism cells. The magnetic particles may bind to the microorganisms by non-specific binding.

The particles (e.g. magnetic particles) may have an outer polymeric surface that comprises polystyrene and/or poly(styrene/divinyl benzene).

The magnetic particles may comprise iron oxide. Preferably, the iron oxide is encapsulated by the outer polymeric surface. The particles may be an amalgam of iron oxide and polymer. The particles may be partially encapsulated by an outer polymeric surface. The polymer may comprise polystyrene,
The particles (e.g. magnetic particles) may have a diameter of between, 0.1 and 0.5 µm, 0.2 and 0.3 µm. Preferably, the magnetic particles may have a diameter of between 0.2 and 0.3 µm. The particles (e.g. magnetic particles) have a diameter of between 0.1 and 3 µm or 0.1 and 2 µm. More preferably, the particles have a diameter of between 0.1 and 1.0 µm.

The particles (e.g. magnetic particles) may have an outer polymeric surface. The outer polymeric surface of the magnetic particles may not be coated with any of (i) an antibody, (ii) a carbohydrate, (iii) a peptide derived from Apolipoprotein H protein, (iv) a Mannose Binding Lectin protein, (v) a polyamine or (vi) a cationic detergent.

The Mannose Binding Lectin (MBL) protein may be a genetically engineered protein based on MBL. For example, it may be a genetically engineered protein comprising the pathogen-binding portion of MBL fused to an Fc region of an immunoglobulin (i.e. FcMBL).

The outer surface (e.g. outer polymeric surface) of the particles (e.g. magnetic particles) may not be coated with any of (i) an antibody, (ii) a carbohydrate or (iii) an innate immune system protein.

The outer surface (e.g. outer polymeric surface) of the particles (e.g. magnetic particles) may not be coated with any of (i) an antibody, (ii) a carbohydrate, (iii) a peptide derived from Apolipoprotein H protein, (iv) Mannose Binding Lectin, or (v) a flocculating agent (e.g. a flocculating agent as defined in WO 03/102184).

The outer surface (e.g. outer polymeric surface) of the particle (e.g. magnetic particles) may not be coated with any of (i) an antibody, (ii) a carbohydrate, (iii) an innate immune system protein or (iv) a flocculating agent (e.g. a flocculating agent as defined in WO 03/102184).

The antibody may be a fragment or derivative of an antibody that retains antigen-specific binding function. Such fragments and derivatives include Fab fragments, ScFv, single domain antibodies, nanoantibodies, heavy chain antibodies etc.

The carbohydrate may be a monosaccharide, oligosaccharide (e.g. a disaccharide or a trisaccharide), a polysaccharide and/or a derivative thereof.

The outer surface (e.g. outer polymeric surface) of the particles (e.g. magnetic particles) may not be coated with a ligand. The outer surface (e.g. outer polymeric surface) of the particles (e.g. magnetic particles) may not be coated with a non-specific ligand (e.g. a non-specific ligand as described in WO01/53525). The outer surface (e.g. outer polymeric surface) of the particles (e.g. magnetic particles) may not be coated with a non-proteinaceous ligand (e.g. a non-proteinaceous ligand as described in WO01/53525).

The outer surface (e.g. outer polymeric surface) of the particles (e.g. magnetic particles) may be carboxylated. The outer surface (e.g. outer polymeric surface) of the particles (e.g. magnetic particles) may be coated only with carboxyl groups.

The outer surface (e.g. outer polymeric surface) of the particles (e.g. magnetic particles) may be coated with streptavidin. The outer surface (e.g. outer polymeric surface) of the particles (e.g. magnetic particles) may be coated with streptavidin and not coated with a ligand. The outer surface (e.g. outer polymeric surface) of the particles (e.g. magnetic particles) may be coated only with streptavidin.

The outer surface (e.g. outer polymeric surface) of the particles (e.g. magnetic particles) may not be coated.

The microorganism may be a pathogenic microorganism, optionally wherein the pathogenic microorganism is a pathogenic bacterium or fungus.

The non-microorganism cells may comprise red blood cells and/or white blood cells.

The invention further provides a composition as defined in the claims. The composition provided herein may be for performing any of the methods described herein. All aspects and embodiments described in relation to the methods of the invention apply *mutatis mutandis* to the related compositions.

The invention provides a composition comprising:
a) a blood containing sample isolated from a subject potentially containing microorganism cells and that contains non-microorganism cells;
b) particles capable of forming complexes with microorganisms, wherein the particles have a diameter of between 0.1 and 3.0 µm, and wherein the particles have an outer surface comprising or coated with any one or more of:
   i) carboxylic acid groups;
   ii) amino groups;
   iii) hydrophobic groups; and
   iv) streptavidin;
c) sodium polyanethol sulfonate; and
d) at least one reagent that selectively lyses non-microorganism cells in the sample whilst retaining intact microorganisms present in the sample, wherein the at least one reagent that selectively lyses non-microorganism cells in the sample whilst retaining intact microorganisms present in the sample comprises a detergent.

A detergent is an example of a reagent that selectively lyses non-microorganism cells in the sample whilst retaining intact microorganisms present in the sample.

The composition may further comprise microorganism cells. The composition may comprise a sample suspected of containing microorganism cells and known to contain non-microorganism cells.

The composition may further comprise a buffer and/or sodium chloride.

In the composition, the detergent may be non-ionic. In the composition, the detergent may not be conjugated to the particles capable of forming complexes with microorganisms. Thus, typically the detergent forms part of a solution to which the particles are added and does not form part of the particles themselves.

In the compostion, the particles have a diameter of between 0.1 and 3 µm, or between 0.1 and 2 µm. Preferably, the particles have a diameter of between 0.1 and 1.0 µm

In the composition, the particles may be (and typically are) magnetic. In the methods, the particles may be superparamagnetic. The particles may comprise iron oxide. The iron oxide may comprise magnetite and/or maghemite. The iron oxide may not comprise a 1:1, 2:1, 3:1 or 4:1 ratio of Fe²⁺ and Fe³⁺.

In the composition, the outer surface of the particles capable of forming complexes with microorganisms may comprise a polymer; optionally the polymer may be carbon-based. The polymer may not comprise an inorganic polymer. The polymer may comprise polystyrene and/or poly(styrene/divinyl benzene).

In the composition, the outer surface of the particles capable of forming complexes with microorganisms comprises or is coated with any one or more of: i) carboxylic acid groups; ii) amino groups; iii) hydrophobic groups; and iv) streptavidin; optionally the carboxylic acid groups; ii) amino groups; iii) hydrophobic groups may not be part of a polypeptide.

In the composition, the microorganism may be a pathogenic microorganism. For example, the pathogenic microorganism may be a pathogenic bacterium or fungus.

In the composition, the non-microorganism cells may comprise red blood cells and/or white blood cells.

In the composition, the sample may comprise blood, urine, saliva or milk, optionally wherein the sample is whole blood.

The invention further provides a kit for performing any of the methods described herein. All aspects and embodiments described in relation to the methods of the invention apply *mutatis mutandis* to the related kits.

The invention provides a kit for performing the method of the invention, comprising:
a) particles capable of forming complexes with microorganisms, wherein the particles have a diameter of between 0.1 and 3.0 µm, and wherein the particles have an outer surface comprising or coated with any one or more of:
   i) carboxylic acid groups;
   ii) amino groups;
   iii) hydrophobic groups; and
   iv) streptavidin;
b) sodium polyanethol sulfonate; and
c) at least one reagent that selectively lyses non-microorganism cells in the sample whilst retaining intact microorganisms present in the sample, wherein the at least one reagent that selectively lyses non-microorganism cells in the sample whilst retaining intact microorganisms present in the sample comprises a detergent

A detergent is an example of a reagent that selectively lyses non-microorganism cells in the sample whilst retaining intact microorganisms present in the sample.

The detergent may be non-ionic. The detergent may not be conjugated to the particles capable of forming complexes with microorganisms. Thus, typically the detergent forms part of a solution to which the particles are added and does not form part of the particles themselves.

In the kit, the particles have a diameter of between 0.1 and 3 µm or between 0.1 and 2 µm. Preferably, the particles have a diameter of between 0.1 and 1.0 µm
In the kit, the particles may be (and typically are) magnetic. The particles may be superparamagnetic. The particles may comprise iron oxide. The iron oxide may comprise magnetite and/or maghemite. The iron oxide may not comprise a 1:1, 2:1, 3:1 or 4:1 ratio of Fe²⁺ and Fe³⁺.

The outer surface of the particles may comprise a polymer; optionally the polymer may be carbon-based. The polymer may not comprise an inorganic polymer. The polymer may comprise polystyrene and/or poly(styrene/divinyl benzene).

The kit may further comprise d) detection means for detecting the absence or presence of microorganisms in the particle-microorganism complexes, wherein the detection means comprises a nucleic acid molecule which acts as a substrate for nucleic acid modifying activity of the microorganisms, and wherein the nucleic acid molecule is at least partially double stranded and comprises uracil residues in the complementary strand.

The kit may further comprise d) detection means for detecting the absence or presence of microorganisms in the particle-microorganism complexes, wherein the detection means comprises a nucleic acid molecule which acts as a substrate for nucleic acid modifying activity of the microorganisms, and wherein the nucleic acid molecule is at least partially double stranded and comprises uracil residues in the complementary strand.

In the kit, the particles may have an outer surface that is not coated with any of (i) an antibody, (ii) a carbohydrate, (iii) a peptide derived from Apolipoprotein H protein, (iv) a Mannose Binding Lectin protein; and the kit may further comprise detection means for detecting the absence or presence of microorganisms in the particle-microorganism complexes, wherein the detection means comprises a nucleic acid molecule which acts as a substrate for nucleic acid modifying activity of the microorganisms, and wherein the nucleic acid molecule is at least partially double stranded and comprises uracil residues in the complementary strand.

The kit may further comprise a buffer and/or sodium chloride.

In the kit, the
detergent may be non-ionic. In the composition, the detergent may not be conjugated to the particles capable of forming complexes with microorganisms.

In the kit, the particles have a diameter of between 0.1 and 3 µm, 0.1 and 2 µm. Preferably, the particles have a diameter of between 0.1 and 1.0 µm

In the kit, the particles may be magnetic. In the methods, the particles may be superparamagnetic.

In the kit, the outer surface of the particles capable of forming complexes with microorganisms may comprise a polymer; optionally the polymer may be carbon-based.

In the kit, the outer surface of the particles capable of forming complexes with microorganisms comprises or is coated with any one or more of: i) carboxylic acid groups; ii) amino groups; iii) hydrophobic groups; and iv) streptavidin; optionally carboxylic acid groups; ii) amino groups; iii) hydrophobic groups may not be part of a polypeptide.

In the kit, the microorganism may be a pathogenic microorganism, optionally wherein the pathogenic microorganism may be a pathogenic bacterium or fungus.

In the kit, the non-microorganism cells may comprise red blood cells and/or white blood cells.

In the kit, the sample may comprise blood, urine, saliva or milk, optionally wherein the sample is whole blood.

The kit may further comprise detection means for detecting the absence or presence of microorganisms in the particle-microorganism complexes.

Any suitable detection means may be employed and they may represent the complete set of reagents needed for detecting the absence or presence of microorganisms in the particle-microorganism complexes.

In certain embodiments, the detection means comprises, or is, a nucleic acid molecule which acts as a substrate for nucleic acid modifying activity of the microorganisms.

In some embodiments, the detection means comprise or further comprise reagents for nucleic acid amplification. The reagents for nucleic acid amplification may comprise a primer pair and/or at least one probe. In some embodiments, those primers and/or probes hybridise with a microorganism nucleic acid molecule. They may therefore allow detection of microorganisms in the sample by detecting the amplified microorganism nucleic acid molecule. Alternatively, the primers or probes hybridise to a nucleic acid molecule which acts as a substrate for nucleic acid modifying activity of the microorganisms. Such nucleic acid molecules are described in further detail herein.

The kit may further comprise detection means for detecting the absence or presence of microorganisms in the particle-microorganism complexes. The detection means may comprise a nucleic acid molecule which acts as a substrate for nucleic acid modifying activity of the microorganisms. The nucleic acid molecule may be at least partially double stranded and may, optionally, comprise uracil residues in the complementary strand. The complementary strand may comprise a base (e.g. dideoxyCytidine) at its 3' end that blocks DNA polymerase-mediated extension of the second strand. The nucleic acid molecule may be any of the nucleic acid molecules described herein.

In the kits, the particles (e.g. magnetic particles) may have an outer surface (e.g.outer polymeric surface) that is not coated with any of (i) an antibody, (ii) a carbohydrate, (iii) a peptide derived from Apolipoprotein H protein, (iv) a Mannose Binding Lectin protein, (v) a polyamine or (vi) a cationic detergent.

In the kits, the particles (e.g. magnetic particles) may have an outer surface (e.g. outer polymeric surface) that is not coated with any of (i) an antibody, (ii) a carbohydrate or (iii) an innate immune system protein.

In the kits, the particles (e.g. magnetic particles) may have an outer surface (e.g. outer polymeric surface) that is not coated with any of (i) an antibody, (ii) a carbohydrate, (iii) a peptide derived from Apolipoprotein H protein, (iv) Mannose Binding Lectin, or (v) a flocculating agent (e.g. a flocculating agent as defined in WO 03/102184).

In the kits, the particles (e.g. magnetic particles) may have an outer surface (e.g. outer polymeric surface) that is not coated with any of (i) an antibody, (ii) a carbohydrate, (iii) an innate immune system protein or (iv) a flocculating agent (e.g. a flocculating agent as defined in WO 03/102184).

In the kits, the particles (e.g. magnetic particles) may have an outer surface (e.g. outer polymeric surface) that is not coated with a ligand.

In the kits, the particles (e.g. magnetic particles) may have an outer surface (e.g. outer polymeric surface) that is coated with streptavidin and is not coated with a ligand.

In the kits, the particles (e.g. magnetic particles) may have an outer surface (e.g. outer polymeric surface) that is coated only with streptavidin.

In the kits, the particles (e.g. magnetic particles) may have an outer surface (e.g. outer polymeric surface) that is coated only with carboxyl groups.

In the kits, the particles (e.g. magnetic particles) may have an outer surface (e.g. outer polymeric surface) that is not coated with any molecule or moiety capable of binding to a microorganism.

In the methods, the particles (e.g. magnetic particles) may have an outer surface (e.g. outer polymeric surface) that is not coated with any molecules or moieties.

The (substrate) nucleic acid molecule may be designed on the basis that the nucleic acid modifying enzyme comprises a DNA or RNA polymerase. In some embodiments, the DNA polymerase is DNA polymerase I. In additional or alternative embodiments, the nucleic acid modifying enzyme comprises a ligase, such as an ATP- or NAD-dependent ligase.

The detection means may further comprise reagents for nucleic acid amplification, optionally wherein the reagents for nucleic acid amplification comprise a primer pair and/or at least one probe that hybridises with the nucleic acid molecule.

In the kit, the reagent capable of lysing microorganisms in the particle-microorganism complexes may comprise the nucleic acid molecule which acts as a substrate for nucleic acid modifying activity of the microorganisms.

The reagent that selectively lyses non-microorganism cells in the sample whilst retaining intact microorganisms present in the sample may comprise a combination of a detergent and one or more enzymes, wherein the one or more enzymes optionally comprise a proteinase and/or a DNAse. Suitable detergents and enzymes are discussed herein.

The kit may further comprise a high pH reagent e.g. a base or a buffer. This may, for example, be NaOH, e.g. 5mM NaOH. Other suitable reagents are described herein.

The kit may further comprise a neutralisation buffer. The neutralisation buffer may be capable of restoring the pH of the sample following the high pH treatment. Suitable reagents are described herein.

The nucleic acid modifying enzyme may comprise: (a) a DNA or RNA polymerase, optionally wherein the DNA polymerase is DNA polymerase I; and/or (b) a ligase, optionally wherein the ligase is an ATP- and/or NAD-dependent ligase.

According to all relevant aspects and embodiments of the invention, the term "sodium polyanethol sulfonate" is intended to encompass all functionally equivalent derivatives and salt forms thereof (e.g. potassium polyanethol sulfonate, magnesium polyanethol sulfonate, etc.)

Throughout the disclosure, the term "particles" and "beads" may be used interchangeably.

### BRIEF DESCRIPTION OF THE FIGURES

Figure 1 is an image of blood samples and shows the extent of blood lysis for each sample-set: E-BUF, UREA, Tris+NaCl, freezing (left to right) (see Example 8).

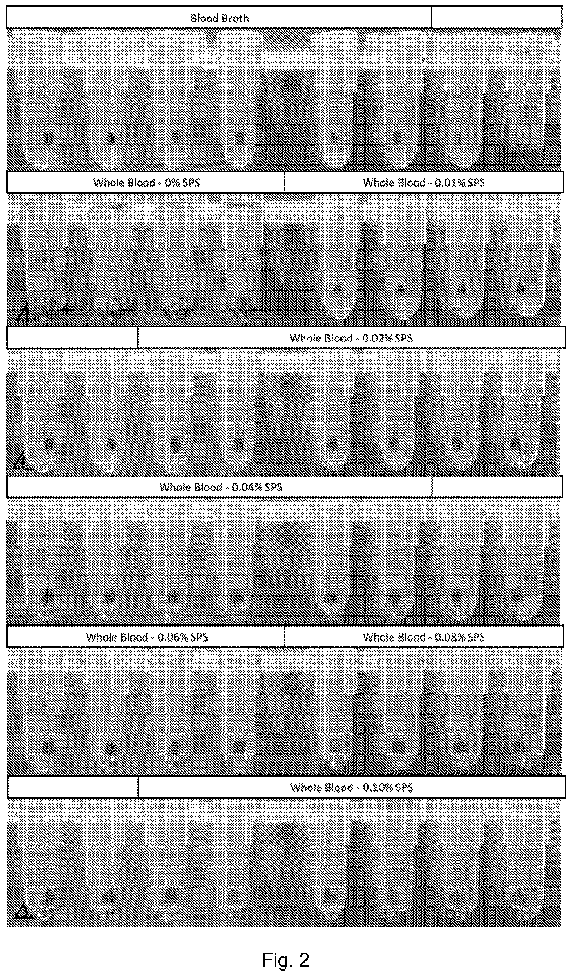

Figure 2 is an image of final sample outputs prior to PCR set-up. The image provides a visual demonstration of the benefit of SPS for sample processing with magnetic beads in blood: SPS appears to enable more thorough removal of blood components as indicated by less red eluates in the presence of SPS. Note, that the BacTec PLUS aerobic broth used for the Blood Broth sample-set also contains SPS (see Example 9).

The invention will be understood with respect to the following non-limiting examples:

### EXPERIMENTAL SECTION

### Abbreviations & definitions:

- 5th%: Fifth Percentile threshold calculation to determine 5% FPR (formula = PERCENTILE.INC(array,0.05))
- BO: Broth Only
- BB: Blood Broth
- Cfu: Colony Forming Unit
- Confirm: A PCR multiplex assay targeting microbial DNA according to gram status (Gram Negative, Gram Positive or Candida)
- CPD: Citrate Phosphate Dextrose
- Ct: Cycle Threshold Value
- CV: Critical Value (cfu): theoretical limit of detection based on cfu value and ΔCt using formula: sample cfu ÷ 2^{ΔCt}
- D1..: Dilution point (10-fold series)
- E*cfu: Extrapolated cfu value using dilution point with highest countable TVC in a dilution series
- EC: *Escherichia coli*
- ETGA: Enzyme Template Generation and Amplification
- IPC: Internal Process Control: PCR template present in LM to demonstrate correct sample processing and verify PCR amplification in ETGA negative samples
- LAWN: Confluent microbial growth
- LM: Microbial Lysis Mix containing a mixture of detergents and microbial lytic enzymes
- MM: Master Mix
- NoCt: No amplification above threshold fluorescence after 50 cycles
- NSC: No Spike Control
- O/n: Overnight
- PC: Polymerase-spike Control
- PCR: Polymerase Chain Reaction
- Pt: Positivity threshold calculated from NSC/NC results
- qPCR: Quantitative Polymerase Chain Reaction
- RT: Room temperature (+19 to +20°C)
- s/n: Supernatant
- SPS: Sodium polyanethol sulfonate
- TNTC: Too Numerous To Count
- TVC: Total Viable Count
- WB: Wash Buffer (containing Tris-HCl + Sodium Chloride + Igepal + Sodium Deoxycholate + Tergitol, unless otherwise stated); or Whole Blood where stated.
- ΔCt: Difference between two Ct values (typically NSC Ct - positive sample Ct)

### Example 1

In a manual format, two bead types (Merck Bio-Estapor (streptavidin-conjugated) 300 nm beads (Product - BE-M08/03; "Bio-Estapor") and ademtech Bio- Adembeads Streptavidin Plus 200 nm beads (Product number 03222; "Bio-Ademtech")) were compared to ApoH Technologies Peps6 beads (Reference - MP20006; "ApoH Peps6").

In experiment 1A, an aliquot of Bio-Estapor beads (25uL) and an aliquot of ApoH Pep6 beads (10uL) were compared for binding. The higher volume of Bio-Estapor reflected the lower number of beads per mL in the material provided compared to the ApoH material. Three organisms were tested: *E. coli* (Gram negative bacterium), *S. epidermidis* (Gram positive bacterium) and *C.albicans* (yeast). 0.5mL of organism suspension was exposed to the beads in 0.5mL "TTGB" microbial binding buffer, provided in the ApoH Peps6 kit ("Peps6 Captobac", Reference MP10031-50T).

After allowing the organism to bind for 30 min, the sample of beads was separated from the liquid supernatant by applying a magnetic field to concentrate the beads and removing the supernatant with a pipette. The beads were gently washed with three aliquots of wash buffer (50mM Tris pH 8, 1% v/v Igepal CA-630, 150mM NaCl, 0.25% v/v Tergitol 15-S-9) and the retained supernatant and the washed beads were analysed for viable organisms by two methods; colony counts on an Agar Petri dish and detection of microbial DNA by the enzymatic template generation and amplification (ETGA) test (as described in Zweitzig et al., 2012. Characterization of a novel DNA polymerase activity assay enabling sensitive, quantitative and universal detection of viable microbes. Nucleic Acids Research 40, 14, e109, 1-12; and in WO2011/130584, WO2013/103744 and WO2016/005768).

The plate counts in Table 1A show that with the Bio-Estapor beads and *E.coli*, the great majority of growth is found from the beads (33 CFU) vs the supernatant (2 CFU) and this is similar to the result from ApoH Peps6. No growth was found with *S. epidermidis* and this organism did not appear to grow in the original broth. *C. albicans* showed approximately 10% of the CFU in the supernatant and 90% bound, for both Bio-Estapor and ApoH Peps6. These results indicate that the Bio-Estapor beads appear to bind organisms at an equivalent rate to the commercially available organism-binding beads Peps6 under the conditions of the test. The sensitive ETGA test supports the results but indicates that *S. epidermidis* may bind to Bio-Estapor better than Peps6 as shown by the lower Cq value.

**Table 1A**

| **Experiment 1 A** | | | | | |
|---|---|---|---|---|---|
| **Estapor beads at 25 µL, ApoH at 10 µL (+ve ctl) and no beads (-ve ctl) vs E. coli, S. epidermidis & C. albicans dilutions in blood broth (Manual protocol).** | | | | | |
| E.c. ONC dilⁿ 2 | | 1.01E+04 | cfu/mL | **E. coli** | |
| S. e. ONC dilⁿ 2 | | No growth | cfu/ml | **Staph epidermidis** | |
| C. a. ONC dilⁿ 1 | | 3.60E+05 | cfu/ml | **Candida albicans** | |

| Tube | Organism | ApoH | Estapor | Plate count CFU | ETGA Resuts Cq |
|---|---|---|---|---|---|
| Bio-Estapor beads | | | | | |
| Bio-Estapor in binding buffer (supernatant) | *E. coli* | | 25 µL | 2 | not tested |
| Bio-Estapor in binding buffer (bound) | | | | 33 | 32.54 |
| Bio-Estapor in binding buffer (supernatant) | *S*. *epidermidis* | | 25µL | 0 | not tested |
| Bio-Estapor in binding buffer (bound) | | | | 0 | 37.63 |
| Bio-Estapor in binding buffer (supernatant) | *C. albicans* | | 25 µL | 34 | not tested |
| Bio-Estapor in binding buffer (bound) | | | | 246 | 33.34 |

| ApoH Peps6 beads | | | | | |
|---|---|---|---|---|---|
| ApoH Peps6 in binding buffer (supernatant) | *E. coli* | 10 µL | | 1 | not tested |
| ApoH Peps6 in binding buffer (bound) | | | | 38 | 33.18 |
| ApoH Peps6 in binding buffer (supernatant) | *S*. *epidermidis* | 10 µL | | 0 | not tested |
| ApoH Peps6 in binding buffer (bound) | | | | 0 | 40.94 |
| ApoH Peps6 in binding buffer (supernatant) | *C. albicans* | 10 µL | | 35 | not tested |
| ApoH Peps6 in binding buffer (bound) | | | | ≈316 | 34.01 |

| No beads | | | | | |
|---|---|---|---|---|---|
| No beads | *E.coli* | | | 43 | not tested |
| No beads | *S. epidermidis* | | | 0 | not tested |
| No beads | *c. albicans* | | | ≈316 | not tested |
| | No bugs / no beads | | | | |
| No beads no microbes | | | | 0 | not tested |

Experiment 1B demonstrates *E. coli* binding under similar conditions to Experiment 1A although in 1B the wash steps were omitted. Experiment 1B shows that another bead, Bio-Ademtech, also binds organisms although at a lower level (see Table 1B). Here the plate counts indicate that approximately one third of the viable counts have bound to the bead. The more sensitive ETGA DNA polymerase assay indicates that half of the organisms remain on the beads, as the Cq for the beads and the supernatant are approximately equal.

**Table 1B**

| **Experiment 1B** | | | | | |
|---|---|---|---|---|---|
| **AdemTech beads and ApoH (+ve ctl) and no beads & no beads/no bugs controls.** | | | | | **- E.** |
| ONC dilⁿ 2 | 1.26E+04 | cfu/mL | | | |

| Tube | AdemTech | ApoH | Estapor | Plate count CFU | ETGA Resuts Cq |
|---|---|---|---|---|---|
| AdemTech beads | | | | | |
| Ademtech beads supernatant | 100µL | | | 78 | 34.09 |
| Ademtech beads bound | 100µL | | | 44 | 34.02 |

| ApoH Peps6 (5µL in 95µL) | | | | | |
|---|---|---|---|---|---|
| ApoH Peps6 supernatant | 5µL | | | 0 | 40.16 |
| ApoH Pep6 bound | 5µL | | | 36 | 32.8 |

| No beads | | | | | |
|---|---|---|---|---|---|
| No beads | | | | 45 | 34.22 |

| No bugs / no beads | | | | | |
|---|---|---|---|---|---|
| No beads/no organisms | | | | 0 | Excluded |

### Example 2

In Experiment 2, ApoH Peps6, Bio-Estapor and Estapor beads with a carboxylated surface (Product MI-030/40; "Estapor COOH") were compared. The number of organisms remaining in the supernatant after binding of *E.coli* to the beads for 30 min was measured using a fluorescent ATP assay (BacTiter-Glo Microbial Cell Viability Assay; Promega Corporation, G8230). Although this is an indirect test in that it does not directly detect the presence of organisms on the bead, it is a useful comparative test for the ligand-based beads (ApoH Peps6) and the non-ligand beads (Bio-Estapor and Estapor COOH). After binding of 1mL of 10⁴ CFU/mL E coli for 30 mL from a phosphate saline buffer, an aliquot of the supernatant was assayed for ATP as a measure of organism content using the BacTiter-Glo assay. The results in Table 2 show that the reduction in levels of organisms in the supernatant for Peps6 beads, Bio-Estapor and Estapor COOH were 33%, 27% and 24% respectively when measured using this technique.

**Table 2**

| | | | **Sample (10^4 CFU/ml E.coli) (RFU)** | **Baseline (No E.coli) (RFU)** | **Baseline corrected (RFU)** | **% Binding Based on Depletion of Supernatant** | **Signal to noise** |
|---|---|---|---|---|---|---|---|
| **Bead Supplier (Type)** | **Date of assay** | **Time** | | | | | |
| | | | | | | | |
| ApoH (Peps6) | 06/03/2018 | am | 1896 | 345 | 1551 | 32% | 5.50 |
| ApoH (Peps6) | 06/03/2018 | am | 1856 | 345 | 1511 | 34% | 5.38 |
| ApoH (Peps6) | 06/03/2018 | pm | 2549 | 378 | 2171 | 31% | 6.74 |
| ApoH (Peps6) | 06/03/2018 | pm | 2398 | 378 | 2020 | 35% | 6.34 |
| | | | | | **Average** | **33%** | |
| | | | | | **Std Dev** | **2.1%** | |
| | | | | | **%CV** | **6.4%** | |
| | | | | | | | |
| BioEstapor (Sav) | 06/03/2018 | am | 2067 | 345 | 1722 | 24% | 5.99 |
| BioEstapor (Sav) | 06/03/2018 | am | 2138 | 345 | 1793 | 21% | 6.20 |
| BioEstapor (Sav) | 06/03/2018 | pm | 2447 | 378 | 2069 | 34% | 6.47 |
| BioEstapor (Sav) | 06/03/2018 | pm | 2618 | 378 | 2240 | 28% | 6.93 |
| | | | | | **Average** | **27%** | |
| | | | | | **Std Dev** | **5.4%** | |
| | | | | | **%CV** | **20.1%** | |
| Estapor (COOH) | 06/03/2018 | am | 2045 | 345 | 1700 | 25% | 5.93 |
| Estapor (COOH) | 06/03/2018 | am | 2500 | 345 | 2155 | 5% | 7.25 |
| Estapor (COOH) | 06/03/2018 | pm | 2416 | 378 | 2038 | 35% | 6.39 |
| Estapor (COOH) | 06/03/2018 | pm | 2520 | 378 | 2142 | 32% | 6.67 |
| | | | | | **Average** | **24%** | |
| | | | | | **Std Dev** | **13.2%** | |
| | | | | | **%CV** | **54.3%** | |
| No Beads | 06/03/2018 | am | 2578 | 345 | 2233 | N/A | 7.47 |
| No Beads | 06/03/2018 | am | 2668 | 345 | 2323 | N/A | 7.73 |
| No Beads | 06/03/2018 | pm | 3594 | 378 | 3216 | N/A | 9.51 |
| No Beads | 06/03/2018 | pm | 3418 | 378 | 3040 | N/A | 9.04 |

### Example 3

Example 3 shows results from testing *E.coli* (EC), *S. aureus* (SA) and *C. albicans* (CA) in dilution series performed by automating the method for magnetic separation described in Example 1. The assay used Bio-Estapor 300nm diameter beads as the capture medium with a binding buffer of TTGB containing 0.25% Tergitol. As 10-fold dilutions of each of the three organisms were made, so a continuous change in the Ct was recorded allowing a dose response curve to be constructed.

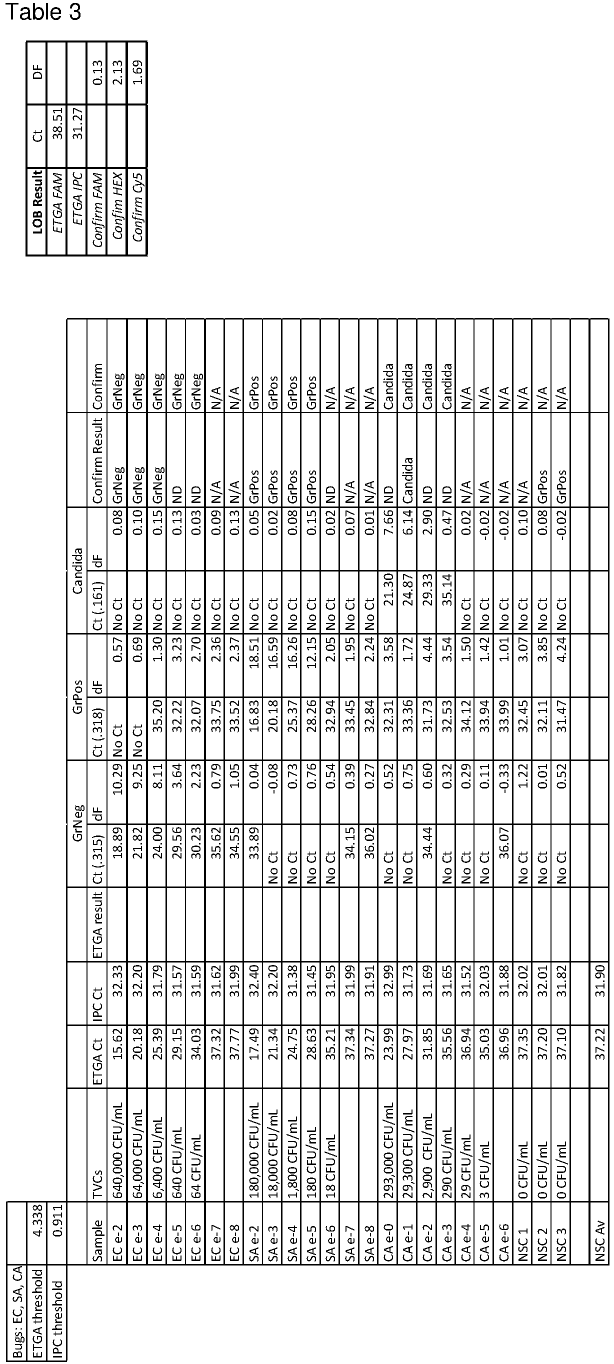

The following examples demonstrate the universal microbial capture of microorganisms by magnetic beads in Momentum's Magnitor test. The Magnitor test consists of two microbial detection read-outs:
- **ETGA:** detection of microbial polymerase from intact microbial cells
- **Confirm:** detection of microbial DNA according to gram status (Gram Negative, Gram Positive, or Candida)

### Key findings:

- Magnetic beads capture bacteria and fungi from simple buffers and a variety of complex biological specimen types
- Microbial capture occurs using a variety of different bead sizes (0.2 to 1.5 µm diameter beads) and surface coatings (e.g. carboxylated, hydrophobic, aminated etc)

Certain binding buffer components can improve microbial detection in the Magnitor assay, for example detergent-based lysis of blood.

### Example 4: Detection of microorganisms is dependent on capture by magnetic beads

### Aim:

Microbial binding performance was assessed for *E*. *coli, S. aureus* and *C. albicans* in a simple Tris+NaCl buffer (pH buffered with physiological salt conc. to prevent microbial osmotic shock that may occur in water only). A 'no bead control' sample-set was also included in this experiment to demonstrate that detection is dependent on the presence of magnetic beads for microbial capture.

### Magnetic bead preparation:

Estapor beads (Merck, Cat# M1-30/40) washed 3 x 1 mL in 1X Tris+NaCl buffer: 40 µL beads resuspended in a final volume of 400 µL 1X Tris+NaCl buffer (1% solid content)

### Protocol:

- Microorganism overnight liquid cultures (o/n) set-up as standard in BacTec PLUS aerobic broth (inoculation of 3 mL broth from agar plate). The following day (approx. 16 hours later) 1.88 µL *E*. *coli and S. aureus* liquid culture added to 3 mL broth, and 18.75 µL *C. albicans* liquid culture added to 3 mL broth; and 2-hour outgrowth performed at 37°C, 500 rpm.
- Following 2-hour outgrowth, microorganism precultures diluted (DF10) in 1X Tris+NaCl buffer (50 mM Tris-HCl [pH8.0] + 150 mM NaCl) to create four dilution points per microorganism.
- 100 µL TVCs performed for each microbial dilution

### Manual simulation of Magnitor performed using DynaMag-2 magnet and manual liquid transfers:

- 1 mL samples added to 2 mL tubes containing 15 µL prewashed beads - Note, 112 µL 1X Tris+NaCl buffer not added to tube with beads (as per standard protocol), because all microbial samples were diluted in the same 1X buffer.
- 30 mins shaking (1000 rpm) @ 37°C
- 5 mins magnetisation on DynaMag-2
- All s/n removed
- 1 mL Wash Buffer (WB) added and tubes mixed for 2 mins @ RT (1000 rpm)
- 3 mins magnetisation on Dynmag-2
- All s/n removed
- 50 µL Lysis Mix (LM) added to tubes off magnet (5 µL Polymerase Control (PC) added to each PC sample tube)
- ETGA reaction performed: 5 mins at 1000 rpm, then 55 mins at 800 rpm @26°C
- Manual qPCR set-up for ETGA and Confirm (10 µL reactions)

### Results:

### TVCs (COL/SAB agar plates)

| | Colonies | *E cfu/mL |
|---|---|---|
| *E. coli D1* | TNTC | 216,000 |
| *E. coli D2* | TNTC | 21,600 |
| *E. coli D3* | *216 | 2,160 |
| *E. coli D4* | 26 | 216 |
| *S. aureus D1* | *671 | 6,710 |
| *S. aureus D2* | 121 | 671 |
| *S. aureus D3* | 33 | 67 |
| *S. aureus D4* | 0 | 7 |
| *C. albicans D1* | *51 | 510 |
| *C. albicans D2* | 6 | 51 |
| *C. albicans D3* | 8 | 5 |
| *C. albicans D4* | 0 | 1 |
| NSC | 0 | - |

| | | |
|---|---|---|
| **E cfu*/*mL values derived from highest countable TVC plate* | | |

### ETGA Ct

| | ***E. coli*** | | ***S. aureus*** | | ***C. albicans*** | |
|---|---|---|---|---|---|---|
| | **(+) BEADS** | **(-) BEADS** | **(+) BEADS** | **(-) BEADS** | **(+) BEADS** | **(-) BEADS** |
| **D1** | 12.64 | 26.25 | 18.99 | 29.29 | 26.57 | 30.61 |
| **D2** | 18.09 | 29.02 | 24.28 | 32.40 | 30.08 | 33.62 |
| **D3** | 22.75 | 32.11 | 28.61 | 32.70 | 33.27 | 33.12 |
| **D4** | 30.52 | 31.01 | 30.85 | 31.56 | 30.80 | 31.65 |
| **NSC1** | 35.26 | 32.22 | 35.73 | 31.37 | 34.69 | 31.31 |
| **NSC2** | 35.67 | 33.13 | 35.61 | 35.13 | 35.29 | 32.40 |
| **NSC3** | 34.92 | 32.38 | 35.28 | 31.54 | 34.31 | 31.77 |
| **PC** | 32.10 | 30.79 | 31.40 | 29.64 | 31.69 | 29.34 |
| **Average NSC** | 35.28 | 32.58 | 35.54 | 32.68 | 34.76 | 31.83 |

### Internal Process Control (IPC) Ct

| | ***E. coli*** | | ***S. aureus*** | | ***C. albicans*** | |
|---|---|---|---|---|---|---|
| | **(+) BEADS** | **(-) BEADS** | **(+) BEADS** | **(-) BEADS** | **(+) BEADS** | **(-) BEADS** |
| **D1** | 37.36 | 31.51 | 32.61 | 32.20 | 32.32 | 31.95 |
| **D2** | 32.85 | 32.11 | 32.26 | 32.38 | 32.10 | 32.26 |
| **D3** | 32.44 | 32.10 | 31.98 | 32.19 | 31.82 | 33.10 |
| **D4** | 32.15 | 32.01 | 32.10 | 32.15 | 31.68 | 32.15 |
| **NSC1** | 32.20 | 32.15 | 32.26 | 31.95 | 32.12 | 32.05 |
| **NSC2** | 32.24 | 32.40 | 32.13 | 33.98 | 32.09 | 32.20 |
| **NSC3** | 32.17 | 32.33 | 32.18 | 32.39 | 32.26 | 32.20 |
| **PC** | 32.30 | 32.62 | 32.18 | 32.51 | 32.50 | 32.24 |

### ETGA ΔCt (averageNSC)

| | ***E. coli*** | | ***S. aureus*** | | ***C. albicans*** | |
|---|---|---|---|---|---|---|
| | **(+) BEADS** | **(-) BEADS** | **(+) BEADS** | **(-) BEADS** | **(+) BEADS** | **(-) BEADS** |
| **D1** | 22.64 | 6.33 | 16.55 | 3.39 | 8.19 | 1.22 |
| **D2** | 17.19 | 3.56 | 11.26 | N/A | 4.69 | N/A |
| **D3** | 12.54 | 0.47 | 6.93 | N/A | 1.49 | N/A |
| **D4** | 4.77 | 1.57 | 4.69 | N/A | 3.97 | N/A |
| **NSC1** | N/A | N/A | N/A | N/A | N/A | N/A |
| **NSC2** | N/A | N/A | N/A | N/A | N/A | N/A |
| **NSC3** | N/A | N/A | N/A | N/A | N/A | N/A |
| **PC** | 3.18 | 1.79 | 4.14 | 3.04 | 3.08 | 2.48 |

### Critical Values based on average NSC (cfu/mL)

| | ***E. coli*** | | ***S. aureus*** | | ***C. albicans*** | |
|---|---|---|---|---|---|---|
| | **(+) BEADS** | **(-) BEADS** | **(+) BEADS** | **(-) BEADS** | **(+) BEADS** | **(-) BEADS** |
| **D1** | 0.03 | 2692.80 | 0.07 | 641.54 | 1.74 | 218.60 |
| **D2** | 0.14 | 1836.42 | 0.27 | N/A | 1.98 | N/A |
| **D3** | 0.36 | 1557.76 | 0.55 | N/A | 1.81 | N/A |
| **D4** | 7.93 | 72.82 | 0.26 | N/A | 0.03 | N/A |
| **NSC1** | N/A | N/A | N/A | N/A | N/A | N/A |
| **NSC2** | N/A | N/A | N/A | N/A | N/A | N/A |
| **NSC3** | N/A | N/A | N/A | N/A | N/A | N/A |
| **PC** | N/A | N/A | N/A | N/A | N/A | N/A |

### Confirm Ct

| | ***E. coli* (GrNeg)** | | ***S. aureus* (GrPos)** | | ***C. albicans* (Candida)** | |
|---|---|---|---|---|---|---|
| | **(+) BEADS** | **(-) BEADS** | **(+) BEADS** | **(-) BEADS** | **(+) BEADS** | **(-) BEADS** |
| **D1** | 24.26 | 42.44 | 28.13 | NoCt | 35.08 | NoCt |
| **D2** | 27.83 | 32.08 | 31.65 | NoCt | 39.30 | 43.21 |
| **D3** | 33.17 | NoCt | 36.79 | NoCt | NoCt | NoCt |
| **D4** | NoCt | NoCt | 36.71 | NoCt | NoCt | NoCt |
| **NSC1** | NoCt | NoCt | NoCt | 41.51 | NoCt | 48.84 |
| **NSC2** | 40.57 | NoCt | NoCt | NoCt | NoCt | NoCt |
| **NSC3** | NoCt | NoCt | NoCt | 40.49 | NoCt | NoCt |
| **PC** | NoCt | NoCt | 43.24 | NoCt | NoCt | NoCt |

| | | | | | | |
|---|---|---|---|---|---|---|
| Positivity threshold (Pt) ≤ 40 Ct | | | | | | |

### Analysis:

- Magnitor results for '(+) BEADS' samples show a very strong cell-density specific ETGA and Confirm signal for all three microorganism species, demonstrating bead-specific binding of a broad range of microorganism groups (GrNeg, GrPos, Candida).
- Note, that Candida results could have followed a better cell density trend, but the liquid culture was quite particulate which may have affected the quality of serial dilutions
- Some evidence of microbial cell carryover in the '(-) BEADS' controls, but this is to be expected with only a single wash step.

### Example 5: Microbial capture from blood by magnetic beads occurs in simple and complex blood lysis buffers, and allows microbial detection comparable to capture by centrifugation

### Aim:

To compare two different blood lysis buffers in two different diluent formats for development of a simple and fast 'Rapid Magnitor' test (no wash step included in protocol).

### Test Conditions:

| | | |
|---|---|---|
| • | 2X EBB | 1 mL 2X EBB + 1 mL Specimen |
| • | 10X EBB | 112 µL 10X EBB + 1 mL Specimen |
| • | 2X B-BUF | 1 mL 2X B-BUF + 1 mL Specimen |
| • | 10X B-BUF | 112 µL 10X B-BUF + 1 mL Specimen |

- 10X EBB: 500 mM Tris-HCl [pH 8.0] + 2.5% Tergitol
- 10X B-BUF: 500 mM Tris-HCl [pH 8.0] + 1.5 M Sodium Chloride + 10% Igepal + 5% Sodium Deoxycholate + 2.5% Tergitol

### Sample set-up:

- *E. coli* o/n liquid culture 1E-3 dilution spiked into blood broth (6.25 µL o/n per mL: 244 µL o/n + 39 mL blood broth) and 60 minute out-growth performed in shaking incubator @ 37°C, 500 rpm
- Following 2-hour outgrowth, samples produced by adding 1 mL specimen to 2 mL tube containing buffer (and 15 µL BioEstapor beads (Merck, Cat# BE-M 08/0.3) for Mag Beads sample-set): triplicate *E*. *coli* (EC) and No Spike Control (NSC) samples per test condition
- 100 µL TVCs performed for NSC and *E. coli* (including dilutions of specimen to ensure countable plates)

### Protocol:

Samples set up as above, and progressed immediately to Spin or Mag Beads protocol

### Spin Protocol

- Samples centrifuged for 3 minutes at 9000 *xg* (tube hinges facing outwards for pellet traceability)
- Supernatants removed
- 50 µL LM added to samples (approx. 10x pipette mixes to resuspend pellets)
- Samples placed in shaking incubator at 900 rpm for 5 minutes and then 800 rpm for 55 minutes (26°C)
- Centrifuge samples at 17000 *xg* for 1 minute before qPCR set-up

### Mag Beads Protocol

- Samples placed in shaking incubator at 900 rpm for 30 minutes (37°C)
- Samples placed on DynaMag-2 magnetic rack for 5 minutes and then supernatants removed
- 50 µL LM added to samples (approx. 10x pipette mixes to resuspend pellets)
- Samples placed in shaking incubator at 900 rpm for 5 minutes and then 800 rpm for 55 minutes (26°C)
- Magnetise samples for 3 minutes before qPCR set-up
   Manual qPCR set-up (10 µL reactions) for ETGA mastermix only

### Results:

### cfu calculation

### Sample and dilutions of sample plated on COL plates (100 µL)

| | **TVC** | ***cfu/mL** |
|---|---|---|
| *E. coli* 1E-3 | TNTC | 68500 |
| *E. coli* 1E-4 | 685 | 6850 |
| *E. coli* 1E-5 | 89 | 685 |
| NSC | 0 | 0 |

### Sample source

### ETGA Ct

| | **Centrifugation** | | | | | **Magnetic Beads** | | | |
|---|---|---|---|---|---|---|---|---|---|
| | **cfu/sample** | **2XEBB** | **10XEBB** | **2XB-BUF** | **10XB-BUF** | **2XEBB** | **10XEBB** | **2XB-BUF** | **10XB-BUF** |
| ***E. coli 1*** | 68,500 | 15.89 | 16.94 | 21.43 | 22.83 | 15.10 | 17.12 | 20.78 | 22.61 |
| ***E. coli 2*** | 68,500 | 15.63 | 16.87 | 22.72 | 21.82 | 15.35 | 17.50 | 21.07 | 23.21 |
| ***E. coli 3*** | 68,500 | 16.05 | 16.85 | 20.04 | 22.20 | 15.69 | 17.72 | 21.77 | 23.20 |
| **NSC1** | - | 23.72 | 25.48 | 47.72 | NoCt | 29.52 | 34.45 | 45.73 | 44.89 |
| **NSC2** | - | 24.51 | 25.01 | 43.55 | 46.85 | 30.02 | 34.30 | 46.30 | 44.26 |
| **NSC3** | - | 24.16 | 25.74 | NoCt | 44.62 | 30.08 | 34.21 | 48.06 | 45.06 |

### Summary data

| | **Centrifugation** | | | | | **Magnetic Beads** | | | |
|---|---|---|---|---|---|---|---|---|---|
| | **cfu/sample** | **2XEBB** | **10XEBB** | **2XB-BUF** | **10XB-BUF** | **2XEBB** | **10XEBB** | **2XB-BUF** | **10XB-BUF** |
| **Ave. *E. coli* ETGA Ct** | 68,500 | 15.86 | 16.89 | 21.40 | 22.28 | 15.38 | 17.45 | 21.21 | 23.01 |
| **Ave.NSC ETGA Ct** | - | 24.13 | 25.41 | 45.64 | 45.73 | 29.88 | 34.32 | 46.70 | 44.74 |
| **Ave. ETGA ΔCt** | 68,500 | 8.27 | 8.52 | 24.24 | 23.45 | 14.49 | 16.87 | 25.49 | 21.73 |

### Analysis:

- Microbial binding by magnetic beads occurs in:
   ∘ Simple and complex blood lysis buffers (EBB = Tris-HCl + Tergitol; B-BUF = Tris-HCl + Sodium Chloride + Igepal + Sodium Deoxycholate + Tergitol), but blood-derived test signal varies depending on blood lysis buffer components
   ∘ Diluted (2X buffer: 1-part blood lysis buffer to 1-part specimen) and concentrated (10X buffer: 1-part blood lysis buffer to 9 parts specimen) sample formats

Furthermore, microbial detection signal for microbial capture by magnetic beads is comparable to capture by centrifugation.

### Example 6: Microbial capture from blood by magnetic beads is not dependent on blood lysis, but downstream microbial detection is improved when microbial binding occurs in lysed blood

### Aim:

Given the recent discovery that multiple bead types/sizes produce similar Magnitor results for microbial serial dilutions and NSCs, it was thought that a component within Momentum's binding buffer might be mediating/facilitating this observed universal microbial binding character. To investigate this possibility, a dilution series of *E. coli* was performed comparing the standard binding buffer (B-BUF) with a detergent-free B-BUF consisting of just Tris-HCl [pH8.0] + NaCl, to test whether the detergents in general are important for microbial binding. Sample-sets were prepared for blood-broth, broth-only and in 1X binding buffer only to compare results for different specimen types.

### Preparation:

100 mL 10X Binding Buffers prepared fresh:
- **B-BUF:** 500 mM Tris-HCl [pH 8.0] + 1.5 M Sodium Chloride + 10% Igepal + 5% Sodium Deoxycholate + 2.5% Tergitol
- **Tris+NaCl:** 500 mM Tris-HCl [pH 8.0] + 1.5 M Sodium Chloride

Estapor beads (Merck, Cat# M1-30/40) washed 3 x 1 mL in respective 1X buffer (diluted 10X B-BUF or 10X Tris+NaCl): 40 µL beads resuspended in a final volume of 400 µL 1X buffer (1% solid content)

### Protocol:

- *E. coli* o/n liquid culture set-up as standard in BacTec PLUS aerobic broth, then following day (approx. 16 hours later) 1.88 µL o/n added to 3 mL broth (equivalent to EC 1E-1 dilution added to broth at 6.25 µL/mL) and 2-hour outgrowth performed at 37°C, 500 rpm.
- Following 2-hour outgrowth, *E. coli* preculture serially diluted (DF10) down to EC 1E-6 in either prewarmed blood-broth (BB), broth only (BO) or 1X buffer (B-BUF or Tris+NaCl).
- 100 µL TVCs performed for all *E. coli* dilutions and NSCs

### Manual simulation of Magnitor performed using DynaMag-2 magnet and manual liquid transfers:

- 1 mL samples added to 2 mL tubes containing 112 µL of binding buffer (either B-BUF or Tris+NaCl: 10X for BB and BO sample-sets; and 1X for Buffer sample-sets) + 15 µL beads (prewashed in respective buffer)
- 30 mins shaking (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 50 µL LM added to tubes off magnet (5 µL Polymerase Control (PC) added to each PC sample tube)
- ETGA reaction performed: 5 mins at 1000 rpm, then 55 mins at 800 rpm @26°C
- Manual qPCR set-up for ETGA and Confirm (10 µL reactions)

### Observations:

- No blood lysis observed for Tris+NaCl, as expected
- Beads more grainy/aggregated in absence of detergents

### Results:

| *10X Binding Buffer* | BB | | BO | | 1X B-BUF | | Tris+NaCl | |
|---|---|---|---|---|---|---|---|---|
| *Specimen* | **TVC** | ***E cfu/mL** | **TVC** | ***E cfu/mL** | **TVC** | ***E cfu/mL** | **TVC** | ***E cfu/mL** |
| *E. coli* 1E-3 | TNTC | 74800 | TNTC | 52000 | TNTC | 47600 | TNTC | 54700 |
| *E. coli* 1E-4 | *748 | 7480 | *520 | 5200 | *476 | 4760 | *547 | 5470 |
| *E. coli* 1E-5 | 88 | 748 | 70 | 520 | 45 | 476 | 57 | 547 |
| *E. coli* 1E-6 | 6 | 75 | 5 | 52 | 2 | 48 | 5 | 55 |
| NSC | 0 | 0 | 0 | 0 | 0 | 0 | 0 | 0 |

| | | | | | | | | |
|---|---|---|---|---|---|---|---|---|
| **Cfu*/*mL values extrapolated from highest countable TVC* | | | | | | | | |

### ETGA Ct

| ***10X Binding*** | **B-BUF** | | | **Tris+NaCl** | | |
|---|---|---|---|---|---|---|
| ***Specimen*** | **BB** | **BO** | **B-BUF** | **BB** | **BO** | **Tris+NaCl** |
| ***E. coli* 1E-3** | 22.84 | 21.63 | 18.93 | 24.71 | 17.78 | 14.91 |
| ***E. coli* 1E-4** | 27.32 | 26.16 | 25.08 | 29.70 | 21.58 | 20.14 |
| ***E. coli* 1E-5** | 31.40 | 30.03 | 29.31 | 33.58 | 25.38 | 24.53 |
| ***E. coli* 1E-6** | 36.87 | 32.91 | 33.62 | 34.97 | 29.36 | 31.24 |
| **NSC 1** | 44.80 | 35.23 | 34.71 | 36.04 | 38.81 | 33.73 |
| **NSC 2** | 43.10 | 34.86 | 35.26 | 35.21 | 38.83 | 34.89 |
| **NSC 3** | 44.11 | 34.54 | 36.72 | 35.41 | 38.56 | 34.19 |
| **PC** | 34.84 | 28.06 | 31.37 | 34.08 | 32.78 | 30.55 |
| **Average NS** | 44.00 | 34.87 | 35.56 | 35.55 | 38.74 | 34.27 |

### IPC Ct

| ***10X Binding*** | **B-BUF** | | | **Tris+NaCl** | | |
|---|---|---|---|---|---|---|
| ***Specimen*** | **BB** | **BO** | **B-BUF** | **BB** | **BO** | **Tris+NaCl** |
| ***E. coli* 1E-3** | 33.89 | 31.43 | 32.14 | 33.59 | 32.15 | 33.36 |
| ***E. coli* 1E-4** | 34.41 | 31.22 | 31.67 | 33.63 | 31.66 | 31.68 |
| ***E. coli* 1E-5** | 33.44 | 31.27 | 31.41 | 32.85 | 31.35 | 31.27 |
| ***E. coli* 1E-6** | 33.27 | 31.34 | 31.46 | 33.16 | 31.11 | 31.49 |
| **NSC 1** | 34.06 | 31.29 | 31.43 | 33.26 | 31.34 | 31.30 |
| **NSC 2** | 35.07 | 31.53 | 31.69 | 33.18 | 31.50 | 31.51 |
| **NSC 3** | 33.79 | 31.29 | 31.54 | 33.25 | 31.11 | 31.77 |
| **PC** | 34.22 | 31.29 | 32.18 | 33.34 | 31.37 | 31.65 |

### ETGA ΔCt (average NSC)

| ***10X Binding*** | **B-BUF** | | | **Tris+NaCl** | | |
|---|---|---|---|---|---|---|
| ***Specimen*** | **BB** | **BO** | **B-BUF** | **BB** | **BO** | **Tris+NaCl** |
| ***E. coli* 1E-3** | 21.16 | 13.25 | 16.63 | 10.85 | 20.95 | 19.35 |
| ***E. coli* 1E-4** | 16.68 | 8.71 | 10.49 | 5.86 | 17.15 | 14.13 |
| ***E. coli* 1E-5** | 12.61 | 4.84 | 6.25 | 1.97 | 13.35 | 9.74 |
| ***E. coli* 1E-6** | 7.14 | 1.97 | 1.94 | 0.58 | 9.38 | 3.03 |
| **NSC 1** | N/A | N/A | N/A | N/A | N/A | N/A |
| **NSC 2** | N/A | N/A | N/A | N/A | N/A | N/A |
| **NSC 3** | N/A | N/A | N/A | N/A | N/A | N/A |
| **PC** | 9.16 | 6.82 | 4.20 | 1.47 | 5.96 | 3.72 |

### Critical Values based on average NSC (cfu/mL)

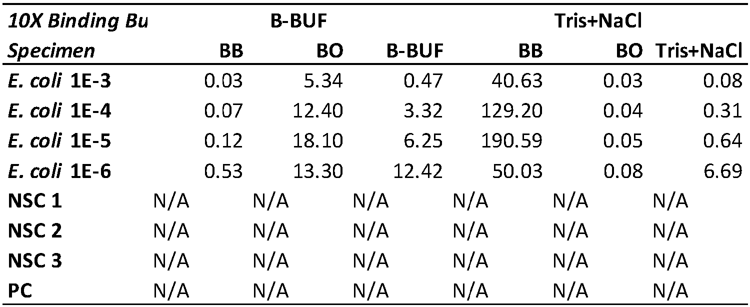

### Confirm GrNeg Ct

| ***10X Binding*** | **B-BUF** | | | **Tris+NaCl** | | |
|---|---|---|---|---|---|---|
| ***Specimen*** | **BB** | **BO** | **B-BUF** | **BB** | **BO** | **Tris+NaCl** |
| ***E. coli* 1E-3** | 31.36 | NoCt | 28.13 | 30.53 | 31.60 | 25.94 |
| ***E. coli* 1E-4** | 36.41 | NoCt | 30.86 | 35.72 | 38.16 | 26.69 |
| ***E. coli* 1E-5** | 37.67 | NoCt | NoCt | NoCt | 39.26 | 34.62 |
| ***E. coli* 1E-6** | NoCt | NoCt | NoCt | NoCt | NoCt | NoCt |
| **NSC 1** | NoCt | NoCt | NoCt | 42.97 | NoCt | NoCt |
| **NSC 2** | NoCt | NoCt | NoCt | NoCt | 39.64* | NoCt |
| **NSC 3** | NoCt | NoCt | NoCt | 49.52 | NoCt | NoCt |
| **PC** | NoCt | NoCt | NoCt | NoCt | NoCt | NoCt |

| | | | | | | |
|---|---|---|---|---|---|---|
| Positivity threshold (Pt) ≤ 40 Ct; * false positives | | | | | | |

### Analysis:

- Detergents are important for producing good ETGA results in the presence of blood (as indicated by poorer ETGA results for '10X Tris+NaCl with BB'), but microbial capture/detection is still evident in the absence of blood lysis (as demonstrated by results for '10X Tris+NaCl with BB' sample-set).
- Good ETGA results in the absence of blood, indicate that detergents, as components of the binding buffer, are not necessary for binding of *E. coli* to beads
- Good ETGA results in the '10X Tris+NaCl with 1X Tris+NaCl' sample-set demonstrate that biological components in blood and/or broth are not required for microbial binding.
- Interestingly, the B-BUF appears to be slightly inhibitory to ETGA signal in 10X B-BUF with BO and 1X B-BUF sample-sets, but recent work elsewhere has shown that Sodium Deoxycholate could be somewhat inhibitory to the assay, so this observation is not unexpected
- Confirm performed best in the '10X Tris+NaCl with 1X Tris+NaCl' sample-set. All other similar sample-sets produced similar Confirm GrNeg results.
- IPC signal was somewhat inhibited by the presence of blood, as can be expected.
- These results demonstrate that neither detergents or the biological sample are mediators of microbial binding for *E. coli*

### .Example 7: In the absence of blood, microbial capture by magnetic beads occurs regardless of any pH buffering or osmotic stabilisation with salt

### Aim:

To further investigate the importance of Momentum's binding buffer in mediating microbial binding the effect of pH buffering and salt on binding was investigated in a clean system (i.e. in the absence of any blood or broth).

### 10X buffer preparation:

25 mL of each buffer made fresh:
- BUF-1 500 mM Tris-HCl [pH7.4] + 1.5 M NaCl
- BUF-2 500 mM Tris-HCl [pH8.0] + 1.5 M NaCl
- BUF-3 500 mM Tris-HCl [pH8.5] + 1.5 M NaCl
- BUF-4 500 mM Tris-HCl [pH8.0] ONLY
- BUF-5 1.5 M NaCl ONLY
- BUF-6 Water ONLY

Estapor beads (Merck,Cat M1-30/40) washed 3 x 1 mL in respective 1X buffer (diluted 10X buffers): 30 µL beads resuspended in a final volume of 300 µL 1X buffer (1% solid content)

### Protocol:

- E.coli o/n liquid cultures set-up as standard in BacTec PLUS aerobic broth (containing SPS) and Nutrient Broth (NB containing no SPS), then the following day (approx. 16 hours later) 1.88 µL o/n added to 3 mL broth (equivalent to EC 1E-1 dilution added to broth at 6.25 µL/mL) for each broth type (NB in morning and PLUS broth in afternoon), and 2-hour outgrowth incubations performed at 37°C, 500 rpm.
- For each experiment (NB and PLUS), 1E-1 *E. coli* preculture diluted down to *E. coli* 1E-6 (DF10) in each 1X buffer (BUF-1 to BUF-6)
- 100 µL TVCs performed using a separate EC dilution set performed in relevant broth (NB or PLUS broth) to prevent plate viability inconsistencies resulting from different 1X buffers

### Manual simulation of Magnitor performed using DynaMag-2 magnet and manual liquid transfers:

- 1 mL samples added to 2 mL tubes containing 112 µL of respective 1X buffer + 15 µL beads (prewashed in respective buffer)
- 30 mins shaking (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

### NB dataset (i.e. No SPS) - morning experiment

| | **Colonies** | ***E cfu/mL** |
|---|---|---|
| ***E. coli* 1e-3** | TNTC | 35000 |
| ***E. coli* 1e-4** | *350 | 3500 |
| ***E. coli* 1e-5** | 12 | 350 |
| ***E. coli* 1e-6** | 5 | 35 |

| | | |
|---|---|---|
| *All buffer (BUF-1 to 6) NC TVCs = 0* | | |

| | | | **ETGA Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 35000 | 19.00 | 18.44 | 17.73 | 17.45 | 19.12 | 16.28 |
| ***E. coli* 1e-4** | *350 | 3500 | 22.60 | 21.23 | 21.53 | 21.16 | 22.51 | 26.22 |
| ***E. coli* 1e-5** | 12 | 350 | 25.57 | 26.94 | 28.18 | 24.77 | 27.51 | 28.71 |
| ***E. coli* 1e-6** | 5 | 35 | 30.41 | 30.47 | 31.37 | 29.92 | 31.36 | 33.81 |
| **NSC 1** | 0 | 0 | 40.37 | 39.45 | 39.70 | 40.18 | 39.22 | 38.16 |
| **NSC 2** | 0 | 0 | 40.63 | 36.20 | 39.74 | 38.03 | 40.01 | 38.64 |
| **NSC 3** | 0 | 0 | 39.76 | 39.36 | 39.09 | 39.67 | 38.47 | 38.17 |
| **PC** | N/A | N/A | 30.24 | 31.51 | 33.06 | 32.57 | 32.90 | 32.08 |
| **NSC Ave.** | | | 40.25 | 38.34 | 39.51 | 39.30 | 39.23 | 38.32 |

| | | | **IPC Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 35000 | 32.17 | 32.36 | 32.32 | 32.30 | 32.05 | 32.31 |
| ***E. coli* 1e-4** | *350 | 3500 | 31.58 | 31.62 | 31.83 | 31.67 | 31.55 | 31.28 |
| ***E. coli* 1e-5** | 12 | 350 | 31.44 | 31.65 | 31.44 | 31.27 | 31.67 | 31.62 |
| ***E. coli* 1e-6** | 5 | 35 | 31.37 | 31.47 | 31.48 | 31.31 | 31.64 | 31.50 |
| **NSC 1** | 0 | 0 | 31.60 | 31.25 | 31.42 | 31.64 | 31.48 | 31.54 |
| **NSC 2** | 0 | 0 | 31.53 | 31.33 | 31.60 | 31.40 | 31.75 | 31.52 |
| **NSC 3** | 0 | 0 | 31.42 | 31.95 | 31.37 | 31.43 | 31.76 | 31.67 |
| **PC** | N/A | N/A | 31.20 | 31.49 | 31.65 | 31.49 | 31.57 | 31.97 |

| | | | **ETGA ΔCt** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 35000 | 21.26 | 19.89 | 21.78 | 21.85 | 20.11 | 22.05 |
| ***E. coli* 1e-4** | *350 | 3500 | 17.65 | 17.10 | 17.98 | 18.13 | 16.73 | 12.10 |
| ***E. coli* 1e-5** | 12 | 350 | 14.68 | 11.40 | 11.34 | 14.53 | 11.72 | 9.61 |
| ***E. coli* 1e-6** | 5 | 35 | 9.85 | 7.86 | 8.14 | 9.38 | 7.87 | 4.51 |
| **NSC 1** | 0 | 0 | N/A | N/A | N/A | N/A | N/A | N/A |
| **NSC 2** | 0 | 0 | N/A | N/A | N/A | N/A | N/A | N/A |
| **NSC 3** | 0 | 0 | N/A | N/A | N/A | N/A | N/A | N/A |
| **PC** | N/A | N/A | 10.02 | 6.83 | 6.45 | 6.72 | 6.33 | 6.24 |

| | | | **ETGA Critical Values (averageNSC)** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 35000 | 0.014 | 0.036 | 0.010 | 0.009 | 0.031 | 0.008 |
| ***E. coli* 1e-4** | *350 | 3500 | 0.017 | 0.025 | 0.014 | 0.012 | 0.032 | 0.795 |
| ***E. coli* 1e-5** | 12 | 350 | 0.013 | 0.130 | 0.135 | 0.015 | 0.104 | 0.448 |
| ***E. coli* 1e-6** | 5 | 35 | 0.038 | 0.150 | 0.124 | 0.053 | 0.149 | 1.535 |
| **NSC 1** | 0 | 0 | N/A | N/A | N/A | N/A | N/A | N/A |
| **NSC 2** | 0 | 0 | N/A | N/A | N/A | N/A | N/A | N/A |
| **NSC 3** | 0 | 0 | N/A | N/A | N/A | N/A | N/A | N/A |
| **PC** | N/A | N/A | N/A | N/A | N/A | N/A | N/A | N/A |

| | | | **Confirm GrNeg Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 35000 | 25.07 | 25.28 | 25.98 | 25.77 | 25.05 | 27.70 |
| ***E. coli* 1e-4** | *350 | 3500 | 28.36 | 28.58 | 28.31 | 30.15 | 30.62 | NoCt |
| ***E. coli* 1e-5** | 12 | 350 | 31.75 | 31.16 | 31.82 | 32.25 | 38.38 | NoCt |
| ***E. coli* 1e-6** | 5 | 35 | NoCt | NoCt | 34.94 | NoCt | NoCt | NoCt |
| **NSC 1** | 0 | 0 | NoCt | NoCt | NoCt | NoCt | NoCt | NoCt |
| **NSC 2** | 0 | 0 | NoCt | NoCt | NoCt | NoCt | NoCt | NoCt |
| **NSC 3** | 0 | 0 | NoCt | NoCt | NoCt | NoCt | NoCt | NoCt |
| **PC** | N/A | N/A | NoCt | NoCt | NoCt | NoCt | NoCt | NoCt |

| | | | | | | | | |
|---|---|---|---|---|---|---|---|---|
| Positivity threshold (Pt) ≤ 40 Ct | | | | | | | | |

### PLUS dataset (i.e with SPS) - afternoon experiment

| | **Colonies** | ***E cfu/mL** |
|---|---|---|
| ***E. coli* 1e-3** | TNTC | 55600 |
| ***E. coli* 1e-4** | *556 | 5560 |
| ***E. coli* 1e-5** | 77 | 556 |
| ***E. coli* 1e-6** | 7 | 55.6 |

### All buffer (BUF-1 to 6) NC TVCs = 0

| | | | **ETGA Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 55600 | 17.30 | 16.32 | 15.48 | 18.29 | 19.15 | 17.61 |
| ***E. coli* 1e-4** | *556 | 5560 | 21.89 | 21.09 | 20.37 | 22.37 | 24.39 | 26.13 |
| ***E. coli* 1e-5** | 77 | 556 | 26.82 | 24.63 | 26.19 | 26.30 | 29.27 | 34.26 |
| ***E. coli* 1e-6** | 7 | 55.6 | 31.76 | 30.71 | 32.60 | 31.57 | 34.20 | 34.68 |
| **NSC 1** | 0 | 0 | 37.74 | 39.10 | 38.95 | 41.09 | 41.03 | 41.46 |
| **NSC 2** | 0 | 0 | 38.25 | 38.48 | 38.90 | 39.11 | 40.58 | 40.84 |
| **NSC 3** | 0 | 0 | 39.46 | 38.90 | 38.03 | 42.26 | 40.81 | 41.02 |
| **PC** | N/A | N/A | 33.25 | 32.41 | 32.62 | 33.85 | 33.49 | 33.26 |
| **NSC Ave.** | | | 38.48 | 38.83 | 38.63 | 40.82 | 40.81 | 41.11 |

| | | | **IPC Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 55600 | 32.46 | 33.44 | 32.97 | 33.31 | 31.98 | 33.00 |
| ***E. coli* 1e-4** | *556 | 5560 | 31.77 | 32.07 | 32.00 | 32.25 | 31.42 | 31.64 |
| ***E. coli* 1e-5** | 77 | 556 | 32.01 | 32.03 | 31.96 | 31.70 | 31.21 | 31.15 |
| ***E. coli* 1e-6** | 7 | 55.6 | 31.96 | 31.65 | 31.75 | 31.55 | 31.55 | 31.63 |
| **NSC 1** | 0 | 0 | 31.84 | 32.11 | 31.99 | 31.13 | 31.58 | 31.65 |
| **NSC 2** | 0 | 0 | 31.90 | 31.51 | 32.08 | 31.25 | 31.46 | 31.64 |
| **NSC 3** | 0 | 0 | 31.94 | 31.84 | 31.68 | 31.49 | 31.44 | 31.59 |
| **PC** | N/A | N/A | 32.17 | 32.24 | 32.34 | 31.73 | 31.37 | 31.87 |

| | | | **ETGA ΔCt** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 55600 | 21.18 | 22.50 | 23.15 | 22.53 | 21.66 | 23.50 |
| ***E. coli* 1e-4** | *556 | 5560 | 16.59 | 17.73 | 18.26 | 18.45 | 16.41 | 14.98 |
| ***E. coli* 1e-5** | 77 | 556 | 11.66 | 14.20 | 12.44 | 14.52 | 11.54 | 6.85 |
| ***E. coli* 1e-6** | 7 | 55.6 | 6.72 | 8.12 | 6.03 | 9.25 | 6.61 | 6.43 |
| **NSC 1** | 0 | 0 | N/A | N/A | N/A | N/A | N/A | N/A |
| **NSC 2** | 0 | 0 | N/A | N/A | N/A | N/A | N/A | N/A |
| **NSC 3** | 0 | 0 | N/A | N/A | N/A | N/A | N/A | N/A |
| **PC** | N/A | N/A | 5.23 | 6.41 | 6.01 | 6.97 | 7.31 | 7.85 |

| | | | **ETGA Critical Values (averageNSC)** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 55600 | 0.023 | 0.009 | 0.006 | 0.009 | 0.017 | 0.005 |
| ***E. coli* 1e-4** | *556 | 5560 | 0.056 | 0.026 | 0.018 | 0.016 | 0.064 | 0.172 |
| ***E. coli* 1e-5** | 77 | 556 | 0.172 | 0.030 | 0.100 | 0.024 | 0.187 | 4.827 |
| ***E. coli* 1e-6** | 7 | 55.6 | 0.528 | 0.200 | 0.849 | 0.092 | 0.569 | 0.644 |
| **NSC 1** | 0 | 0 | N/A | N/A | N/A | N/A | N/A | N/A |
| **NSC 2** | 0 | 0 | N/A | N/A | N/A | N/A | N/A | N/A |
| **NSC 3** | 0 | 0 | N/A | N/A | N/A | N/A | N/A | N/A |
| **PC** | N/A | N/A | N/A | N/A | N/A | N/A | N/A | N/A |

| | | | **Confirm GrNeg Ct** | | | | | |
|---|---|---|---|---|---|---|---|---|
| | **Colonies** | ***E cfu/mL** | **BUF-1** | **BUF-2** | **BUF-3** | **BU F-4** | **BUF-5** | **BUF-6** |
| ***E. coli* 1e-3** | TNTC | 55600 | 28.12 | 27.49 | 26.73 | 28.36 | 28.13 | 27.82 |
| ***E. coli* 1e-4** | *556 | 5560 | 32.91 | 27.92 | 27.85 | 30.15 | 30.85 | NoCt |
| ***E. coli* 1e-5** | 77 | 556 | 34.43 | 34.83 | 38.66 | 43.88 | NoCt | NoCt |
| ***E. coli* 1e-6** | 7 | 55.6 | NoCt | NoCt | NoCt | NoCt | NoCt | NoCt |
| **NSC 1** | 0 | 0 | NoCt | NoCt | NoCt | NoCt | NoCt | NoCt |
| **NSC 2** | 0 | 0 | NoCt | NoCt | NoCt | NoCt | NoCt | NoCt |
| **NSC 3** | 0 | 0 | NoCt | NoCt | NoCt | NoCt | NoCt | NoCt |
| **PC** | N/A | N/A | NoCt | NoCt | NoCt | NoCt | NoCt | NoCt |

| | | | | | | | | |
|---|---|---|---|---|---|---|---|---|
| Positivity threshold (Pt) ≤ 40 Ct | | | | | | | | |

### Analysis:

- All buffers, including water only, demonstrated capture of *E. coli* similarly well (as indicated by similar ETGA and Confirm results) - however, there was some indication of osmotic microbial lysis at lower cell densities in water only (BUF-6)
- Both PLUS broth and NB grown *E. coli* produced very similar Magnitor results for all buffers tested, indicating that SPS plays no obvious role in mediating microbial binding of E. *coli*
- These results indicate that no buffer components are essential for binding of *E. coli* to Estapor (carboxylated) beads

### Example 8: Microbial capture from blood by magnetic beads can be performed using a variety of different blood lysis methods

### Aim:

To determine whether microbial capture and detection can occur when alternative blood lysis methods are employed.

### Preparation:

Binding Buffers were prepared as follows:
- **E-BUF** = 500 mM Tris-HCl [pH 8.0] + 1.5 M Sodium Chloride + 10% Igepal + 2.5% Tergitol
- **UREA** = 83 mM Tris-HCl [pH 8.0] + 10 M Urea
- **Tris+NaCl** = 500 mM Tris-HCl [pH 8.0] + 1.5 M Sodium Chloride

BioEstapor beads (Merck, Cat# BE-M 08/0.3) were re-suspended prior to use.

### Protocol:

- *S. aureus* o/n liquid culture set-up as standard in BacTec PLUS aerobic broth, then following day (approx. 16 hours later) 3.0 µL o/n added to 3 mL blood broth (1E-3 dilution) and 4-hour outgrowth performed at 37°C, 500 rpm.
- Following the 4-hour outgrowth, *S. aureus* pre-culture serially diluted (DF10) down to 1E-6 in prewarmed blood broth.
- 100 µL TVCs performed for all *S. aureus* dilutions and NSCs

### Manual sample processing using DynaMag-2 magnet and manual liquid transfers by pipette:

### Initial set-up

- For samples using Urea, 0.25 mL specimens added to 2 mL tubes containing 0.75 mL of UREA + 15 µL beads
- For samples to be frozen, 1 mL specimens added to 2 mL tubes then snap frozen on dry ice for 5 minutes. The specimens were thawed at 37°C for 5 minutes, then 112 µL Tris+NaCl + 15 µL beads were added
- For samples using E-BUF or Tris+NaCl, 1 mL specimens added to 2 mL tubes containing 112 µL of binding buffer (either E-BUF or Tris+NaCl) + 15 µL beads

### Processing of all samples

- 30 mins orbital mixing (1000 rpm) @ 37°C
- 5 mins magnetisation on DynaMag-2
- All s/n removed
- 1 mL WB added and tubes mixed for 3 mins @ 37°C (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Observations:

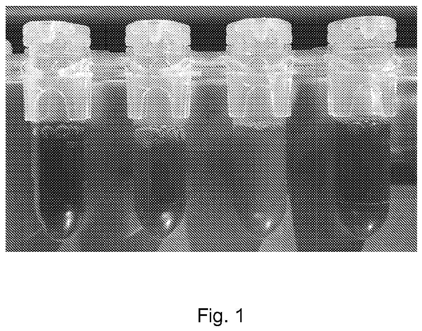

- Blood lysis observed for the frozen samples after thawing (see Figure 1)
- Blood lysis observed to be almost instantaneous with UREA
- Some beads appeared to be lost during processing for the samples using UREA
- No apparent blood lysis observed for Tris+NaCl sample-set (as expected)

### Results:

| *Specimen* | TVC | *E cfu/mL |
|---|---|---|
| *S. aureus* 1E-4 | TNTC | 3,360,000 |
| *S. aureus* 1E-5 | TNTC | 336,000 |
| *S. aureus* 1E-6 | *3360 | 33,600 |
| NSC | 0 | 0 |

| | | |
|---|---|---|
| **Cfu*/*mL values extrapolated from highest countable TVC* | | |

### ETGA Ct

| | **BLOOD LYSIS METHOD** | | | |
|---|---|---|---|---|
| ***Specimen*** | **E-BUF** | **UREA** | **Tris+NaCl** | **FREEZING** |
| ***S. aureus* 1E-4** | 14.26 | 34.98 | 13.90 | 14.42 |
| ***S. aureus* 1E-5** | 18.50 | 30.26 | 17.79 | 18.99 |
| ***S. aureus* 1E-6** | 22.59 | 35.96 | 22.07 | 22.96 |
| **NSC 1** | 42.11 | 37.55 | 32.56 | 35.14 |
| **NSC 2** | 41.13 | 36.52 | 32.72 | 34.60 |
| **NSC 3** | 43.83 | 36.71 | 32.42 | 34.58 |
| **Average NSC** | 42.36 | 36.92 | 32.57 | 34.77 |

### ETGA ΔCt (average NSC)

| | **BLOOD LYSIS METHOD** | | | |
|---|---|---|---|---|
| ***Specimen*** | **E-BUF** | **UREA** | **Tris+NaCl** | **FREEZING** |
| ***S. aureus* 1E-4** | 28.10 | 1.94 | 18.67 | 20.35 |
| ***S. aureus* 1E-5** | 23.85 | 6.66 | 14.78 | 15.78 |
| ***S. aureus* 1E-6** | 19.77 | 0.96 | 10.49 | 11.82 |
| **NSC 1** | N/A | N/A | N/A | N/A |
| **NSC 2** | N/A | N/A | N/A | N/A |
| **NSC 3** | N/A | N/A | N/A | N/A |

### Critical Values based on average NSC (cfu/mL)

| | **BLOOD LYSIS METHOD** | | | |
|---|---|---|---|---|
| ***Specimen*** | **E-BUF** | **UREA** | **Tris+NaCl** | **FREEZING** |
| ***S. aureus* 1E-4** | 0.01 | 875527.09 | 8.06 | 2.52 |
| ***S. aureus* 1E-5** | 0.02 | 3313.90 | 11.96 | 5.96 |
| ***S. aureus* 1E-6** | 0.04 | 17241.36 | 23.34 | 9.31 |
| **NSC 1** | N/A | N/A | N/A | N/A |
| **NSC 2** | N/A | N/A | N/A | N/A |
| **NSC 3** | N/A | N/A | N/A | N/A |

### IPC Ct

| | **BLOOD LYSIS METHOD** | | | |
|---|---|---|---|---|
| ***Specimen*** | **E-BUF** | **UREA** | **Tris+NaCl** | **FREEZING** |
| ***S. aureus* 1E-4** | 35.68 | 36.10 | 37.31 | 38.12 |
| ***S. aureus* 1E-5** | 34.49 | 36.18 | 35.39 | 35.54 |
| ***S. aureus* 1E-6** | 34.22 | 35.76 | 35.12 | 34.50 |
| **NSC 1** | 34.40 | 36.51 | 34.39 | 34.87 |
| **NSC 2** | 34.18 | 35.86 | 34.52 | 34.09 |
| **NSC 3** | 34.85 | 36.31 | 34.25 | 34.04 |

### Confirm GrPos Ct

| | **BLOOD LYSIS METHOD** | | | |
|---|---|---|---|---|
| ***Specimen*** | **E-BUF** | **UREA** | **Tris+NaCl** | **FREEZING** |
| ***S. aureus* 1E-4** | 22.34 | 23.47 | 18.74 | 19.55 |
| ***S. aureus* 1E-5** | 26.03 | 26.33 | 21.19 | 23.03 |
| ***S. aureus* 1E-6** | 27.67 | 30.92 | 24.73 | 26.37 |
| **NSC 1** | 46.59 | 35.42* | NoCt | 48.06 |
| **NSC 2** | 40.33 | NoCt | NoCt | NoCt |
| **NSC 3** | NoCt | NoCt | 45.99 | 41.75 |

| | | | | |
|---|---|---|---|---|
| Positivity threshold (Pt) ≤ 40 Ct; * false positives | | | | |

Figure 1 shows the extent of blood lysis for each sample-set: E-BUF, UREA, Tris+NaCl, freezing (left to right)

### Analysis:

- Microbial capture and detection of *S. aureus* by magnetic beads is comparable for alternative lysis methods and no blood lysis, as determined by Confirm.

However, microbial detection by ETGA is improved to differing extents by alternative blood lysis methods, due to effects on the reduction of blood-derived ETGA signal.

### Example 9: SPS is needed for optimal bead performance, sample processing and microbial detection in whole blood

### Aim:

To determine the optimal SPS concentration for the Magnitor Rapid test using 1 mL whole blood samples. The secondary objective was to assess the effect of SPS on microbial viability in whole blood as determined by TVCs.

### Test conditions:

2 x 5 mL whole blood or BacTec PLUS aerobic blood broth (1:3 ratio) aliquoted for each sample-set (E. *coli and* NSC sample). Then SPS added as follows:

| **Sample-set** | **10% SPS (µL)** |
|---|---|
| BB | None |
| WB 0% | None |
| WB 0.01% | 5 |
| WB 0.02% | 10 |
| WB 0.04% | 20 |
| WB 0.06% | 30 |
| WB 0.08% | 40 |
| WB 0.10% | 50 |

Then 5 µL of *E. coli* 1E-2 preculture added to each 5-mL specimen tube to recreate standard 1E-5 dilution sample

### Protocol:

- 1.88 µL neat o/n in Nutrient Broth (NB) added to 3 mL NB; and incubated for 2 hours at 37°C, 500 rpm
- After 2 hours, *E. coli* preculture diluted 10-fold in warm NB; and then 5 µL added to each 5 mL specimen tube (prepared as shown in test conditions)
- Magnitor Test initiated immediately, and TVCs performed as detailed below.

### Manual simulation of Magnitor performed using DynaMag-2 magnet and manual liquid transfers:

- 112 µL E-BUF (500 mM Tris-HCl [pH 8.0] + 1.5 M Sodium Chloride + 10% Igepal + 2.5% Tergitol) + 15 µL Beads (BioEstapor, Merck, Cat# BE-M 08/0.3) preloaded into each sample-tube, then 1 mL specimens added to sample tubes
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

### TVC analysis

- 100 µL on COL plates at Time Zero
- Sample bijous, containing approximately 2 mL sample, left at room temperature (20.4°C) on bench (static)
- TVCs performed at time points shown in table: samples mixed thoroughly before plating

| | | **WB SPS % (colonies)** | | | | | | |
|---|---|---|---|---|---|---|---|---|
| | **BB** | **0** | **0.01** | **0.02** | **0.04** | **0.06** | **0.08** | **0.1** |
| **EC Time ZERO** | 263 | 303 | 147 | 96 | 354 | 128 | 322 | 126 |
| **EC Time 2hrs** | 428 | 256 | 498 | 448 | 1106 | 760 | 491 | 341 |
| **EC Time 4hrs** | TNTC | 790 | TNTC | TNTC | TNTC | TNTC | TNTC | TNTC |
| **EC Time 6hrs** | TNTC | TNTC | TNTC | TNTC | TNTC | TNTC | TNTC | TNTC |
| **EC Time 21hrs** | LAWN | LAWN | LAWN | LAWN | LAWN | LAWN | LAWN | LAWN |
| **NSC Time ZERO** | 0 | 0 | N/A | N/A | N/A | N/A | N/A | N/A |
| **NSC Time 2hrs** | 0 | 0 | N/A | N/A | N/A | N/A | N/A | N/A |
| **NSC Time 4hrs** | 0 | 0 | N/A | N/A | N/A | N/A | N/A | N/A |
| **NSC Time 6hrs** | 0 | 0 | N/A | N/A | N/A | N/A | N/A | N/A |
| **NSC Time 21hrs** | 0 | 0 | N/A | N/A | N/A | N/A | N/A | N/A |
| **FOLD INCR. 0-2hr** | 1.63 | 0.84 | 3.39 | 4.67 | 3.12 | 5.94 | 1.52 | 2.71 |

### Magnitor Rapid test performed at Time ZERO

### ETGA results

| | | **WB SPS %** | | | | | | |
|---|---|---|---|---|---|---|---|---|
| | **BB** | **0** | **0.01** | **0.02** | **0.04** | **0.06** | **0.08** | **0.1** |
| **EC 1** | 26.94 | *28.72 | 27.90 | 26.41 | 29.31 | 28.70 | 32.31 | 33.76 |
| **EC 2** | 27.31 | 45.22 | 29.12 | 25.88 | 29.43 | 28.50 | 31.94 | 34.37 |
| **EC 3** | 27.34 | 41.32 | 28.14 | 27.24 | 29.70 | 27.62 | 31.40 | 34.08 |
| **NSC 1** | 39.06 | 43.71 | 33.39 | 33.20 | 38.93 | 44.62 | 50.00 | 50.00 |
| **NSC 2** | 39.75 | 44.30 | 33.06 | 33.77 | 39.93 | 44.11 | 50.00 | 50.00 |
| **NSC 3** | 40.28 | 46.58 | 32.81 | 34.41 | 38.54 | 43.30 | 50.00 | 50.00 |
| **Ave. EC** | 27.20 | 43.27 | 28.39 | 26.51 | 29.48 | 28.27 | 31.88 | 34.07 |
| **Ave. NSC** | 39.70 | 44.86 | 33.09 | 33.79 | 39.13 | 44.01 | 50.00 | 50.00 |
| **Ave. ΔCt** | 12.50 | 1.59 | 4.70 | 7.28 | 9.65 | 15.74 | 18.12 | 15.93 |
| **CV (cfu/mL)** | 0.45 | 1003.27 | 56.59 | 6.16 | 4.40 | 0.02 | 0.01 | 0.02 |

| | | | | | | | | |
|---|---|---|---|---|---|---|---|---|
| *Note: NoCt changed to 50 Ct for analysis; *Outlier excluded due to substantial pellet loss during processing* | | | | | | | | |

### IPC results

| | | **WB SPS %** | | | | | | |
|---|---|---|---|---|---|---|---|---|
| | **BB** | **0** | **0.01** | **0.02** | **0.04** | **0.06** | **0.08** | **0.1** |
| **EC 1** | 34.03 | 42.50 | 42.19 | 40.42 | 37.87 | 35.81 | 38.14 | 37.06 |
| **EC 2** | 34.83 | NoCt | 39.34 | 39.01 | 38.38 | 35.70 | 36.80 | 37.11 |
| **EC 3** | 34.47 | NoCt | 40.49 | 37.85 | 38.34 | 36.11 | 36.80 | 37.66 |
| **NSC 1** | 34.15 | NoCt | 39.75 | 39.26 | 36.56 | 36.10 | 36.80 | 37.17 |
| **NSC 2** | 34.00 | NoCt | 39.12 | 38.40 | 37.81 | 35.98 | 37.87 | 38.60 |
| **NSC 3** | 34.18 | 45.91 | 40.58 | 37.32 | 36.90 | 36.36 | 38.09 | 37.46 |
| **Ave. NSC** | 34.11 | 45.91 | 39.81 | 38.33 | 37.09 | 36.15 | 37.59 | 37.74 |

### Confirm GrNeg results

| | | **WB SPS %** | | | | | | |
|---|---|---|---|---|---|---|---|---|
| | **BB** | **0** | **0.01** | **0.02** | **0.04** | **0.06** | **0.08** | **0.1** |
| **EC 1** | 32.33 | 30.05 | 27.94 | 29.15 | 28.71 | 31.58 | 29.20 | 29.27 |
| **EC 2** | 31.45 | 34.24 | 29.18 | 28.16 | 28.75 | 29.77 | 29.80 | 28.65 |
| **EC 3** | 31.71 | 34.97 | 28.32 | 29.75 | 29.82 | 29.26 | 28.87 | 29.61 |
| **NSC 1** | NoCt | NoCt | NoCt | NoCt | NoCt | NoCt | NoCt | 47.82 |
| **NSC 2** | 43.07 | NoCt | NoCt | NoCt | NoCt | NoCt | NoCt | NoCt |
| **NSC 3** | 47.16 | NoCt | NoCt | NoCt | NoCt | NoCt | NoCt | NoCt |
| **Ave. EC** | 31.83 | 33.08 | 28.48 | 29.02 | 29.09 | 30.20 | 29.29 | 29.18 |

### Analysis:

- SPS indicates a benefit of providing microbial protection/viability in whole blood based on TVC assay; but no major issues with *E. coli* viability in whole blood generally
- Incorporation of SPS improved sample processing efficacy and microbial detection performance for both ETGA and Confirm readouts
- SPS at 0.06% produced the best results for TVC-based viability; ETGA detection (best results taking *E. coli* and NSC sample Cts into account); and PCR inhibition as indicated by IPC Ct values. Confirm GrNeg results were also improved by SPS addition to whole blood, but the exact concentration of SPS was less critical.

### Example 10: Microbial capture from blood by magnetic beads occurs using a variety of commercially available carboxylated bead products of similar size (~300 nm diameter)

### Aim:

To compare alternative carboxylated magnetic beads of similar size using Momentum's Magnitor assay

### Test conditions:

| I.D | Bead | Size (µm) |
|---|---|---|
| A | PS-MAG-COOH (microparticles GmbH #S2003) | 0.27 |
| B | Sphero Carboxyl magnetic particles (Spherotech #CM-025-10H) | 0.1 - 0.4 |
| C | SuperMag Carboxylic Acid Beads (Ocean Nanotech #SC0201) | 0.2 |
| D | Carboxyl-Adembeads (Ademtech #02120) | 0.2 |
| E | Carboxyl beads (FG Beads #TAS8848N1140) | 0.2 |
| G | Bio-Estapor (Merck #BE-M08/03) - streptavidin-conjugated | 0.3 |

### Protocol:

*E. coli and S. pyogenes* o/n liquid cultures set up as standard in 3 mL broth and blood broth (BacTec PLUS aerobic) respectively, and incubated for 16-20 hours (37°C)

The following day:
- *E. coli* liquid culture diluted to 1E-3 in blood broth and then spiked into blood broth (6.25 µL per mL blood broth); and pre-incubated for 1hr 30mins (37°C)
- *S. pyogenes* liquid culture diluted to 1E-1 in blood broth and then spiked into blood broth (6.25 µL per mL blood broth); and pre-incubated for 2hr 30mins (37°C)

### E. coli experiment performed in morning

- *E. coli* 1E-3 pre-culture serially diluted in blood broth to produce 5 dilution points (1E-3 to 1E-7)
- Samples set up by adding 1 mL specimen to 2 mL tubes preloaded with 15 µl 1% solid beads + 112 µL Binding Buffer; and Magnitor V4.0 test performed: 5 dilution points + 3 NSCs (8 sample-set) per bead type with three bead types tested on each epMotion 5073m

### S. pyogenes experiment performed in afternoon

- *S. pyogenes* 1E-3 pre-culture serially diluted in blood broth to produce 5 dilution points (1E-1 to 1E-5)
- Samples set up by adding 1 mL specimen to 2 mL tubes preloaded with 15 µl 1% solid beads + 112 µL Binding Buffer; and Magnitor V4.0 test performed: 5 dilution points + 3 NSCs (8 sample-set) per bead type with three bead types tested on each epMotion 5073m

### Magnitor V4.0 protocol (automated sample processing on epMotion 5073m)

- 30 mins orbital mixing (1000 rpm) @ 37°C
- 15 mins magnetisation
- 1 mL s/n removed
- 0.82 mL WB added to tubes whilst beads magnetised
- 1 mL s/n removed
- 50 µL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 µL reactions)

### Results:

Internal Positivity Thresholds (Pt) calculated using NSCs (n= 6) for each bead type: formula = PERCENTILE.INC(array,0.05)

| | **A** | **B** | **C** | **D** | **E** | **G** |
|---|---|---|---|---|---|---|
| **NSC1** | 35.12 | 37.10 | 41.11 | 45.29 | 43.55 | 40.33 |
| **NSC2** | 35.00 | 38.85 | 42.73 | 46.18 | 43.15 | 40.37 |
| **NSC3** | 34.57 | 37.02 | 40.49 | 50.00 | 42.32 | 41.79 |
| **NSC4** | 33.77 | 38.25 | 41.20 | 44.74 | 43.82 | 43.80 |
| **NSC5** | 33.72 | 38.22 | 40.52 | 45.25 | 45.08 | 44.12 |
| **NSC6** | 35.06 | 41.19 | 41.93 | 44.33 | 43.32 | 42.39 |
| **Pt (5th%)** | **33.74** | **37.04** | **40.49** | **44.44** | **42.53** | **40.34** |

| | | | | | | |
|---|---|---|---|---|---|---|
| *Note, that Pt (5th%) method will generate one false positive within n=6: highlighted in red font within main results table below* | | | | | | |

### E. coli

| | | **ETGA** | | | | | **Confirm** | | | |
|---|---|---|---|---|---|---|---|---|---|---|
| | | **TVCs** | ***E CFU/ml** | **ETGA Ct** | **Result** | **IPC Ct** | **GrNeg Ct** | **GrPos Ct** | **Cand. Ct** | **I.D.** |
| **A** | ***E. coli* 1e-3** | TNTC | 28,800 | 27.20 | Positive | 35.15 | 30.99 | NoCt | NoCt | GrNeg |
| | ***E. coli* 1e-4** | *288 | 2,880 | 31.30 | Positive | 34.91 | NoCt | NoCt | NoCt | No ID |
| | ***E. coli* 1e-5** | 35 | 288 | 33.31 | Positive | 35.08 | NoCt | NoCt | NoCt | No ID |
| | ***E. coli* 1e-6** | 4 | 29 | 35.47 | Negative | 35.37 | NoCt | NoCt | NoCt | No ID |
| | ***E. coli* 1e-7** | 0 | 3 | 34.63 | Negative | 35.09 | NoCt | NoCt | NoCt | No ID |
| | **NSC1** | 0 | - | 35.12 | Negative | 35.67 | NoCt | NoCt | NoCt | No ID |
| | **NSC2** | 0 | - | 35.00 | Negative | 35.10 | NoCt | NoCt | NoCt | No ID |
| | **NSC3** | 0 | - | 34.57 | Negative | 35.27 | NoCt | NoCt | NoCt | No ID |
| **B** | ***E. coli* 1e-3** | TNTC | 28,800 | 25.67 | Positive | 34.45 | 36.51 | NoCt | NoCt | GrNeg |
| | ***E. coli* 1e-4** | *288 | 2,880 | 31.31 | Positive | 34.02 | NoCt | NoCt | NoCt | No ID |
| | ***E. coli* 1e-5** | 35 | 288 | 32.92 | Positive | 34.74 | NoCt | NoCt | NoCt | No ID |
| | ***E. coli* 1e-6** | 4 | 29 | 36.56 | Positive | 34.26 | NoCt | NoCt | NoCt | No ID |
| | ***E. coli* 1e-7** | 0 | 3 | 38.68 | Negative | 34.46 | NoCt | NoCt | NoCt | No ID |
| | **NSC1** | 0 | - | 37.10 | Negative | 33.86 | NoCt | NoCt | NoCt | No ID |
| | **NSC2** | 0 | - | 38.85 | Negative | 34.28 | NoCt | NoCt | NoCt | No ID |
| | **NSC3** | 0 | - | 37.02^{†} | Positive | 33.95 | 40.26 | NoCt | NoCt | No ID |
| **C** | ***E. coli* 1e-3** | TNTC | 28,800 | 23.59 | Positive | 35.38 | 29.32 | NoCt | NoCt | GrNeg |
| | ***E. coli* 1e-4** | *288 | 2,880 | 27.50 | Positive | 35.16 | 34.29 | NoCt | NoCt | GrNeg |
| | ***E. coli* 1e-5** | 35 | 288 | 31.34 | Positive | 35.16 | 42.48 | NoCt | NoCt | No ID |
| | ***E. coli* 1e-6** | 4 | 29 | 32.91 | Positive | 35.29 | NoCt | NoCt | NoCt | No ID |
| | ***E. coli* 1e-7** | 0 | 3 | 40.91 | Negative | 35.61 | NoCt | NoCt | NoCt | No ID |
| | **NSC1** | 0 | - | 41.11 | Negative | 34.63 | NoCt | NoCt | NoCt | No ID |
| | **NSC2** | 0 | - | 42.73 | Negative | 35.09 | 49.10 | NoCt | NoCt | No ID |
| | **NSC3** | 0 | - | 40.49^{†} | Positive | 35.58 | NoCt | NoCt | NoCt | No ID |
| **D** | ***E. coli* 1e-3** | TNTC | 28,800 | 24.41 | Positive | 35.51 | 31.63 | NoCt | NoCt | GrNeg |
| | ***E. coli* 1e-4** | *288 | 2,880 | 28.00 | Positive | 34.72 | NoCt | NoCt | NoCt | No ID |
| | ***E. coli* 1e-5** | 35 | 288 | 32.33 | Positive | 35.39 | NoCt | NoCt | NoCt | No ID |
| | ***E. coli* 1e-6** | 4 | 29 | 33.46 | Positive | 34.66 | NoCt | NoCt | NoCt | No ID |
| | ***E. coli* 1e-7** | 0 | 3 | 48.92 | Negative | 35.09 | NoCt | NoCt | NoCt | No ID |
| | **NSC1** | 0 | - | 45.29 | Negative | 35.40 | NoCt | NoCt | NoCt | No ID |
| | **NSC2** | 0 | - | 46.18 | Negative | 35.11 | NoCt | NoCt | NoCt | No ID |
| | **NSC3** | 0 | - | 50.00 | Negative | 34.70 | NoCt | NoCt | NoCt | No ID |
| **E** | ***E. coli* 1e-3** | TNTC | 28,800 | 22.77 | Positive | 35.70 | 30.68 | NoCt | NoCt | GrNeg |
| | ***E. coli* 1e-4** | *288 | 2,880 | 27.16 | Positive | 35.49 | 39.05 | NoCt | NoCt | GrNeg |
| | ***E. coli* 1e-5** | 35 | 288 | 33.84 | Positive | 34.91 | NoCt | NoCt | NoCt | No ID |
| | ***E. coli* 1e-6** | 4 | 29 | 42.64 | Negative | 35.16 | NoCt | NoCt | NoCt | No ID |
| | ***E. coli* 1e-7** | 0 | 3 | 42.40 | Positive | 35.16 | NoCt | NoCt | NoCt | No ID |
| | **NSC1** | 0 | - | 43.55 | Negative | 35.30 | NoCt | NoCt | NoCt | No ID |
| | **NSC2** | 0 | - | 43.15 | Negative | 34.55 | NoCt | NoCt | NoCt | No ID |
| | **NSC3** | 0 | - | 42.32^{†} | Positive | 34.19 | NoCt | NoCt | NoCt | No ID |
| **G** | ***E. coli* 1e-3** | TNTC | 28,800 | 22.91 | Positive | 36.48 | 32.84 | NoCt | NoCt | GrNeg |
| | ***E. coli* 1e-4** | *288 | 2,880 | 27.54 | Positive | 35.61 | NoCt | NoCt | NoCt | No ID |
| | ***E. coli* 1e-5** | 35 | 288 | 31.36 | Positive | 35.86 | 42.94 | NoCt | NoCt | No ID |
| | ***E. coli* 1e-6** | 4 | 29 | 41.03 | Negative | 35.44 | NoCt | NoCt | NoCt | No ID |
| | ***E. coli* 1e-7** | 0 | 3 | 42.24 | Negative | 35.45 | NoCt | NoCt | NoCt | No ID |
| | **NSC1** | 0 | - | 40.33^{†} | Positive | 35.78 | NoCt | 43.85 | NoCt | No ID |
| | **NSC2** | 0 | - | 40.37 | Negative | 35.31 | NoCt | NoCt | NoCt | No ID |
| | **NSC3** | 0 | - | 41.79 | Negative | 36.90 | NoCt | 38.72^{†} | NoCt | GrPos |

| | | | | | | | | | | |
|---|---|---|---|---|---|---|---|---|---|---|
| Confirm Positivity threshold (Pt) ≤ 40 Ct; false positives^{†} | | | | | | | | | | |

### S. pyogenes

| | | | **ETGA** | | | | **Confirm** | | | |
|---|---|---|---|---|---|---|---|---|---|---|
| | | **TVCs** | ***E CFU/ml** | **ETGA Ct** | **Result** | **IPC Ct** | **GrNeg Ct** | **GrPos Ct** | **Cand. Ct** | **I.D.** |
| **A** | ***S. pyogenes* 1e-1** | TNTC | 2,210,000 | 26.20 | Positive | 34.95 | NoCt | 27.74 | NoCt | GrPos |
| | ***S. pyogenes* 1e-2** | TNTC | 221,000 | 30.05 | Positive | 34.52 | NoCt | 31.28 | NoCt | GrPos |
| | ***S. pyogenes* 1e-3** | TNTC | 22,100 | 32.49 | Positive | 34.77 | NoCt | 35.19 | NoCt | GrPos |
| | ***S. pyogenes* 1e-4** | *221 | 2,210 | 33.67 | Positive | 35.15 | NoCt | NoCt | NoCt | No ID |
| | ***S. pyogenes* 1e-5** | 3 | 221 | 33.30 | Positive | 34.33 | NoCt | NoCt | NoCt | No ID |
| | **NSC1** | 0 | - | 33.77 | Negative | 35.22 | NoCt | NoCt | NoCt | No ID |
| | **NSC2** | 0 | - | 33.72^{†} | Positive | 35.09 | NoCt | NoCt | NoCt | No ID |
| | **NSC3** | 0 | - | 35.06 | Negative | 34.93 | NoCt | NoCt | NoCt | No ID |
| **B** | ***S. pyogenes* 1e-1** | TNTC | 2,210,000 | 30.57 | Positive | 33.56 | NoCt | 32.72 | NoCt | GrPos |
| | ***S. pyogenes* 1e-2** | TNTC | 221,000 | 34.19 | Positive | 33.45 | NoCt | 38.13 | NoCt | GrPos |
| | ***S. pyogenes* 1e-3** | TNTC | 22,100 | 36.31 | Positive | 34.20 | NoCt | NoCt | NoCt | No ID |
| | ***S. pyogenes* 1e-4** | *221 | 2,210 | 37.67 | Negative | 33.58 | NoCt | NoCt | NoCt | No ID |
| | ***S. pyogenes* 1e-5** | 3 | 221 | 36.79 | Positive | 33.44 | NoCt | NoCt | NoCt | No ID |
| | **NSC1** | 0 | - | 38.25 | Negative | 34.07 | 43.83 | NoCt | NoCt | No ID |
| | **NSC2** | 0 | - | 38.22 | Negative | 33.86 | NoCt | NoCt | NoCt | No ID |
| | **NSC3** | 0 | - | 41.19 | Negative | 34.23 | NoCt | NoCt | NoCt | No ID |
| **C** | ***S. pyogenes* 1e-1** | TNTC | 2,210,000 | 26.21 | Positive | 35.04 | NoCt | 26.56 | NoCt | GrPos |
| | ***S. pyogenes* 1e-2** | TNTC | 221,000 | 30.61 | Positive | 34.96 | NoCt | 30.38 | NoCt | GrPos |
| | ***S. pyogenes* 1e-3** | TNTC | 22,100 | 34.17 | Positive | 35.19 | NoCt | 34.06 | NoCt | GrPos |
| | ***S. pyogenes* 1e-4** | *221 | 2,210 | 38.99 | Positive | 34.78 | NoCt | NoCt | NoCt | No ID |
| | ***S. pyogenes* 1e-5** | 3 | 221 | 41.83 | Negative | 34.25 | NoCt | NoCt | NoCt | No ID |
| | **NSC1** | 0 | - | 41.20 | Negative | 34.91 | NoCt | NoCt | NoCt | No ID |
| | **NSC2** | 0 | - | 40.52 | Negative | 35.07 | 46.11 | NoCt | NoCt | No ID |
| | **NSC3** | 0 | - | 41.93 | Negative | 34.18 | 43.77 | NoCt | NoCt | No ID |
| **D** | ***S. pyogenes* 1e-1** | TNTC | 2,210,000 | 27.51 | Positive | 35.47 | NoCt | 28.26 | NoCt | GrPos |
| | ***S. pyogenes* 1e-2** | TNTC | 221,000 | 31.66 | Positive | 35.00 | NoCt | 30.96 | NoCt | GrPos |
| | ***S. pyogenes* 1e-3** | TNTC | 22,100 | 34.50 | Positive | 35.42 | NoCt | 34.86 | NoCt | GrPos |
| | ***S. pyogenes* 1e-4** | *221 | 2,210 | 38.78 | Positive | 35.53 | NoCt | 42.04 | NoCt | No ID |
| | ***S. pyogenes* 1e-5** | 3 | 221 | 43.26 | Positive | 35.40 | NoCt | NoCt | NoCt | No ID |
| | **NSC1** | 0 | - | 44.74 | Negative | 34.96 | NoCt | NoCt | NoCt | No ID |
| | **NSC2** | 0 | - | 45.25 | Negative | 34.55 | NoCt | NoCt | NoCt | No ID |
| | **NSC3** | 0 | - | 44.33^{†} | Positive | 35.20 | NoCt | NoCt | NoCt | No ID |
| **E** | ***S. pyogenes* 1e-1** | TNTC | 2,210,000 | 26.32 | Positive | 34.59 | NoCt | 27.87 | NoCt | GrPos |
| | ***S. pyogenes* 1e-2** | TNTC | 221,000 | 30.65 | Positive | 35.46 | NoCt | 30.83 | NoCt | GrPos |
| | ***S. pyogenes* 1e-3** | TNTC | 22,100 | 34.55 | Positive | 35.19 | NoCt | 35.01 | NoCt | GrPos |
| | ***S. pyogenes* 1e-4** | *221 | 2,210 | 39.11 | Positive | 35.15 | NoCt | 41.25 | NoCt | No ID |
| | ***S. pyogenes* 1e-5** | 3 | 221 | 43.96 | Negative | 35.59 | NoCt | 46.72 | NoCt | No ID |
| | **NSC1** | 0 | - | 43.82 | Negative | 36.22 | NoCt | NoCt | NoCt | No ID |
| | **NSC2** | 0 | - | 45.08 | Negative | 35.37 | NoCt | 42.20 | NoCt | No ID |
| | **NSC3** | 0 | - | 43.32 | Negative | 35.31 | NoCt | NoCt | NoCt | No ID |
| **G** | ***S. pyogenes* 1e-1** | TNTC | 2,210,000 | 26.36 | Positive | 36.24 | NoCt | 28.73 | NoCt | GrPos |
| | ***S. pyogenes* 1e-2** | TNTC | 221,000 | 30.74 | Positive | 36.09 | NoCt | 32.02 | NoCt | GrPos |
| | ***S. pyogenes* 1e-3** | TNTC | 22,100 | 34.60 | Positive | 35.96 | NoCt | 35.34 | NoCt | GrPos |
| | ***S. pyogenes* 1e-4** | *221 | 2,210 | 39.37 | Positive | 35.53 | NoCt | 42.28 | NoCt | No ID |
| | ***S. pyogenes* 1e-5** | 3 | 221 | 41.78 | Negative | 34.66 | NoCt | 33.29 | NoCt | GrPos |
| | **NSC1** | 0 | - | 43.80 | Negative | 35.29 | NoCt | 35.27^{†} | NoCt | GrPos |
| | **NSC2** | 0 | - | 44.12 | Negative | 35.37 | NoCt | 38.56^{†} | NoCt | GrPos |
| | **NSC3** | 0 | - | 42.39 | Negative | 35.96 | NoCt | NoCt | NoCt | No ID |

| | | | | | | | | | | |
|---|---|---|---|---|---|---|---|---|---|---|
| Confirm Positivity threshold (Pt) ≤ 40 Ct; false positives^{†} | | | | | | | | | | |

### Observations:

- Bead B difficult to resuspend before diluting down to 1% solid content; and appeared visually more dilute after dilution to 1% solid
- At the end of processing, samples were placed on DynaMag-2 magnetic rack, and all bead types C-G magnetised similarly apart from: Bead A, which appeared to have heavy pellets; and Bead B, which had very small bead pellets

### Analysis:

All carboxylated magnetic beads tested here demonstrate microbial binding as determined by ETGA and Confirm readouts. However, the sensitivity of microbial detection does vary somewhat, depending on the level of blood-derived ETGA signal and/or assay inhibition

### Example 11: Microbial capture from blood by magnetic beads occurs using a variety of different bead sizes and functional coatings

### Aim:

To compare microbial capture performance for a variety of commercially-available magnetic beads of different size and functional coating using Momentum's Magnitor test (ETGA and Confirm technologies). Two experiments were performed to demonstrate microbial capture for automated (Protocol 1) and manual (Protocol 2) sample processing. Importantly, Protocol 2 included three bead resuspension washes to more convincingly demonstrate that ETGA/Confirm signal is specific to bead-bound microbial cells, rather than sample carryover (as opposed to Protocol 1, which included a single beads-magnetised wash step).

### Test conditions:

| Heading | Description | Product | Diameter (µm) | Ferrite % | Polymer |
|---|---|---|---|---|---|
| COOH-0.2 | Very Small Estapor^{®} Carboxylated Nanospheres (-COOH) | Merck #M1-020/50 | 0.160-0.240 | >50 | Polystyrene |
| COOH-1.0 | Original Estapor^{®} Carboxylated Microspheres (-COOH) | Merck #M1-070/40 | 0.700-1.300 | 35-45 | Polystyrene |
| HYDRO-1.0 | Original Estapor^{®} Hydrophobic Microspheres | Merck #MS-070/40 | 0.700-1.300 | 35-50 | Polystyrene |
| NH2-1.5 | Original Estapor^{®} Aminated Microspheres (-NH2) | Merck #M2-070/40 | 1.000-2.000 | 35-45 | Polystyrene |
| Peps6 | Magnetic beads covered with Peps6 | ApoH Technologies Ltd #MP20006 | 0.200 | Unknown | Unknown |
| Speed | SpeedBeads^{™} magnetic carboxylate modified particles (two layers of magnetite) | GE Healthcare #65152105050250 | 1.000 | 40 | Polystyrene |
| BioEsta | Streptavidin coated Small Estapor^{®} Carboxylated Nanospheres (-COOH) | Merck #BE-M08/03 | 0.251-0.400 | 40-60 | Polystyrene |

All beads washed in 1 mL 1X E-BUF (50 mM Tris-HCl [pH 8.0] + 150 mM Sodium Chloride + 1% Igepal + 0.25% Tergitol) and resuspended to 1% solid in 1X E-BUF

### Sample set-up (performed separately for Protocol 1 and 2 which were performed on different days):

- *E. coli* o/n liquid cultures set up as standard in 3 mL broth (BacTec PLUS aerobic) and incubated for 16-20 hours (37°C)
- The following day, *E. coli* liquid culture diluted to 1E-3 in blood broth and 2-hour outgrowth incubation performed (37°C @ 500 rpm)
- Following the 2-hour outgrowth incubation, *E. coli* 1E-3 pre-culture serially diluted in blood broth to produce three dilution points (EC 1E-6 to 1E-8)
- 1 mL specimens (three *E. coli* dilutions and three NSC samples: 6 sample-set) added to 2 mL sample tubes preloaded with 112 µL 10X E-BUF (500 mM Tris-HCl [pH 8.0] + 1.5 M Sodium Chloride + 10% Igepal + 2.5% Tergitol) + 15 µL beads (1% solid), then Magnitor test initiated according to either Protocol 1 or Protocol 2 (see below):

### Protocol 1 (automated sample processing on epMotion 5073m):

- 30 mins orbital mixing (1000 rpm) @ 37°C
- 15 mins magnetisation
- 1 mL s/n removed
- 0.82 mL WB added to tubes whilst beads magnetised
- 1 mL s/n removed
- 50 µL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 µL reactions)

### Protocol 2 (manual sample processing using DynaMag-2 magnet and manual liquid transfers by pipette):

- 30 mins orbital mixing (1000 rpm) @ 37°C
- 5 mins magnetisation on DynaMag-2
- All s/n removed
- 1 mL WB added and tubes mixed for 2 mins @ RT (1000 rpm)
- 5 mins magnetisation on Dynmag-2
- All s/n removed
- 1 mL WB added and tubes mixed for 2 mins @ RT (1000 rpm)
- 5 mins magnetisation on Dynmag-2
- All s/n removed
- 1 mL WB added and tubes mixed for 2 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

### Protocol 1 (automated sample processing on epMotion 5073m) - performed on 20190221

| | | | **ETGA Ct** | | | | | | |
|---|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** | **Peps6** |
| ***E. coli* 1E-6** | TNTC | 18500 | 20.97 | 20.13 | 21.01 | 21.36 | 21.98 | 21.01 | 19.85 |
| ***E. coli* 1E-7** | *185 | 1850 | 25.28 | 24.23 | 26.43 | 24.61 | 25.06 | 25.46 | 26.27 |
| ***E. coli* 1E-8** | 12 | 185 | 27.56 | 28.64 | 30.54 | 27.24 | 30.63 | 30.59 | 28.63 |
| **NSC 1** | 0 | 0 | 38.19 | 36.32 | 38.73 | 37.86 | 39.18 | 37.94 | 40.26 |
| **NSC 2** | 0 | 0 | 38.33 | 36.16 | 39.41 | 37.19 | 38.90 | 39.22 | 38.82 |
| **NSC 3** | 0 | 0 | 34.34 | 35.78 | 37.67 | 36.76 | 40.05 | 39.16 | 39.42 |

| | | | **Confirm GrNeg Ct** | | | | | | |
|---|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** | **Peps6** |
| ***E. coli* 1E-6** | TNTC | 18500 | 32.56 | 30.75 | 33.89 | 32.42 | 30.51 | 32.63 | 46.19 |
| ***E. coli* 1E-7** | *185 | 1850 | 40.82 | 33.67 | NoCt | 31.99 | 33.47 | NoCt | NoCt |
| ***E. coli* 1E-8** | 12 | 185 | 39.21 | NoCt | NoCt | 39.38 | NoCt | NoCt | NoCt |
| **NSC 1** | 0 | 0 | NoCt | 42.19 | 40.84 | 44.46 | 40.55 | NoCt | NoCt |
| **NSC 2** | 0 | 0 | NoCt | 40.61 | 47.17 | NoCt | NoCt | NoCt | NoCt |
| **NSC 3** | 0 | 0 | NoCt | 37.42^{†} | 41.04 | NoCt | NoCt | NoCt | NoCt |

| | | | | | | | | | |
|---|---|---|---|---|---|---|---|---|---|
| Positivity threshold (Pt) ≤ 40 Ct; false positives^{†} | | | | | | | | | |

| | | | **IPC Ct** | | | | | | |
|---|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** | **Peps6** |
| ***E. coli* 1E-6** | TNTC | 18500 | 35.89 | 35.14 | 35.72 | 36.28 | 36.06 | 36.07 | 34.86 |
| ***E. coli* 1E-7** | *185 | 1850 | 35.98 | 35.14 | 34.74 | 36.15 | 35.08 | 35.59 | 35.63 |
| ***E. coli* 1E-8** | 12 | 185 | 34.56 | 34.83 | 34.93 | 36.15 | 34.72 | 35.40 | 34.82 |
| **NSC 1** | 0 | 0 | 34.45 | 34.83 | 34.56 | 35.91 | 35.55 | 35.49 | 34.74 |
| **NSC 2** | 0 | 0 | 34.51 | 34.71 | 35.13 | 36.07 | 34.92 | 35.04 | 34.42 |
| **NSC 3** | 0 | 0 | 35.04 | 34.85 | 34.45 | 35.62 | 35.62 | 35.34 | 34.85 |

### Protocol 2 (manual sample processing using DynaMag-2 magnet and manual liquid transfers by pipette) -performed on 20190228

| | | | | | **ETGA Ct** | | | |
|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** |
| ***E. coli* 1E-6** | *724 | 7240 | 23.97 | 25.45 | 24.80 | 26.29 | 25.95 | 24.32 |
| ***E. coli* 1E-7** | 76 | 724 | 27.64 | 30.33 | 29.14 | 31.52 | 30.34 | 27.35 |
| ***E. coli* 1E-8** | 7 | 72 | 32.20 | 30.81 | 36.25 | 32.91 | 29.64 | 31.54 |
| **NSC 1** | 0 | 0 | 40.47 | 39.30 | 40.27 | 34.36 | 43.39 | 41.61 |
| **NSC 2** | 0 | 0 | 42.23 | 37.84 | 41.03 | 33.96 | 43.47 | 41.70 |
| **NSC 3** | 0 | 0 | 42.74 | 39.66 | 41.53 | 34.34 | 43.27 | 40.19 |

| | | | | **Confirm GrNeg Ct** | | | | |
|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** |
| ***E. coli* 1E-6** | *724 | 7240 | 27.84 | 28.54 | 27.26 | 27.05 | 27.72 | 26.90 |
| ***E. coli* 1E-7** | 76 | 724 | 31.29 | 32.46 | 37.65 | 33.26 | 32.58 | 29.29 |
| ***E. coli* 1E-8** | 7 | 72 | 36.95 | NoCt | NoCt | 36.41 | 31.61 | 34.06 |
| **NSC 1** | 0 | 0 | NoCt | 33.69^{†} | NoCt | NoCt | NoCt | NoCt |
| **NSC 2** | 0 | 0 | NoCt | 31.78^{†} | NoCt | NoCt | 42.70 | NoCt |
| **NSC 3** | 0 | 0 | NoCt | 35.24^{†} | NoCt | NoCt | NoCt | NoCt |

| | | | | | | | | |
|---|---|---|---|---|---|---|---|---|
| Positivity threshold (Pt) ≤ 40 Ct; false positives^{†} | | | | | | | | |

| | | | | | **IPC Ct** | | | |
|---|---|---|---|---|---|---|---|---|
| | **Colonies** | ***E cfu/mL** | **COOH-0.2** | **COOH-1.0** | **HYDRO-1.0** | **NH2-1.5** | **Speed** | **BioEsta** |
| ***E. coli* 1E-6** | *724 | 7240 | 33.22 | 33.94 | 33.24 | 37.43 | 34.15 | 33.30 |
| ***E. coli* 1E-7** | 76 | 724 | 32.44 | 32.91 | 32.78 | 36.47 | 33.70 | 32.53 |
| ***E. coli* 1E-8** | 7 | 72 | 33.29 | 33.07 | 32.57 | 35.99 | 33.80 | 33.18 |
| **NSC 1** | 0 | 0 | 33.32 | 33.02 | 33.07 | 35.78 | 33.83 | 32.99 |
| **NSC 2** | 0 | 0 | 32.95 | 33.44 | 32.95 | 35.99 | 33.88 | 33.54 |
| **NSC 3** | 0 | 0 | 33.61 | 33.66 | 33.23 | 35.77 | 34.33 | 33.25 |

### Analysis:

These results demonstrate that a variety of different bead sizes and functional coatings produce comparable levels of microbial binding as determined by ETGA and Confirm readouts.

### Example 12: Magnetic beads of different size and functional coating can be used to capture a broad range of microbial species (Gram Negative, Gram Positive and Candida) from blood

### Aim:

To compare microbial capture performance for a variety of commercially-available magnetic beads of different size and functional coating using Momentum's Magnitor test (ETGA and Confirm technologies). *E. coli was* tested previously (Bead size and coating I: source experiment: 20190221_WP7_Bead-Comparison_Analysis and 20190228_WP7_Bead-Comparison-3-wash_Analysis): to expand on this previous work, three additional microbial species were tested (*S. aureus, S. pneumoniae* and *C. albicans*)*.*

### Test conditions:

| Heading | Description | Product | Diameter (µm) | Ferrite % | Polymer |
|---|---|---|---|---|---|
| COOH-0.2 | Very Small Estapor^{®} Carboxylated Nanospheres | Merck #M1-020/50 | 0.160-0.240 | >50 | Polystyrene |
| COOH-1.0 | Original Estapor^{®} Carboxylated Microspheres | Merck #M1-070/40 | 0.700-1.300 | 35-45 | Polystyrene |
| HYDRO-1.0 | Original Estapor^{®} Hydrophobic Microspheres | Merck #MS-070/40 | 0.700-1.300 | 35-50 | Polystyrene |
| NH2-1.5 | Original Estapor^{®} Aminated Microspheres (-NH2) | Merck #M2-070/40 | 1.000-2.000 | 35-45 | Polystyrene |

All beads washed in 1 mL 1X Tris+NaCl and resuspended to 1% solid in 1X Tris+NaCl

### Protocol:

### Sample set-up:

- Microorganism overnight liquid cultures (o/n) set-up in BacTec PLUS aerobic broth (inoculation of 3 mL broth from agar plate). The following day (approx 16 hours later) 300 µL *S*. *pneumoniae* and *C. albicans* liquid culture inoculated in 3 mL blood broth (1E-1 dilution), and 3 µL S. *aureus* liquid culture inoculated in 3 mL blood broth (1E-3 dilution); and 2 hour outgrowth performed at 37°C, 500 rpm.
- Following 2-hour outgrowth, microbial pre-cultures diluted (DF10) in blood broth to create three dilution points per microorganism.
- 100 µL TVCs performed for each microbial dilution

### Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 µL beads (1% solid) and 112 µL E-BUF (500 mM Tris-HCl [pH 8.0] + 1.5 M Sodium Chloride + 10% Igepal + 2.5% Tergitol)
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

| | | | | **ETGA Ct** | | |
|---|---|---|---|---|---|---|
| | **Colonies** | ***E cfu/mL** | **COOH-0.2** | **COOH-1.0** | **HYDRO-1.0** | **NH2-1.5** |
| ***S. aureus* 1E-5** | TNTC | 131,000 | 21.48 | 21.37 | 21.00 | 22.19 |
| ***S. aureus* 1E-6** | TNTC | 13,100 | 26.12 | 26.13 | 25.73 | 26.58 |
| ***S. aureus* 1E-7** | *131 | 1,310 | 30.76 | 30.29 | 30.25 | 30.38 |
| ***C. albicans* 1E-2** | LAWN | 956,000 | 26.68 | 26.04 | 26.24 | 26.64 |
| ***C. albicans* 1E-3** | TNTC | 95,600 | 28.08 | 26.46 | 27.08 | 26.76 |
| ***C. albicans* 1E-4** | *956 | 9,560 | 31.20 | 31.09 | 30.64 | 31.14 |
| ***S. pneumoniae* 1E-2** | LAWN | 732,000 | 30.52 | 31.34 | 30.61 | 30.41 |
| ***S. pneumoniae* 1E-3** | TNTC | 73,200 | 35.07 | 35.38 | 34.28 | 34.01 |
| ***S. pneumoniae* 1E-4** | *732 | 7,320 | 38.73 | 38.02 | 37.75 | 34.93 |
| **NSC 1** | 0 | - | 42.44 | 36.99 | 41.11 | 33.84 |
| **NSC 2** | 0 | - | 41.52 | 38.95 | 40.56 | 34.52 |
| **NSC 3** | 0 | - | 41.64 | 39.35 | 40.17 | 35.27 |

| | | | **Confirm Ct** | | | | | | | | | | | |
|---|---|---|---|---|---|---|---|---|---|---|---|---|---|---|
| | | | **COOH-0.2** | | | **COOH-1.0** | | | **HYDRO-1.0** | | | **NH2-1.5** | | |
| | **Colonies** | ***E *cfu*/*mL*** | **GrNeg** | **GrPos** | **Candida** | **GrNeg** | **GrPos** | **Candida** | **GrNeg** | **GrPos** | **Candida** | **GrNeg** | **GrPos** | **Candida** |
| ***S. aureus* 1E-5** | TNTC | 131,000 | NoCt | 29.03 | NoCt | NoCt | 29.05 | NoCt | NoCt | 28.63 | NoCt | NoCt | 26.17 | NoCt |
| ***S. aureus* 1E-6** | TNTC | 13,100 | NoCt | 32.30 | NoCt | NoCt | 33.17 | NoCt | NoCt | 32.67 | NoCt | NoCt | 29.46 | NoCt |
| ***S. aureus* 1E-7** | *131 | 1,310 | NoCt | 39.23 | NoCt | 40.08 | NoCt | NoCt | NoCt | 41.21 | NoCt | NoCt | 33.84 | NoCt |
| ***C. albicans* 1E-2** | LAWN | 956,000 | NoCt | NoCt | 28.35 | NoCt | NoCt | 27.45 | NoCt | NoCt | 26.37 | NoCt | NoCt | 27.24 |
| ***C. albicans* 1E-3** | TNTC | 95,600 | NoCt | NoCt | 30.93 | NoCt | 36.10* | 31.54 | NoCt | NoCt | 30.17 | NoCt | NoCt | 29.05 |
| ***C. albicans* 1E-4** | *956 | 9,560 | NoCt | NoCt | NoCt | NoCt | NoCt | 38.86 | NoCt | NoCt | 48.57 | NoCt | 39.91* | 44.85 |
| ***S. pneumoniae* 1E-2** | LAWN | 732,000 | NoCt | 25.20 | NoCt | NoCt | 26.26 | NoCt | NoCt | 24.58 | NoCt | NoCt | 26.31 | NoCt |
| ***S. pneumoniae* 1E-3** | TNTC | 73,200 | NoCt | 29.16 | NoCt | NoCt | 29.85 | NoCt | NoCt | 28.22 | NoCt | NoCt | 31.18 | NoCt |
| ***S. pneumoniae* 1E-4** | *732 | 7,320 | NoCt | 32.38 | NoCt | NoCt | 33.47 | NoCt | NoCt | 32.33 | NoCt | NoCt | 33.74 | NoCt |
| **NSC 1** | 0 | - | NoCt | NoCt | NoCt | NoCt | NoCt | NoCt | NoCt | NoCt | NoCt | NoCt | 41.24 | NoCt |
| **NSC2** | 0 | - | NoCt | NoCt | NoCt | NoCt | NoCt | NoCt | NoCt | NoCt | NoCt | NoCt | NoCt | NoCt |
| **NSC3** | 0 | - | NoCt | NoCt | NoCt | NoCt | NoCt | NoCt | NoCt | NoCt | NoCt | NoCt | NoCt | NoCt |

| | | | | | | | | | | | | | | |
|---|---|---|---|---|---|---|---|---|---|---|---|---|---|---|
| Positivity threshold (Pt) ≤ 40 Ct; *false positives | | | | | | | | | | | | | | |

| | | | | **IPC Ct** | | |
|---|---|---|---|---|---|---|
| | **Colonies** | ***E cfu/mL** | **COOH-0.2** | **COOH-1.0** | **HYDRO-1.0** | **NH2-1.5** |
| ***S. aureus* 1E-5** | TNTC | 131,000 | 33.22 | 33.19 | 33.14 | 34.50 |
| ***S. aureus* 1E-6** | TNTC | 13,100 | 33.19 | 32.55 | 33.07 | 34.55 |
| ***S. aureus* 1E-7** | *131 | 1,310 | 33.61 | 32.80 | 33.05 | 34.20 |
| ***C. albicans* 1E-2** | LAWN | 956,000 | 34.75 | 34.71 | 34.80 | 35.47 |
| ***C. albicans* 1E-3** | TNTC | 95,600 | 33.59 | 33.02 | 33.49 | 34.54 |
| ***C. albicans* 1E-4** | *956 | 9,560 | 33.14 | 33.02 | 32.81 | 34.06 |
| ***S. pneumoniae* 1E-2** | LAWN | 732,000 | 34.02 | 33.22 | 33.56 | 34.17 |
| ***S. pneumoniae* 1E-3** | TNTC | 73,200 | 33.29 | 33.05 | 33.44 | 35.28 |
| ***S. pneumoniae* 1E-4** | *732 | 7,320 | 33.23 | 32.84 | 33.02 | 33.75 |
| **NSC 1** | 0 | - | 33.17 | 33.21 | 33.50 | 33.96 |
| **NSC 2** | 0 | - | 33.38 | 33.17 | 33.32 | 34.50 |
| **NSC 3** | 0 | - | 33.55 | 33.59 | 33.05 | 34.20 |

### Analysis:

- These results demonstrate that a variety of different bead sizes and functional coatings produce comparable levels of microbial binding as determined by ETGA and Confirm readouts.

### Example 13: Magnetic beads of different size and functional coating can be used to capture a broad range of microbial species (Gram Negative, Gram Positive and Candida) from a simple Tris+NaCl buffer

### Aim:

To compare microbial capture performance for a variety of commercially-available magnetic beads of different size and functional coating using Momentum's Magnitor test (ETGA and Confirm technologies). This experiment was performed using a simple buffer (50 mM Tris-HCl [pH 8.0] + 150 mM NaCl) as the specimen and wash buffer i.e. no detergents used until the addition of microbial lysis mix.

### Test conditions:

| Heading | Description | Product | Diameter (µm) | Ferrite % | Polymer |
|---|---|---|---|---|---|
| COOH-0.2 | Very Small Estapor^{®} Carboxylated Nanospheres | Merck #M1-020/50 | 0.160-0.240 | >50 | Polystyrene |
| COOH-1.0 | Original Estapor^{®} Carboxylated Microspheres | Merck #M1-070/40 | 0.700-1.300 | 35-45 | Polystyrene |
| HYDRO-1.0 | Original Estapor^{®} Hydrophobic Microspheres | Merck #MS-070/40 | 0.700-1.300 | 35-50 | Polystyrene |
| NH2-1.5 | Original Estapor^{®} Aminated Microspheres (-NH2) | Merck #M2-070/40 | 1.000-2.000 | 35-45 | Polystyrene |

All beads washed in 1 mL 1X Tris+NaCl (50 mM Tris-HCl [pH 8.0] + 150 mM NaCl) and resuspended to 1% solid in 1X Tris+NaCl

### Protocol:

### Sample set-up:

- Microorganism overnight liquid cultures (o/n) set-up in BacTec PLUS aerobic broth (inoculation of 3 mL broth from agar plate). The following day (approx 16 hours later) 3 µL *E. coli and S. aureus* liquid culture inoculated in 3 mL broth (1E-3 dilution), and 300 µL *C. albicans* liquid culture inoculated in 3 mL broth (1E-1 dilution); and 2-hour outgrowth performed at 37°C, 500 rpm.
- Following 2-hour outgrowth, microbial pre-cultures diluted (DF10) in 1X Tris+NaCl buffer to create three dilution points per microorganism.
- 100 µL TVCs performed for each microbial dilution

### Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 µL beads (1% solid)
- 30 mins orbital mixing (1000 rpm) @ 37°C
- 5 mins magnetisation on DynaMag-2
- All s/n removed
- 1 mL WB (1X Tris+NaCl) added and tubes mixed for 3 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

| | | | | **ETGA Ct** | | |
|---|---|---|---|---|---|---|
| | **Colonies** | ***E cfu/mL** | **COOH-0.2** | **COOH-1.0** | **HYDRO-1.0** | **NH2-1.5** |
| ***E. coli* 1E-6** | *629 | 6,290 | 18.68 | 19.24 | 22.36 | 30.63 |
| ***E. coli* 1E-7** | 65 | 629 | 22.43 | 22.29 | 25.24 | 31.25 |
| ***E. coli* 1E-8** | 21 | 63 | 28.70 | 28.21 | 29.44 | 31.35 |
| ***S. aureus* 1E-5** | *791 | 7,910 | 18.32 | 18.22 | 18.08 | 25.72 |
| ***S. aureus* 1E-6** | 102 | 791 | 22.26 | 22.94 | 22.58 | 30.12 |
| ***S. aureus* 1E-7** | 5 | 79 | 27.65 | 26.37 | 26.61 | 30.26 |
| ***C. albicans* 1E-2** | LAWN | 811,000 | 25.38 | 25.51 | 26.02 | 29.07 |
| ***C. albicans* 1E-3** | TNTC | 81,100 | 27.13 | 27.71 | 27.50 | 30.09 |
| ***C. albicans* 1E-4** | *811 | 8,110 | 28.93 | 28.78 | 29.03 | 30.59 |
| **NSC** 1 | 0 | - | 35.04 | 32.14 | 36.86 | 32.12 |
| **NSC** 2 | 0 | - | 36.37 | 31.65 | 36.47 | 31.61 |
| **NSC** 3 | 0 | - | 36.54 | 32.51 | 35.46 | 30.97 |

| | | | **Confirm Ct** | | | | | | | | | | | |
|---|---|---|---|---|---|---|---|---|---|---|---|---|---|---|
| | | | **COOH-0.2** | | | **COOH-1.0** | | | **HYDRO-1.0** | | | **NH2-1.5** | | |
| | **Colonies** | ***E cfu/mL** | **GrNeg** | **GrPos** | **Candida** | **GrNeg** | **GrPos** | **Candida** | **GrNeg** | **GrPos** | **Candida** | **GrNeg** | **GrPos** | **Candida** |
| ***E. coli* 1E-6** | *629 | 6,290 | 28.24 | NoCt | NoCt | 26.59 | NoCt | NoCt | 27.42 | NoCt | NoCt | 27.39 | NoCt | NoCt |
| ***E. coli* 1E-7** | 65 | 629 | 38.82 | NoCt | NoCt | 29.58 | NoCt | NoCt | 29.43 | NoCt | NoCt | NoCt | NoCt | NoCt |
| ***E. coli* 1E-8** | 21 | 63 | NoCt | NoCt | NoCt | NoCt | NoCt | NoCt | 34.63 | NoCt | NoCt | NoCt | 46.54 | NoCt |
| ***S. aureus* 1E-5** | *791 | 7,910 | NoCt | 29.56 | NoCt | NoCt | 28.61 | NoCt | NoCt | 28.79 | NoCt | NoCt | NoCt | NoCt |
| ***S. aureus* 1E-6** | 102 | 791 | NoCt | 32.82 | NoCt | NoCt | 31.94 | NoCt | NoCt | 31.52 | NoCt | NoCt | NoCt | NoCt |
| ***S. aureus* 1E-7** | 5 | 79 | NoCt | 36.19 | NoCt | NoCt | 35.89 | NoCt | NoCt | 35.26 | NoCt | NoCt | NoCt | NoCt |
| ***C. albicans* 1E-2** | LAWN | 811,000 | NoCt | 43.68 | 28.74 | NoCt | 41.77 | 27.34 | NoCt | 32.91 | 26.74 | NoCt | NoCt | 31.90 |
| ***C. albicans* 1E-3** | TNTC | 81,100 | NoCt | NoCt | 29.59 | 43.60 | NoCt | 29.14 | NoCt | 36.69 | 29.16 | NoCt | 38.39 | 48.53 |
| ***C. albicans* 1E-4** | *811 | 8,110 | NoCt | NoCt | 31.92 | NoCt | NoCt | 30.81 | NoCt | NoCt | 30.45 | NoCt | 32.56 | 40.26 |
| **NSC 1** | 0 | - | NoCt | NoCt | NoCt | NoCt | NoCt | NoCt | NoCt | NoCt | NoCt | NoCt | 41.43 | NoCt |
| **NSC 2** | 0 | - | NoCt | NoCt | NoCt | NoCt | NoCt | NoCt | NoCt | NoCt | NoCt | NoCt | NoCt | NoCt |
| **NSC 3** | 0 | - | NoCt | 41.20 | NoCt | NoCt | NoCt | NoCt | NoCt | NoCt | NoCt | NoCt | 40.79 | NoCt |

| | | | | | | | | | | | | | | |
|---|---|---|---|---|---|---|---|---|---|---|---|---|---|---|
| Positivity threshold (Pt) ≤ 40 Ct; false positives shown in red font | | | | | | | | | | | | | | |

| | | | **IPC Ct** | | | |
|---|---|---|---|---|---|---|
| | **Colonies** | ***E cfu/mL** | **COOH-0.2** | **COOH-1.0** | **HYDRO-1.0** | **NH2-1.5** |
| ***E. coli* 1E-6** | *629 | 6,290 | 31.24 | 30.86 | 30.98 | 36.67 |
| ***E. coli* 1E-7** | 65 | 629 | 31.10 | 30.46 | 30.27 | 35.56 |
| ***E. coli* 1E-8** | 21 | 63 | 31.01 | 30.25 | 30.51 | 37.00 |
| ***S. aureus* 1E-5** | *791 | 7,910 | 31.43 | 31.17 | 31.02 | NoCt |
| ***S. aureus* 1E-6** | 102 | 791 | 30.89 | 31.11 | 30.71 | 37.43 |
| ***S. aureus* 1E-7** | 5 | 79 | 30.84 | 30.56 | 30.69 | 45.23 |
| ***C. albicans* 1E-2** | LAWN | 811,000 | 35.64 | 33.75 | 33.48 | NoCt |
| ***C. albicans* 1E-3** | TNTC | 81,100 | 32.98 | 31.94 | 33.61 | 40.29 |
| ***C. albicans* 1E-4** | *811 | 8,110 | 31.34 | 31.44 | 31.74 | 37.11 |
| **NSC 1** | 0 | - | 31.03 | 30.57 | 30.62 | 37.31 |
| **NSC 2** | 0 | - | 31.37 | 30.55 | 30.51 | 35.33 |
| **NSC 3** | 0 | - | 31.32 | 30.37 | 30.75 | 36.67 |

### Analysis:

- These results demonstrate that in a clean system (i.e. simple Tris+NaCl buffer instead of a biological specimen) a variety of different bead sizes and functional surfaces (carboxylated and hydrophobic) produce comparable levels of microbial binding as determined by ETGA and Confirm readouts.
- Interestingly, aminated beads (NH2-1.5) produced very poor Magnitor results in specimen type, indicative of little/no microbial binding. This observation differs to the situation in blood broth specimens, where aminated beads produced comparable levels of microbial capture to the other beads tested.

### Example 14: Magnetic beads of different size and functional coating can be used to capture a broad range of microbial species (Gram Negative, Gram Positive and Candida) from non-lysed blood

### Aim:

To compare microbial capture performance for a variety of commercially-available magnetic beads of different size and functional coating using Momentum's Magnitor test (ETGA and Confirm technologies) in the absence of blood lysis i.e. no detergents in binding buffer

### Test conditions:

| Heading | Description | Product | Diameter (µm) | Ferrite % | Polymer |
|---|---|---|---|---|---|
| COOH-0.2 | Very Small Estapor^{®} Carboxylated Nanospheres | Merck #M1-020/50 | 0.160-0.240 | >50 | Polystyrene |
| COOH-1.0 | Original Estapor^{®} Carboxylated Microspheres | Merck #M1-070/40 | 0.700-1.300 | 35-45 | Polystyrene |
| HYDRO-1.0 | Original Estapor^{®} Hydrophobic Microspheres | Merck #MS-070/40 | 0.700-1.300 | 35-50 | Polystyrene |
| NH2-1.5 | Original Estapor^{®} Aminated Microspheres (-NH2) | Merck #M2-070/40 | 1.000-2.000 | 35-45 | Polystyrene |

All beads washed in 1 mL 1X Tris+NaCl (50 mM Tris-HCl [pH 8.0] + 150 mM NaCl) and resuspended to 1% solid in 1X Tris+NaCl

### Protocol:

### Sample set-up:

- Microorganism overnight liquid cultures (o/n) set-up in BacTec PLUS aerobic broth (inoculation of 3 mL broth from agar plate). The following day (approx 16 hours later) 3 µL *E. coli* and *S. aureus* liquid culture inoculated in 3 mL broth (1E-3 dilution), and 300 µL *C. albicans* liquid culture inoculated in 3 mL blood broth (1E-1 dilution); and 2-hour outgrowth performed at 37°C, 500 rpm.
- Following 2-hour outgrowth, microbial pre-cultures diluted (DF10) in blood broth to create three dilution points per microorganism.
- 100 µL TVCs performed for each microbial dilution

### Manual simulation of Magnitor performed using DynaMag-2 magnet and manual liquid transfers:

- 1 mL specimens (three dilutions per microorganism species and three NSC samples: 12 sample-set) added to 2 mL sample tubes preloaded 15 µL beads (1% solid) and 112 µL Binding Buffer (Tris-HCl + Sodium Chloride)
- 30 mins orbital mixing (1000 rpm) @ 37°C
- 5 mins magnetisation on DynaMag-2
- All s/n removed
- 1 mL WB added and tubes mixed for 3 mins @ RT (1000 rpm)
- 5 mins magnetisation on Dynmag-2
- All s/n removed
- 50 µL LM added to tubes off magnet
- ETGA reaction performed: 5 mins at 1000 rpm, then 55 mins at 800 rpm @26°C
- Manual qPCR set-up for ETGA and Confirm (10 µL reactions)

### Results:

| | | | **ETGA Ct** | | | |
|---|---|---|---|---|---|---|
| | **Colonies** | ***E cfu/mL** | **COOH-0.2** | **COOH-1.0** | **HYDRO-1.0** | **NH2-1.5** |
| ***E. coli* 1E-6** | *671 | 6,710 | 28.37 | 28.22 | 28.83 | 28.40 |
| ***E. coli* 1E-7** | 69 | 671 | 32.26 | 32.90 | 31.21 | 31.73 |
| ***E. coli* 1E-8** | 24 | 67 | 35.02 | 33.23 | 34.05 | 32.73 |
| ***S. aureus* 1E-5** | TNTC | 27,600 | 22.23 | 22.86 | 22.45 | 22.57 |
| ***S. aureus* 1E-6** | *276 | 2,760 | 26.62 | 26.64 | 26.53 | 26.09 |
| ***S. aureus* 1E-7** | 28 | 276 | 30.37 | 29.44 | 30.72 | 31.01 |
| ***C. albicans* 1E-2** | LAWN | 649,000 | 34.01 | 34.00 | 33.52 | 33.48 |
| ***C. albicans* 1E-3** | TNTC | 64,900 | 35.00 | 33.89 | 34.23 | 33.66 |
| ***C. albicans* 1E-4** | *649 | 6,490 | 35.47 | 35.08 | 34.48 | 33.73 |
| **NSC 1** | 0 | - | 33.95 | 35.32 | 32.42 | 31.15 |
| **NSC 2** | 0 | - | 32.27 | 34.34 | 32.47 | 30.76 |
| **NSC 3** | 0 | - | 33.89 | 33.37 | 32.45 | 31.24 |

| | | | **Confirm Ct** | | | | | | | | | | | |
|---|---|---|---|---|---|---|---|---|---|---|---|---|---|---|
| | | | **COOH-0.2** | | | **COOH-1.0** | | | **HYDRO-1.0** | | | **NH2-1.5** | | |
| | **Colonies** | ***E cfu/mL** | **GrNeg** | **GrPos** | **Candida** | **GrNeg** | **GrPos** | **Candida** | **GrNeg** | **GrPos** | **Candida** | **GrNeg** | **GrPos** | **Candida** |
| ***E. coll* 1E-6** | *671 | 6,710 | 30.24 | No Ct | No Ct | 30.51 | No Ct | No Ct | 31.59 | No Ct | No Ct | 30.47 | No Ct | No Ct |
| ***E. coli* 1E-7** | 69 | 671 | 38.27 | No Ct | No Ct | 48.35 | No Ct | No Ct | No Ct | No Ct | No Ct | 35.36 | No Ct | No Ct |
| ***E. coli* 1E-8** | 24 | 67 | No Ct | No Ct | No Ct | No Ct | No Ct | No Ct | No Ct | No Ct | No Ct | No Ct | No Ct | No Ct |
| ***S. aureus* 1E-5** | TNTC | 27,600 | No Ct | 24.94 | No Ct | No Ct | 24.55 | No Ct | No Ct | 24.34 | No Ct | No Ct | 24.24 | No Ct |
| ***S. aureus* 1E-6** | *276 | 2,760 | No Ct | 27.34 | No Ct | No Ct | 27.54 | 48.19 | No Ct | 28.18 | No Ct | No Ct | 27.37 | No Ct |
| ***S. aureus* 1E-7** | 28 | 276 | No Ct | 30.39 | No Ct | No Ct | 29.72 | No Ct | No Ct | 31.12 | No Ct | No Ct | 29.65 | No Ct |
| ***C. albicans* 1E-2** | LAWN | 649,000 | No Ct | 40.65 | 31.51 | 40.74 | No Ct | 29.89 | No Ct | No Ct | 30.24 | No Ct | No Ct | 28.70 |
| ***C. albicans* 1E-3** | TNTC | 64,900 | No Ct | No Ct | No Ct | 42.94 | No Ct | 34.41 | No Ct | No Ct | No Ct | No Ct | No Ct | No Ct |
| ***C. albicans* 1E-4** | *649 | 6,490 | No Ct | No Ct | No Ct | No Ct | No Ct | No Ct | No Ct | No Ct | No Ct | No Ct | No Ct | No Ct |
| **NSC 1** | 0 | - | No Ct | No Ct | No Ct | No Ct | No Ct | No Ct | No Ct | 45.94 | No Ct | No Ct | No Ct | No Ct |
| **NSC 2** | 0 | - | No Ct | No Ct | No Ct | 42.39 | No Ct | No Ct | No Ct | No Ct | No Ct | No Ct | No Ct | No Ct |
| **NSC 3** | 0 | - | No Ct | No Ct | No Ct | No Ct | No Ct | No Ct | No Ct | No Ct | No Ct | No Ct | No Ct | No Ct |

| | | | | | | | | | | | | | | |
|---|---|---|---|---|---|---|---|---|---|---|---|---|---|---|
| Positivity threshold (Pt) ≤ 40 Ct; false positives shown in red font | | | | | | | | | | | | | | |

| | | | **IPC Ct** | | | |
|---|---|---|---|---|---|---|
| | **Colonies** | ***E cfu/mL** | **COOH-0.2** | **COOH-1.0** | **HYDRO-1.0** | **NH2-1.5** |
| ***E. coli* 1E-6** | *671 | 6,710 | 32.75 | 32.82 | 33.21 | 33.42 |
| ***E. coli* 1E-7** | 69 | 671 | 33.02 | 33.61 | 32.90 | 33.81 |
| ***E. coli* 1E-8** | 24 | 67 | 34.18 | 33.17 | 33.58 | 33.21 |
| ***S. aureus* 1E-5** | TNTC | 27,600 | 33.31 | 33.77 | 33.86 | 34.06 |
| ***S. aureus* 1E-6** | *276 | 2,760 | 33.46 | 33.20 | 33.74 | 33.07 |
| ***S. aureus* 1E-7** | 28 | 276 | 32.96 | 33.06 | 33.93 | 33.71 |
| ***C. albicans* 1E-2** | LAWN | 649,000 | 32.94 | 33.30 | 33.81 | 33.95 |
| ***C. albicans* 1E-3** | TNTC | 64,900 | 33.82 | 33.25 | 33.71 | 34.42 |
| ***C. albicans* 1E-4** | *649 | 6,490 | 33.58 | 33.53 | 33.32 | 34.25 |
| **NSC 1** | 0 | - | 34.42 | 34.62 | 33.47 | 33.77 |
| **NSC 2** | 0 | - | 33.62 | 33.76 | 33.61 | 33.86 |
| **NSC 3** | 0 | - | 33.83 | 33.81 | 33.71 | 33.33 |

### Analysis:

- These results demonstrate that a variety of different bead sizes and functional coatings produce comparable levels of microbial binding from blood in the absence of blood lysis as determined by ETGA and Confirm readouts
- However, microbial detection by ETGA is substantially reduced by an increase in NSC ETGA signal in the absence of blood lysis, when compared to previous experiments with blood lysis during microbial binding.

### Example 15: Microbial capture by magnetic beads occurs in a variety of complex biological specimen types

### Aim:

To investigate whether microbial capture using magnetic beads is possible for other complex biological fluids, in addition to blood

### Test conditions:

| Specimen type | Description |
|---|---|
| Tris+NaCl | Tris buffer with NaCl as non-biological control sample-set (two identical sample-sets processing in parallel for Magnitor and Regrowth assays) |
| Blood | Whole blood with CPD and SPS anticoagulants |
| Saliva | Saliva diluted to 50% with distilled H2O |
| Urine | Mid-stream urine |
| Milk | Semi-skimmed Pasteurised Cow's milk |

| | |
|---|---|
| Note: Tris+NaCl: 50 mM Tris-HCl [pH 8.0] + 150 mM Sodium Chloride | |

### Protocol:

- *E. coli o*/*n* liquid cultures set-up as standard in BacTec PLUS aerobic broth, then the following day (approx 16 hours later) 3 µL o/n added to 3 mL broth *(E. coli* 1E-3), and 2-hour outgrowth incubation performed at 37°C, 500 rpm.
- Following 2-hour outgrowth, *E. coli* 1E-3 preculture diluted to produce five serial dilution points (E. *coli* 1E-6 to 1E-9) in each specimen type. 100 µL TVCs performed on COL agar plates.

### Manual simulation of Magnitor performed using DynaMag-2 magnet and manual liquid transfers:

- 1 mL specimens added to 2 mL sample tubes preloaded with 112 µL Binding Buffer (500 mM Tris-HCl [pH 8.0] + 1.5 M Sodium Chloride + 10% Igepal + 2.5% Tergitol + 0.5% Sodium Deoxycholate) + 15 µL beads (BioEstapor beads; Merck #BE-M08/03 (1% solid)) - Note, sample tubes for Tris+NaCl sample-sets not preloaded with 112 µL binding buffer (to avoid inclusion of detergents, which might inhibit microbial growth for the Regrowth assay)
- 30 mins shaking (1000 rpm) @ 37°C
- 5 mins magnetisation on DynaMag-2
- All s/n removed
- 1 mL WB added and tubes mixed for 3 mins @ RT (1000 rpm) - note, 1 mL Tris+NaCl buffer added instead of wash buffer for Tris+NaCl sample-sets to avoid inclusion of detergents, which might inhibit microbial growth for the Regrowth assay
- 5 mins magnetisation on Dynmag-2
- All s/n removed
- 50 µL LM added to tubes off magnet - note, beads resuspended in 100 µL Tris+NaCl buffer for Regrowth assay
- ETGA reaction performed: 5 mins at 1000 rpm, then 55 mins at 800 rpm @26°C
- Manual qPCR set-up for ETGA and Confirm (10 µL reactions)

### Results:

### TVCs (100 µL on COL plates)

| Specimen | Colonies |
|---|---|
| Tris+NaCl *E. coli* D1 | TNTC |
| Tris+NaCl *E. coli* D2 | 661 |
| Tris+NaCl *E. coli* D3 | 121 |
| Tris+NaCl *E. coli* D4 | 21 |
| Tris+NaCl *E. coli* D5 | 0 |
| Tris+NaCl NSC | 0 |
| Blood NSC | 0 |
| Saliva NSC | LAWN |
| Urine NSC | 5 |
| Milk NSC | 3 |

### Regrowth assay on paired Tris+NaCl sample-set

1. 100 µl of Tris+NaCl specimens plated/inoculated - 'Specimen'
2. 100 µl of supernatant plated/inoculated after microbial binding step - 'After binding'
3. 100 µl of supernatant plated/inoculated after wash step - 'After washing'
4. 50 µl of beads resuspended in 100 µL Tris+NaCl buffer and plated/inoculated (i.e. 50% material on plate and 50% material inoculated into liquid culture) - 'Beads'

### Plates and Liquid Cultures incubated overnight at 37°C

| | **TVC (colonies)** | | | **Nutrient Broth (Growth: YES/NO)** | | | | |
|---|---|---|---|---|---|---|---|---|
| | **Specimen** | **After bind** | **After wash** | **Beads** | **Specimen** | **After bind** | **After wash** | **Beads** |
| ***E. coli* D1** | TNTC | 555 | 453 | LAWN | YES | YES | YES | YES |
| ***E. coli* D2** | 661 | 104 | 5 | TNTC | YES | YES | YES | YES |
| ***E. coli* D3** | 121 | 12 | 1 | 366 | YES | YES | NO | YES |
| ***E. coli* D4** | 21 | 12 | 0 | 7 | YES | YES | NO | YES |
| ***E. coli* D5** | 0 | 2 | 0 | 0 | YES | YES | NO | YES |
| **NSC** | 0 | 0 | 0 | 0 | NO | NO | NO | NO |

| | | | | | | | | |
|---|---|---|---|---|---|---|---|---|
| ***Tris+NaCl sample-set*** | | | | | | | | |

### Magnitor Results:

| | | | **ETGA Ct** | | | | |
|---|---|---|---|---|---|---|---|
| | **Colonies** | ***E cfu/mL** | **Tris+NaCl** | **Blood** | **Saliva** | **Urine** | **Milk** |
| ***E. coli* D1** | TNTC | 66100 | 14.49 | 21.22 | 26.45 | 23.13 | 20.30 |
| ***E. coli* D2** | *661 | 6610 | 20.62 | 25.73 | 28.50 | 26.22 | 24.97 |
| ***E. coli* D3** | 121 | 661 | 24.09 | 29.41 | 28.14 | 29.39 | 28.22 |
| ***E. coli* D4** | 21 | 66 | 29.84 | 33.98 | 28.20 | 30.83 | 30.99 |
| ***E. coli* D5** | 0 | 7 | 35.13 | 36.56 | 28.33 | 30.40 | 30.07 |
| **NSC 1** | 0 | 0 | 35.74 | 38.41 | 27.32 | 31.05 | 30.70 |
| **NSC 2** | 0 | 0 | 34.90 | 39.26 | 28.06 | 31.06 | 30.48 |
| **NSC 3** | 0 | 0 | 35.04 | 39.45 | 28.73 | 31.08 | 30.89 |
| **Ave. NSC** | | | 35.23 | 39.04 | 28.04 | 31.06 | 30.69 |
| **Pt (5th%)** | | | **34.91** | **38.49** | **27.39** | **31.05** | **30.50** |

### Positivity Thresholds (Pt) calculated using NSCs (n= 3) for each specimen type using formula = PERCENTILE.INC(array,0.05)

| | | | **Confirm GrNeg** | | | | **Confirm GrPos** | | | | | |
|---|---|---|---|---|---|---|---|---|---|---|---|---|
| | **Colonies** | ***E cfu/mL** | **Tris+NaCl** | **Blood** | **Saliva** | **Urine** | **Milk** | **Tris+NaCl** | **Blood** | **Saliva** | **Urine** | **Milk** |
| ***E. coli* D1** | TNTC | 66100 | 20.56 | 27.24 | NoCt | 30.16 | 21.25 | NoCt | NoCt | 27.22 | 32.87 | 28.01 |
| ***E. coli* D2** | *661 | 6610 | 27.03 | 31.35 | NoCt | NoCt | 25.38 | NoCt | NoCt | 28.52 | 33.07 | 27.43 |
| ***E. coli* D3** | 121 | 661 | 27.64 | 37.88 | NoCt | NoCt | 29.58 | NoCt | NoCt | 29.99 | 31.79 | 26.94 |
| ***E. coli* D4** | 21 | 66 | NoCt | NoCt | NoCt | NoCt | 43.46 | NoCt | NoCt | 28.12 | 35.25 | 26.91 |
| ***E. coli* D5** | 0 | 7 | NoCt | NoCt | NoCt | NoCt | NoCt | 40.65 | NoCt | 28.09 | 35.43 | 27.38 |
| **NSC 1** | 0 | 0 | NoCt | NoCt | NoCt | NoCt | NoCt | NoCt | 42.73 | 28.58 | 35.36 | 26.83 |
| **NSC 2** | 0 | 0 | NoCt | NoCt | NoCt | NoCt | 45.84 | NoCt | NoCt | 28.83 | 32.96 | 27.28 |
| **NSC 3** | 0 | 0 | NoCt | NoCt | NoCt | NoCt | NoCt | 35.55 | 42.95 | 26.77 | 32.95 | 27.40 |

| | | | | | | | | | | | | |
|---|---|---|---|---|---|---|---|---|---|---|---|---|
| Positivity threshold (Pt) ≤ 40 Ct *Note, no observable amplification in Candida channel for Confirm* | | | | | | | | | | | | |

| | | | **IPC Ct** | | | | |
|---|---|---|---|---|---|---|---|
| | **Colonies** | ***E cfu/mL** | **Tris+NaCl** | **Blood** | **Saliva** | **Urine** | **Milk** |
| ***E. coli* D1** | TNTC | 66100 | 38.08 | 34.81 | NoCt | 32.46 | 36.65 |
| ***E. coli* D2** | *661 | 6610 | 38.79 | 34.37 | NoCt | 32.83 | 37.05 |
| ***E. coli* D3** | 121 | 661 | 46.28 | 34.24 | NoCt | 32.76 | 36.44 |
| ***E. coli* D4** | 21 | 66 | 43.87 | 34.31 | NoCt | 32.87 | 35.68 |
| ***E. coli* D5** | 0 | 7 | 38.34 | 34.61 | NoCt | 32.60 | 35.72 |
| **NSC 1** | 0 | 0 | 37.18 | 34.07 | 41.24 | 33.06 | 36.76 |
| **NSC 2** | 0 | 0 | 38.20 | 33.79 | NoCt | 32.89 | 35.60 |
| **NSC 3** | 0 | 0 | 36.24 | 34.14 | NoCt | 33.10 | 35.97 |

### Analysis:

- These results demonstrate that magnetic beads can be used to capture microorganisms from a variety of complex biological specimen types as determined by ETGA and Confirm readouts.
- The Regrowth assay demonstrates that *E*. *coli* can regrow on agar and liquid culture after binding to magnetic beads, as determined by observable growth for 'Beads' sample-set.

### Example 16: Microbial capture and detection is possible from non-blood specimens in the absence of specimen lysis

### Aim:

To show microbial capture and detection in non-blood specimens of milk and urine using non-lysing binding buffer and non-lysing wash buffer.

### Preparation:

- **10X Tris+NaCl binding buffer** = 500 mM Tris-HCl [pH 8.0] + 1.5 M Sodium Chloride
- **1X Tris+NaCl wash buffer** = 1 in 10 dilution of 10 X Tris+NaCl binding buffer
- **Fresh (Human) urine**
- **Semi-skimmed (pasteurised) cow's milk**

BioEstapor beads (Merck, Cat# BE-M 08/0.3) were re-suspended prior to use.

### Protocol:

- *E. coli, S. aureus, C. albicans* and *S. pneumoniae* o/n liquid cultures set-up as standard in BacTec PLUS aerobic broth
- The following day (approx 16 hours later) 3 µL of *E*. *coli and S. aureus* o/n used to inoculate fresh 3 mL broth cultures (1E-3 dilutions), and 300 µL *C. albicans* and *S*. *pneumoniae* used to inoculate fresh 3 mL broth cultures (1E-1 dilutions); and 2-hour outgrowth performed at 37°C, 500 rpm.
- Following 2-hour outgrowth, microbial pre-cultures were serially diluted (DF10) in pre-warmed fresh urine and fresh milk to produce five dilution points: *E. coli* 1E-5 to 1E-9; *S. aureus* 1E-5 to 1E-9; *C. albicans* 1E-2 to 1E-6; and *S. pneumoniae* 1E-2 to 1E-6.
- 100 µL TVCs performed for each microbial dilution tested in milk and urine; NSCs for milk and urine were plated on three types of agar plate (SAB, COL and CBA)
- 1 mL specimens (five dilutions of each microbial species and four NSC samples: 24 samples per specimen type) added to 2 mL sample tubes preloaded with 112 µL Binding Buffer + 15 µL beads (1% solid), then automated Magnitor test initiated.

### Automated sample processing on epMotion 5073m:

- 30 mins orbital mixing (1000 rpm) @ 37°C
- 15 mins magnetisation
- 1 mL s/n removed
- 0.82 mL WB (1X Tris+NaCl) added to tubes whilst beads magnetised
- 1 mL s/n removed
- 50 µL LM added to tubes whilst beads magnetised
- Magnetisation switched off and ETGA reaction performed: 5 mins at 1000 rpm, then 55 mins at 800 rpm @26°C
- qPCR set-up for ETGA and Confirm (10 µL reactions)

### Results:

| | Urine | | Milk | |
|---|---|---|---|---|
| *Specimen* | **TVC** | ***E cfu/mL** | **TVC** | ***E cfu/mL** |
| *E. coli* 1E-5 | TNTC | 141000 | TNTC | 181000 |
| *E. coli* 1E-6 | TNTC | 14100 | TNTC | 18100 |
| *E. coli* 1E-7 | *141 | 1410 | *181 | 1810 |
| *E. coli* 1E-8 | 32 | 141 | 16 | 181 |
| *E. coli* 1E-9 | 3 | 14 | 6 | 18 |
| *S. aureus* 1E-5 | TNTC | 19600 | *812 | 8120 |
| *S. aureus* 1E-6 | *196 | 1960 | 69 | 812 |
| *S. aureus* 1E-7 | 11 | 196 | 15 | 81 |
| *S. aureus* 1E-8 | 2 | 20 | 8 | 8 |
| *S. aureus* 1E-9 | 1 | 2 | 9 | 1 |
| *C. albicans* 1E-2 | TNTC | 564000 | TNTC | 597000 |
| *C. albicans* 1E-3 | TNTC | 56400 | TNTC | 59700 |
| *C. albicans* 1E-4 | *564 | 5640 | *597 | 5970 |
| *C. albicans* 1E-5 | 89 | 564 | 107 | 597 |
| *C. albicans* 1E-6 | 11 | 56 | 6 | 60 |
| *S. pneumoniae* 1E-2 | TNTC | 4080000 | TNTC | 5740000 |
| *S. pneumoniae* 1E-3 | TNTC | 408000 | TNTC | 574000 |
| *S. pneumoniae* 1E-4 | TNTC | 40800 | TNTC | 57400 |
| *S. pneumoniae* 1E-5 | *408 | 4080 | *574 | 5740 |
| *S. pneumoniae* 1E-6 | 55 | 408 | 66 | 574 |
| NSC_SAB plate | 0 | 20 | 2 | 100 |
| NSC_COL plate | 0 | 20 | *10 | 100 |
| NSC_CBA plate | *2 | 20 | 10 | 100 |

| | | | | |
|---|---|---|---|---|
| **Cfu*/*mL values extrapolated from highest countable TVC* *(NB: Urine is a non-sterile solution, therefore, colonies on the NSC plates are not unexpected*) *(NB: pasteurised milk contains microorganisms, therefore, there should be colonies on the NSC plates)* | | | | |

### ETGA Ct

| ***Specimen*** | **Urine** | **Milk** |
|---|---|---|
| ***E. Coli* 1E-5** | 15.80 | 18.97 |
| ***E. Coli* 1E-6** | 21.69 | 21.38 |
| ***E. Coli* 1E-7** | 26.06 | 22.19 |
| ***E. Coli* 1E-8** | 29.54 | 22.18 |
| ***E. Coli* 1E-9** | 30.48 | 22.51 |
| ***S. aureus* 1E-5** | 19.57 | 21.47 |
| ***S. aureus* 1E-6** | 23.73 | 22.09 |
| ***S. aureus* 1E-7** | 27.82 | 22.44 |
| ***S. aureus* 1E-8** | 28.88 | 22.15 |
| ***S. aureus* 1E-9** | 28.95 | 22.28 |
| ***C. albicans* 1E-2** | 22.46 | 22.62 |
| ***C. albicans* 1E-3** | 24.56 | 22.51 |
| ***C. albicans* 1E-4** | 28.56 | 22.17 |
| ***C. albicans* 1E-5** | 29.82 | 22.39 |
| ***C. albicans* 1E-6** | 29.43 | 22.00 |
| ***S. pneumoniae* 1E-2** | 24.12 | 23.02 |
| ***S. pneumoniae* 1E-3** | 26.07 | 22.22 |
| ***S. pneumoniae* 1E-4** | 29.00 | 22.24 |
| ***S. pneumoniae* 1E-5** | 30.78 | 22.06 |
| ***S. pneumoniae* 1E-6** | 29.27 | 22.32 |
| **NSC 1** | 29.48 | 22.15 |
| **NSC 2** | 29.51 | 22.17 |
| **NSC 3** | 29.99 | 22.13 |
| **NSC 4** | 29.99 | 22.48 |
| **Average NSC** | 29.74 | 22.23 |

### IPC Ct

| ***Specimen*** | **Urine** | **Milk** |
|---|---|---|
| ***E. Coli* 1E-5** | 32.36 | 40.12 |
| ***E. Coli* 1E-6** | 31.64 | 37.94 |
| ***E. Coli* 1E-7** | 32.35 | 43.03 |
| ***E. Coli* 1E-8** | 33.06 | 37.06 |
| ***E. Coli* 1E-9** | 33.29 | 41.03 |
| ***S. aureus* 1E-5** | 32.37 | 36.09 |
| ***S. aureus* 1E-6** | 32.41 | 39.29 |
| ***S. aureus* 1E-7** | 32.48 | 38.13 |
| ***S. aureus* 1E-8** | 32.99 | 36.77 |
| ***S. aureus* 1E-9** | 33.11 | 37.21 |
| ***C. albicans* 1E-2** | 38.08 | 46.11 |
| ***C. albicans* 1E-3** | 33.72 | 37.14 |
| ***C. albicans* 1E-4** | 32.20 | 37.10 |
| ***C. albicans* 1E-5** | 32.43 | 36.26 |
| ***C. albicans* 1E-6** | 32.58 | 39.70 |
| ***S. pneumoniae* 1E-2** | 31.78 | 39.47 |
| ***S. pneumoniae* 1E-3** | 31.45 | 41.49 |
| ***S. pneumoniae* 1E-4** | 31.96 | 38.17 |
| ***S. pneumoniae* 1E-5** | 32.71 | 37.63 |
| ***S. pneumoniae* 1E-6** | 32.90 | 48.38 |
| **NSC 1** | 33.42 | 42.68 |
| **NSC 2** | 33.63 | 44.35 |
| **NSC 3** | 33.53 | 38.45 |
| **NSC 4** | 33.51 | 37.14 |

| | | |
|---|---|---|
| *NB: Pasteurised milk contains bacteria; these showed as a consistent ETGA Ct~22* | | |

### Confirm Ct

| | **Urine** | | | **Milk** | | |
|---|---|---|---|---|---|---|
| ***Specimen*** | **GrNeg** | **GrPos** | **Candida** | **GrNeg** | **GrPos** | **Candida** |
| ***E. Coli* 1E-5** | 28.86 | NoCt | NoCt | 22.71 | 22.56 | NoCt |
| ***E. Coli* 1E-6** | 29.38 | NoCt | NoCt | 29.95 | 23.01 | NoCt |
| ***E. Coli* 1E-7** | NoCt | NoCt | NoCt | NoCt | 22.61 | NoCt |
| ***E. Coli* 1E-8** | NoCt | 45.77 | NoCt | NoCt | 22.73 | NoCt |
| ***E. Coli* 1E-9** | NoCt | NoCt | NoCt | NoCt | 22.78 | NoCt |
| ***S. aureus* 1E-5** | NoCt | 30.03 | NoCt | NoCt | 21.46 | NoCt |
| ***S. aureus* 1E-6** | NoCt | 34.47 | NoCt | NoCt | 22.41 | NoCt |
| ***S. aureus* 1E-7** | NoCt | 38.97 | NoCt | NoCt | 22.33 | NoCt |
| ***S. aureus* 1E-8** | NoCt | 37.85 | NoCt | NoCt | 22.39 | NoCt |
| ***S. aureus* 1E-9** | NoCt | NoCt | NoCt | NoCt | 23.03 | NoCt |
| ***C. albicans* 1E-2** | NoCt | 41.75 | 28.06 | NoCt | 21.59 | 31.08 |
| ***C. albicans* 1E-3** | NoCt | NoCt | 28.61 | NoCt | 23.00 | 33.04 |
| ***C. albicans* 1E-4** | NoCt | NoCt | 36.26 | NoCt | 22.09 | 39.74 |
| ***C. albicans* 1E-5** | NoCt | NoCt | NoCt | NoCt | 36.30 | NoCt |
| ***C. albicans* 1E-6** | NoCt | NoCt | NoCt | NoCt | 22.07 | NoCt |
| ***S. pneumoniae* 1E-2** | NoCt | 27.07 | NoCt | NoCt | 20.58 | NoCt |
| ***S. pneumoniae* 1E-3** | NoCt | 31.78 | NoCt | NoCt | 22.08 | NoCt |
| ***S. pneumoniae* 1E-4** | NoCt | 42.22 | NoCt | NoCt | 22.47 | NoCt |
| ***S. pneumoniae* 1E-5** | NoCt | NoCt | NoCt | NoCt | 23.00 | NoCt |
| ***S. pneumoniae* 1E-6** | NoCt | 36.55 | NoCt | NoCt | 23.29 | NoCt |
| **NSC 1** | NoCt | NoCt | 43.91 | NoCt | 22.18 | NoCt |
| **NSC** 2 | NoCt | NoCt | NoCt | NoCt | 22.15 | NoCt |
| **NSC 3** | NoCt | 40.96 | NoCt | NoCt | 21.87 | NoCt |
| **NSC** 4 | NoCt | NoCt | NoCt | NoCt | 22.28 | NoCt |

| | | | | | | |
|---|---|---|---|---|---|---|
| Positivity threshold (Pt) ≤ 40 Ct | | | | | | |

### Analysis:

- These results demonstrate that microbial capture by magnetic beads is possible in alternative specimens to blood (specifically urine and milk) in the absence of specimen lysis, as determined by ETGA and Confirm read-outs.
- The presence of commensal microorganisms in these specimen types (particularly milk), does however, effect the level of background signal for ETGA and Confirm readouts.

## Claims

1. A method of separating microorganisms from non-microorganism cells in a non-microorganism cell-containing sample, the method comprising:
a) incubating the sample with particles to form particle-microorganism complexes, wherein the step of incubating is performed in the presence of sodium polyanethol sulfonate and a reagent that selectively lyses non-microorganism cells in the sample whilst retaining intact microorganisms present in the sample; and
b) separating the particle-microorganism complexes from the non-microorganism cells, wherein the sample is a blood containing sample, wherein the particles have a diameter of between 0.1 and 3.0 µm, and wherein the outer surface of the particles comprises or is coated with any one or more of:
i) carboxylic acid groups;
ii) amino groups;
iii) hydrophobic groups; and
iv) streptavidin.

2. The method of claim 1, wherein the method is a method of detecting the absence or presence of a microorganism in a sample that also contains non-microorganism cells further comprising:
c) detecting the absence or presence of microorganisms in the particle-microorganism complexes.

3. The method of claim 2, wherein step (c) comprises:
(A)
(i) detecting an enzymatic activity of a nucleic acid molecule associated with the microorganism, (ii) detecting the microorganism directly by cytometry or microscopy, (iii) detecting the microorganism following cell culture, (iv) detecting the microorganism by nucleic acid amplification or (v) detecting the microorganism by nucleic acid sequencing; or
(B)
i) lysing the microorganisms in the particle-microorganism complexes;
ii) incubating the lysate with a nucleic acid molecule which acts as a substrate for nucleic acid modifying activity of the microorganisms; and
iii) specifically determining the absence or presence of a modified nucleic acid molecule resulting from the action of the nucleic acid modifying enzyme on the substrate nucleic acid molecule to indicate the absence or presence of the microorganism,
optionally wherein step (i) comprises adding a lysis reagent containing the substrate nucleic acid molecule.

4. The method according to claim 3B), wherein the nucleic acid modifying enzyme comprises a DNA or RNA polymerase, optionally wherein the DNA polymerase is DNA polymerase I.

5. The method of according to any one of claims 2 to 4, wherein the method is a method of detecting the absence or presence of a microorganism infection in a subject, wherein the sample is a blood containing sample from the subject.

6. The method of any preceding claim, wherein the method further comprises washing the separated particle-microorganism complexes to remove non-microorganism cells or lysate.

7. The method of any preceding claim, wherein step b):
i) further comprises removing the non-microorganism cells from the particle-microorganism complexes; and/or
ii) is performed using a magnetic field or centrifugation.

8. The method of any one of claims 1 to 7, wherein the reagent that selectively lyses non-microorganism cells in the sample whilst retaining intact microorganisms present in the sample comprises a detergent; optionally wherein the detergent is non-ionic and/or the detergent is not conjugated to the particles capable of forming complexes with microorganisms.

9. A composition comprising:
a) a blood containing sample isolated from a subject potentially containing microorganism cells and that contains non-microorganism cells;
b) particles capable of forming complexes with microorganisms, wherein the particles have a diameter of between 0.1 and 3.0 µm, and wherein the particles have an outer surface comprising or coated with any one or more of:
i) carboxylic acid groups;
ii) amino groups;
iii) hydrophobic groups; and
iv) streptavidin;
c) sodium polyanethol sulfonate; and
d) at least one reagent that selectively lyses non-microorganism cells in the sample whilst retaining intact microorganisms present in the sample, wherein the at least one reagent that selectively lyses non-microorganism cells in the sample whilst retaining intact microorganisms present in the sample comprises a detergent.

10. A kit for performing the method of any one of claims 1 to 8, comprising:
a) particles capable of forming complexes with microorganisms, wherein the particles have a diameter of between 0.1 and 3.0 µm, and wherein the particles have an outer surface comprising or coated with any one or more of:
i) carboxylic acid groups;
ii) amino groups;
iii) hydrophobic groups; and
iv) streptavidin;
b) sodium polyanethol sulfonate; and
c) at least one reagent that selectively lyses non-microorganism cells in the sample whilst retaining intact microorganisms present in the sample, wherein the at least one reagent that selectively lyses non-microorganism cells in the sample whilst retaining intact microorganisms present in the sample comprises a detergent.

11. The kit of claim 10 for performing the method of any one of claims 2 to 8 further comprising:
detection means for detecting the absence or presence of microorganisms in the particle-microorganism complexes, wherein the microorganism is a pathogenic bacterium or fungus and wherein the detection means comprises a nucleic acid molecule (DNA) which acts as a substrate for nucleic acid modifying activity of the microorganisms, and wherein the nucleic acid molecule (DNA) is at least partially double stranded and comprises uracil residues in the complementary strand.

12. The composition of claim 9, or the kit of claim 10 or 11 further comprising a buffer and/or sodium chloride.

13. The method of any one of claims 1 to 8, the composition of claim 9 or 12, or the kit of any one of claims 10 to 12, wherein the particles: a) have a diameter of between 0.1 and 2.0 µm; and/or
b) are magnetic.

14. The method of any one of claims 1 to 8 or 13, the composition of any one of claims 9, 12 or 13, or the kit of any one of claims 10 to 13, wherein the outer surface of the particles capable of forming complexes with microorganisms comprises a polymer, optionally wherein the polymer is carbon-based.

15. The method of any one of claims 1 to 8, 13 or 14, the composition of any one of claims 9, or 12 to 14, or the kit of any one of claims 10 or 12 to 14, wherein:
a) the microorganism is a pathogenic microorganism, optionally wherein the pathogenic microorganism is a pathogenic bacterium or fungus; and/or
b) the non-microorganism cells comprise red blood cells and/or white blood cells.

## Patentansprüche

1. Verfahren zum Trennen von Mikroorganismen von Nicht-Mikroorganismus-Zellen in einer Probe, die Nicht-Mikroorganismus-Zellen enthält, wobei das Verfahren umfasst:
a) Inkubieren der Probe mit Partikeln, um Partikel-Mikroorganismus-Komplexe zu bilden, wobei der Schritt des Inkubierens in der Gegenwart von Natriumpolyanetholsulfonat und einem Reagenz durchgeführt wird, das Nicht-Mikroorganismus-Zellen in der Probe selektiv lysiert, während es intakte Mikroorganismen bewahrt, die in der Probe vorliegen; und
b) Trennen der Partikel-Mikroorganismus-Komplexe von den Nicht-Mikroorganismus-Zellen,
wobei die Probe eine Blut-enthaltende Probe ist, wobei die Partikel einen Durchmesser von zwischen 0,1 und 3,0 µm aufweisen, und wobei die äußere Oberfläche der Partikel eins oder mehrere aus:
i) Carbonsäuregruppen;
ii) Aminogruppen;
iii) hydrophobe Gruppen; und
iv) Streptavidin
umfasst oder damit beschichtet ist.

2. Verfahren nach Anspruch 1, wobei das Verfahren ein Verfahren zum Nachweisen der Abwesenheit oder Anwesenheit eines Mikroorganismus in einer Probe ist, die auch Nicht-Mikroorganismus-Zellen enthält, und ferner umfasst:
c) Nachweisen der Abwesenheit oder Anwesenheit von Mikroorganismen in den Partikel-Mikroorganismus-Komplexen.

3. Verfahren nach Anspruch 2, wobei Schritt (c) umfasst:
(A)
(i) Nachweisen einer enzymatischen Aktivität eines Nukleinsäuremoleküls, das mit dem Mikroorganismus assoziiert ist, (ii) direktes Nachweisen des Mikroorganismus durch Zytometrie oder Mikroskopie, (iii) Nachweisen des Mikroorganismus nach Zellkultur, (iv) Nachweisen des Mikroorganismus durch Nukleinsäureamplifikation oder (v) Nachweisen des Mikroorganismus durch Nukleinsäuresequenzierung; oder
(B)
i) Lysieren der Mikroorganismen in den Partikel-Mikroorganismus-Komplexen;
ii) Inkubieren des Lysats mit einem Nukleinsäuremolekül, das als ein Substrat für eine Nukleinsäure-modifizierende Aktivität der Mikroorganismen dient; und
iii) spezifisches Bestimmen der Abwesenheit oder Anwesenheit eines modifizierten Nukleinsäuremoleküls, das aus der Einwirkung des Nukleinsäure-modifizierenden Enzyms auf das Substrat-Nukleinsäuremolekül resultiert, um die Abwesenheit oder Anwesenheit des Mikroorganismus anzuzeigen,
wobei gegebenenfalls Schritt (i) Zugeben eines Lyse-Reagenzes umfasst, das das Substrat-Nukleinsäuremolekül enthält.

4. Verfahren nach Anspruch 3B), wobei das Nukleinsäure-modifizierende Enzym eine DNA- oder RNA-Polymerase umfasst, wobei gegebenenfalls die DNA-Polymerase DNA-Polymerase I ist.

5. Verfahren nach einem der Ansprüche 2 bis 4, wobei das Verfahren ein Verfahren zum Nachweisen der Abwesenheit oder Anwesenheit einer Mikroorganismeninfektion in einem Individuum ist, wobei die Probe eine Blut-enthaltende Probe von dem Individuum ist.

6. Verfahren nach einem der vorhergehenden Ansprüche, wobei das Verfahren ferner Waschen der getrennten Partikel-Mikroorganismus-Komplexe umfasst, um Nicht-Mikroorganismus-Zellen oder Lysat zu entfernen.

7. Verfahren nach einem der vorhergehenden Ansprüche, wobei Schritt b):
i) ferner Entfernen der Nicht-Mikroorganismus-Zellen von den Partikel-Mikroorganismus-Komplexen umfasst; und/oder
ii) unter Verwendung eines Magnetfelds oder von Zentrifugation durchgeführt wird.

8. Verfahren nach einem der Ansprüche 1 bis 7, wobei das Reagenz, das Nicht-Mikroorganismus-Zellen in der Probe selektiv lysiert, während es intakte Mikroorganismen bewahrt, die in der Probe vorliegen, ein Detergens umfasst; wobei gegebenenfalls das Detergens nichtionisch ist und/oder das Detergens nicht an die Partikel konjugiert ist, die fähig sind zum Bilden von Komplexen mit Mikroorganismen.

9. Zusammensetzung umfassend:
a) eine aus einem Individuum isolierte Blut-enthaltende Probe, die möglicherweise Mikroorganismuszellen enthält und die Nicht-Mikroorganismus-Zellen enthält;
b) Partikel, die fähig sind, Komplexe mit Mikroorganismen zu bilden, wobei die Partikel einen Durchmesser von zwischen 0,1 und 3,0 µm aufweisen, und wobei die Partikel eine äußere Oberfläche aufweisen, die eins oder mehrere aus:
i) Carbonsäuregruppen;
ii) Aminogruppen;
iii) hydrophobe Gruppen; und
iv) Streptavidin
umfasst oder damit beschichtet ist;
c) Natriumpolyanetholsulfonat; und
d) mindestens ein Reagenz, das Nicht-Mikroorganismus-Zellen in der Probe selektiv lysiert, während es intakte Mikroorganismen bewahrt, die in der Probe vorliegen, wobei das mindestens eine Reagenz, das Nicht-Mikroorganismus-Zellen in der Probe selektiv lysiert, während es intakte Mikroorganismen bewahrt, die in der Probe vorliegen, ein Detergens umfasst.

10. Kit zum Durchführen des Verfahrens nach einem der Ansprüche 1 bis 8, umfassend:
a) Partikel, die fähig sind, Komplexe mit Mikroorganismen zu bilden, wobei die Partikel einen Durchmesser von zwischen 0,1 und 3,0 µm aufweisen, und wobei die Partikel eine äußere Oberfläche aufweisen, die eins oder mehrere aus:
i) Carbonsäuregruppen;
ii) Aminogruppen;
iii) hydrophobe Gruppen; und
iv) Streptavidin
umfasst oder damit beschichtet ist;
b) Natriumpolyanetholsulfonat; und
c) mindestens ein Reagenz, das Nicht-Mikroorganismus-Zellen in der Probe selektiv lysiert, während es intakte Mikroorganismen bewahrt, die in der Probe vorliegen, wobei das mindestens eine Reagenz, das Nicht-Mikroorganismus-Zellen in der Probe selektiv lysiert, während es intakte Mikroorganismen bewahrt, die in der Probe vorliegen, ein Detergens umfasst.

11. Kit nach Anspruch 10 zum Durchführen des Verfahrens nach einem der Ansprüche 2 bis 8, ferner umfassend:
Nachweismittel zum Nachweisen der Abwesenheit oder Anwesenheit von Mikroorganismen in den Partikel-Mikroorganismus-Komplexen, wobei der Mikroorganismus ein pathogenes/r Bakterium oder Pilz ist und wobei das Nachweismittel ein Nukleinsäuremolekül (DNA) umfasst, das als ein Substrat für eine Nukleinsäure-modifizierende Aktivität der Mikroorganismen dient, und wobei das Nukleinsäuremolekül (DNA) mindestens teilweise doppelsträngig ist und Uracilreste in dem komplementären Strang umfasst.

12. Zusammensetzung nach Anspruch 9 oder Kit nach Anspruch 10 oder 11, ferner umfassend einen Puffer und/oder Natriumchlorid.

13. Verfahren nach einem der Ansprüche 1 bis 8, Zusammensetzung nach Anspruch 9 oder 12 oder Kit nach einem der Ansprüche 10 bis 12, wobei die Partikel: a) einen Durchmesser zwischen 0,1 und 2,0 µm aufweisen und/oder
b) magnetisch sind.

14. Verfahren nach einem der Ansprüche 1 bis 8 oder 13, Zusammensetzung nach einem der Ansprüche 9, 12 oder 13 oder Kit nach einem der Ansprüche 10 bis 13, wobei die äußere Oberfläche der Partikel, die fähig sind zum Bilden von Komplexen mit Mikroorganismen, ein Polymer umfasst, wobei gegebenenfalls das Polymer kohlenstoffbasiert ist.

15. Verfahren nach einem der Ansprüche 1 bis 8, 13 oder 14, Zusammensetzung nach einem der Ansprüche 9 oder 12 bis 14 oder Kit nach einem der Ansprüche 10 oder 12 bis 14, wobei
a) der Mikroorganismus ein pathogener Mikroorganismus ist, wobei gegebenenfalls der pathogene Mikroorganismus ein pathogenes/r Bakterium oder Pilz ist; und/oder
b) die Nicht-Mikroorganismus-Zellen rote Blutkörperchen und/oder weiße Blutkörperchen umfassen.

## Revendications

1. Procédé de séparation de micro-organismes de cellules non micro-organismes dans un échantillon contenant des cellules non micro-organismes, le procédé comprenant les étapes consistant à :
a) incuber l'échantillon avec des particules pour former des complexes particule/micro-organisme, dans lequel l'étape d'incubation est réalisée en présence de polyanéthol sulfonate de sodium et d'un réactif qui lyse sélectivement les cellules non micro-organismes dans l'échantillon tout en conservant intacts des micro-organismes présents dans l'échantillon ; et
b) séparer les complexes particule/micro-organisme des cellules non micro-organismes, dans lequel l'échantillon est un échantillon contenant du sang, dans lequel les particules ont un diamètre compris entre 0,1 et 3,0 µm, et dans lequel la surface externe des particules comprend ou est revêtue d'un ou plusieurs parmi :
i) des groupes acide carboxylique ;
ii) des groupes amino ;
iii) des groupes hydrophobes ; et
iv) de la streptavidine.

2. Procédé selon la revendication 1, dans lequel le procédé est un procédé de détection de l'absence ou de la présence d'un micro-organisme dans un échantillon qui contient également des cellules non micro-organismes comprenant en outre l'étape consistant à :
c) détecter l'absence ou la présence de micro-organismes dans les complexes particule-micro-organisme.

3. Procédé selon la revendication 2, dans lequel l'étape (C) comprend les étapes consistant à :
(A)
(i) détecter une activité enzymatique d'une molécule d'acide nucléique associée au micro-organisme, (ii) détecter le micro-organisme directement par cytométrie ou microscopie, (iii) détecter le micro-organisme après une culture cellulaire, (iv) détecter le micro-organisme par amplification d'acide nucléique ou (v) détecter le micro-organisme par séquençage d'acide nucléique ; ou
(B)
i) lyser les micro-organismes dans les complexes particule/micro-organisme ;
ii) incuber le lysat avec une molécule d'acide nucléique qui agit comme un substrat pour l'activité de modification d'acide nucléique des micro-organismes ; et
iii) déterminer spécifiquement l'absence ou la présence d'une molécule d'acide nucléique modifiée résultant de l'action de l'enzyme de modification d'acide nucléique sur la molécule d'acide nucléique de substrat pour indiquer l'absence ou la présence du micro-organisme, facultativement dans lequel l'étape (i) comprend l'ajout d'un réactif de lyse contenant la molécule d'acide nucléique de substrat.

4. Procédé selon la revendication 3 (B), dans lequel l'enzyme de modification d'acide nucléique comprend une ADN ou une ARN polymérase, facultativement dans lequel l'ADN polymérase est l'ADN polymérase I.

5. Procédé selon l'une quelconque des revendications 2 à 4, dans lequel le procédé est un procédé de détection de l'absence ou de la présence d'une infection par un micro-organisme chez un sujet, dans lequel l'échantillon est un échantillon contenant du sang provenant du sujet.

6. Procédé selon l'une quelconque des revendications précédentes, dans lequel le procédé comprend en outre un lavage des complexes particule-micro-organisme séparés pour éliminer des cellules ou un lysat de non micro-organismes.

7. Procédé selon l'une quelconque des revendications précédentes, dans lequel l'étape b) :
i) comprend en outre un retrait des cellules non micro-organismes à partir des complexes particule-microorganisme ; et/ou
ii) est effectuée en utilisant un champ magnétique ou une centrifugation.

8. Procédé selon l'une quelconque des revendications 1 à 7, dans lequel le réactif qui lyse sélectivement les cellules non micro-organismes dans l'échantillon tout en conservant intacts des micro-organismes présents dans l'échantillon comprend un détergent ; facultativement dans lequel le détergent est non ionique et/ou le détergent n'est pas conjugué aux particules capables de former des complexes avec des micro-organismes.

9. Composition, comprenant :
a) un échantillon contenant du sang isolé à partir d'un sujet contenant potentiellement des cellules micro-organismes et qui contient des cellules non micro-organismes ;
b) des particules capables de former des complexes avec des micro-organismes, dans laquelle les particules présentent un diamètre compris entre 0,1 et 3,0 µm, et dans laquelle les particules présentent une surface extérieure comprenant ou revêtue de l'un quelconque ou de plusieurs parmi :
i) des groupes acide carboxylique ;
ii) des groupes amino ;
iii) des groupes hydrophobes ; et
iv) de la streptavidine ;
c) du polyanéthol sulfonate de sodium ; et
d) au moins un réactif qui lyse sélectivement les cellules non micro-organismes dans l'échantillon tout en conservant intacts des micro-organismes présents dans l'échantillon, dans laquelle le au moins un réactif qui lyse sélectivement les cellules non micro-organismes dans l'échantillon tout en conservant intacts des micro-organismes présents dans l'échantillon comprend un détergent.

10. Kit pour effectuer le procédé selon l'une quelconque des revendications 1 à 8, comprenant :
a) des particules capables de former des complexes avec des micro-organismes, dans lequel les particules présentent un diamètre compris entre 0,1 et 3,0 µm, et dans lequel les particules présentent une surface externe comprenant ou revêtue de l'un quelconque ou de plusieurs parmi :
i) des groupes acide carboxylique ;
ii) des groupes amino ;
iii) des groupes hydrophobes ; et
iv) de la streptavidine ;
b) du polyanéthol sulfonate de sodium ; et
c) au moins un réactif qui lyse sélectivement des cellules non micro-organismes dans l'échantillon tout en conservant intacts des micro-organismes présents dans l'échantillon, dans lequel le au moins un réactif qui lyse sélectivement les cellules non micro-organismes dans l'échantillon tout en conservant intacts des micro-organismes présents dans l'échantillon comprend un détergent.

11. Kit selon la revendication 10 pour effectuer le procédé selon l'une quelconque des revendications 2 à 8, comprenant en outre : des moyens de détection pour détecter l'absence ou la présence de micro-organismes dans les complexes particule-microorganisme, dans lequel le micro-organisme est une bactérie ou un champignon pathogène et dans lequel les moyens de détection comprennent une molécule d'acide nucléique (ADN) qui agit comme un substrat pour une activité de modification d'acide nucléique des micro-organismes, et dans lequel la molécule d'acide nucléique (ADN) est au moins partiellement à double brin et comprend des résidus d'uracile dans le brin complémentaire.

12. Composition selon la revendication 9, ou kit selon la revendication 10 ou 11, comprenant en outre un tampon et/ou du chlorure de sodium.

13. Procédé selon l'une quelconque des revendications 1 à 8, composition selon la revendication 9 ou 12, ou kit selon l'une quelconque des revendications 10 à 12, les particules : a) présentant un diamètre compris entre 0,1 et 2,0 µm ; et/ou
b) étant magnétiques.

14. Procédé selon l'une quelconque des revendications 1 à 8 ou 13, composition selon l'une quelconque des revendications 9, 12 ou 13, ou kit selon l'une quelconque des revendications 10 à 13, la surface extérieure des particules capables de former des complexes avec des micro-organismes comprenant un polymère, facultativement le polymère étant à base de carbone.

15. Procédé selon l'une quelconque des revendications 1 à 8, 13 ou 14, composition selon l'une quelconque des revendications 9, ou 12 à 14, ou kit selon l'une quelconque des revendications 10 ou 12 à 14 :
a) le micro-organisme étant un micro-organisme pathogène, facultativement le micro-organisme pathogène étant une bactérie ou un champignon pathogène ; et/ou
b) les cellules non micro-organismes comprenant des globules rouges et/ou des globules blancs.
